# EUROPEAN PATENT SPECIFICATION

(11) **EP 0 477 972 B1**
(45) Date of publication and mention of the grant of the patent: **04.09.1996**
(21) Application number: 91116561.1
(22) Date of filing: 27.09.1991
(51) Int. Cl.: C12Q 1/70, C12Q 1/68, C12P 19/34, C07H 21/04

(54) **Nucleotide sequences useful as type-specific probes, PCR primers and LCR probes for the amplification and detection of human papilloma virus, and related kits and methods**
Nukleotid-Sequenzen nützlich als typenspezifische Sonden, PCR Primers und LCR Sonden zur Amplifikation und zum Nachweis von humanem Papillomavirus, sowie dazu verwendete Kits und Verfahren
Séquences nucléotidiques utiles comme sondes spécifiques du type amorces de PCR et sondes pour l'amplification et détection du virus-papilloma humain, et kits et procédés utilisés dans ce but

(30) Priority: 28.09.1990 US 589948; 28.09.1990 US 590105; 28.09.1990 US 590253
(43) Date of publication of application: 01.04.1992
(73) Proprietor: ABBOTT LABORATORIES, Abbott Park, Illinois 60064-3500 (US)
(72) Inventor: Joseph, Jeffrey L., Cherry Hill, New Jersey 08002 (US); Bouma, Stanley R., Mundelein, Illinois 60060 (US); Marshall, Ronald L., Zion, Illinois 60099 (US); Laffler, Thomas G., Libertyville, Illinois 60048 (US)
(74) Representative: Modiano, Guido, Dr.-Ing.

(56) References cited:
- EP-A- 0 301 968
- EP-A- 0 320 308
- EP-A- 0 402 132
- EP-A- 0 425 995
- WO-A-87/05630
- WO-A-89/05357
- WO-A-89/09940
- WO-A-90/02821
- WO-A-91/10675

## Description

This invention relates generally to human papilloma virus, and more particularly, relates to nucleotide sequences of short strands of human papilloma virus which can be amplified and/or used to determine the presence of human papilloma virus products in a test sample, and some of which also can be amplified and/or used to determine the specific type of human papilloma virus of types 16 and 18 present in the test sample.

Human papilloma virus (HPV) ) is recognized as a venereally-transmitted disease of the anogenital tract which often is associated with the pathogenesis of cervical cancer and its precursor lesions. More than 56 types of HPV have been characterized. Of these, at least 21 types infect the anogenital tract. L. Gregoire et al., J. Clin. Micro 27 (12):2660- 2665( 1989). These mucosotropic viruses are associated most frequently with benign condyloma or latent infections. However, the presence of HPV in premalignant lesions and invasive cancers, particularly of the cervix, may reflect the oncogenic potential of these viruses. See P. M. Howley, in Important Advances in Oncology, D. T. DeVita, Jr. et al., eds., J. B. Lippincott, Philadelphia, PA (1987) at pages 55-73.

Certain HPV types, namely, HPV type 16 and type 18, and to a lesser extent HPV types 31, 33 and 35, are found in a high proportion of invasive cervical cancers and their metastases. However, many HPV types which infect the anogenital tract, such as HPV types 6 and 11, are found most commonly in benign condyloma and only rarely are found in invasive cancers. HPV detected in the anogenital tract can be classified broadly as low risk papilloma viruses ( HPV types 6 and 11), intermediate risk papilloma viruses ( HPV types 31 , 33 and 35) or high risk papilloma viruses ( HPV types 16 and 18), based on the association of the particular HPV type with malignancy. A. T. Lorincz et al., J Nat'l Cancer Inst. 79:671 (1987). Thus, the detection of the presence of HPV and the determination of the specific type of HPV can provide a diagnostic and prognostic tool useful for determining the clinical significance associated with certain HPV types. The early detection of HPV by sensitive and specific reagents and methodologies also could provide earlier therapeutic management and counseling.

A need therefore exists for accurate and reliable methods to identify and type HPV in clinical specimens. However, known polyclonal antisera prepared by immunizing animals with disrupted virions are capable of detecting HPV antigens in only about 30-70% of cutaneous and mucosal warts. Further, the antisera are broadly cross-reactive. Available immunological tests have two major drawbacks. First, only well-differentiated cells apparantly are capable of viral antigen expression. HPV-infected tissues which show higher degrees of neoplasia, such as carcinoma in situ, rarely contain HPV antigen. Thus, the further the development of the malignancy, the smaller the amount of detectable virus in the tested tissue. Secondly, these immunological tests are unable to identify specific viral types.

It is known that papilloma viruses share amino acid sequences in the major capsid proteins. See, for example, C.C. Baker, in The Papovaviridae (Vol. 2), P. M. Howley and N. P. Salzman, eds., Plenum Publ. Corp., New York (1987) at pages 321-385. The DNAs of this virus cross-hybridize, indicating homologous sequences. M. F. Law et al., J. Virol. 58:225-229 (1979). Thus, molecular hybridization techniques have been developed as a more sensitive and specific means of detecting and differentiating HPV DNA and RNA in clinical specimens. See A. T, Lorinez, Obstetrics and Gynecol. Clinics of N. America 14:451 ( 1987).

Sequences specific for the DNA and RNA of human papilloma virus are known and have been published. See, for example, PCT application No. WO 89/69940 published October 19, 1989, PCT application No. WO 86/05816 published October 9, 1986 and European Patent Application No. 0 301 968 published February 1, 1989.

The molecular hybridization techniques used to detect homologous DNA sequences are sensitive and can be highly specific if used with probes which bind to nucleic acid sequences which are unique to a particular HPV type. However, the concentration of total viral DNA in a given clinical sample may be below the limit of sensitivity of the test. For example, the amount of viral DNA in dysplastic cervical lesions is reduced with increasing dysplasia.

To overcome this problem of sensitivity, viral DNA sequences can be amplified by using, for example, the polymerase chain reaction (PCR) or the ligase chain reaction (LCR) techniques. The products thus obtained can be identified by using conventional hybridization techniques for identification of virus types, such as Southern blotting. See C. Oste, Biotechniques 6:163( 1988), K. B. Mullis, U. S. Patent No. 4,683,202, and EP-A-320 308 (BioTechnica).

Both PCR and LCR serve to amplify the DNA present in a test sample to detectable levels. In practice, the level of sensitivity is about 50 to 100 copies per sample. The next most sensitive technique is dot-blot, which can detect about 10,000 molecules, while Southern blot reliably detects about 100,000 copies of DNA per sample.

Thus, the appropriate diagnosis of HPV may require two steps. In one strategy, the presence of a clinically relevant type of HPV is first detected with a group-specific primer. After the presence of HPV is detected, differentiation between types can be performed by using a type-specific probe having low homology between the HPVs of the group. Alternatively, differentiation can be performed using a mixture of type-specific probes at the outset, provided these probes amplify DNA independently of each other, and that they can be detected independently. In the past, such tasks were attempted using specific antibodies. In general, nucleic acid probes and primers allow greater discrimination among subtypes than do antibodies. The use of DNA-based tests increases both sensitivity and specificity over prior-art antibody-based tests.

It therefore would be advantageous to provide oligonucleotide strands of DNA which could be amplified and used to detect the presence, if any, of HPV in a test sample. It also would be advantageous to provide short oligonucleotide strands of DNA which could be amplified and used to detect the presence, if any, of specific types of HPV in the test sample. The combined use of oligonucleotide strands would be advantgeous for allowing for the specific and sensitive in vitro diagnosis of the presence and specific type of HPV present in test samples.

### SUMMARY OF THE INVENTION

Oligonucleotides of from about 10 to about 60 nucleotides are provided which can be amplified and used either to detect specific sequences of specific types of human papilloma virus, or consensus regions with high homology among different types. The presence of HPV is determined by contacting the test sample with sequences provided to detect the presence, if any, of HPV types 6, 11, 16, 18, 31, 33 and 61. This may be done with or without prior amplification, for example, by PCR or LCR. Either type-specific or consensus amplification is also possible. Two oligonucleotides are provided if the sequence is to be amplified by PCR, and four oligonucleotides provided if amplification is by LCR, in accordance with these known amplification procedures. After the presence of HPV is detected, the type of HPV present in the sample can be determined by using HPV type-specific probes, by subsequent rounds of PCR, or by LCR. Alternatively, the presence of type-specific HPV can be determined by contacting the test sample directly with type-specific nucleotide sequence provided by the invention for the detection of HPV types 16 and 18. Also provided are methods for using the oligonucleotides and kits for amplifying and detecting the presence of human papilloma virus.

### BRIEF DESCRIPTION OF THE DRAWINGS

FIG. 1 is a photograph of a gel following electrophoresis showing the results when the primers PCR 1 and PCR5 were used to amplify selected plasmids wherein HPV 6 is in lane 1, HPV 11 is in lane 2, HPV 16 is in lane 3, HPV 18 is in lane 4, and HPV 31 is in lane 5, HPV 33 is in lane 6, HPV 61 is in lane 7, and molecular weight standards are in lane 8.

FIG. 2 is a photograph of a gel following electrophoresis showing the results when the primers PCR 1, PCR2, PCR3, PCR4 and PCR5 were used to amplify plasmid p65.16.8 (HPV 16). PCR1 and PCR5 are primers according to the invention.

FIG. 3 is a photograph of the ethidium bromide-stained gels wherein PCR 1 4 and PCR15 are used in conjunction with IWDO to obtain amplified PCR product.

FIG. 4 is a graph of results obtained from performing LCR on 10⁷ molecules of the selected target using LCR5A, LCR5A', LCR5B and LCR5B'. The rate of reaction of 4-methyl lumbelliferone is expressed as fluorescence counts/second/second and plotted against the target HPV type.

FIG. 5 is a graph of results obtained from performing LCR on 10⁷ molecules of the selected target using LCR6A, LCR6A', LCR6B and LCR6B'. The rate of reaction of 4-methyllumbelliferone is expressed as fluorescence counts/second/second and plotted against the target HPV type.

FIG. 6 is a graph of results obtained from perfomring LCR on 10⁷ molecules of the selected target using LCR7A, LCR7A', LCR7B and LCR7B'. The rate of reaction of 4-methyllumbelliferone is expressed as fluorescence counts/second/second and plotted against the target HPV type.

FIG. 7 is a graph of results obtained from perfomring LCR on 10⁷ molecules of the selected target using LCR8A, LCR8A', LCR8B and LCR8B'. The rate of reaction of 4-methyllumbelliferone is expressed as fluorescence counts/second/second and plotted against the target HPV type.

### DETAILED DESCRIPTION OF THE INVENTION

The appropriate diagnosis of HPV requires two sets of conditions. The first enables the detection of all pertinent types, and the second set allows differentiation among them. In the past, such tasks have been attempted using specific antibodies. In general, nucleic acid probes and primers allow greater discrimination among subtypes than do antibodies. Thus, the use of DNA-based tests tends to increase both sensitivity and specificity over antibody-based tests.

U. S. Patents No. 4,683,195 and 4,683,202 teach a method of amplifying DNA sequences by using PCR. This method now is a standard procedure in many molecualr biology laboratories. Examples 1-3 which follow below utilize the procedures taught in these two patents and the method as described in the package insert of the commercially-available Gene-Amp™ kit (Document No. 55635-6/89, Perkin-Elmer/Cetus, Emeryville, CA).

In PCR, two complementary polynucleotide strands are amplified by treating the strands with two oligonucleotide primers such that an extension product of each primer is synthesized which is complementary to each nucleic acid strand. The primers are selected such that the extension product of one primer forms a template for the synthesis of an extension product from the other primer once the extension product of the one primer is separated from the template. A chain reaction is maintained by a cycle of denaturing the primer extension products from their templates, treating the single-stranded molecule generated with the same primers to re-anneal, and allowing the primers to form further extension products. The cycle is repeated for any many times as it takes to increase the target nucleic acid segments to a concentration where they can be detected.

The amplified target sequence can be detected by any of several known techniques; for example, by denaturing the double-stranded products formed by PCR, and treating those products with one or more reporter probes which hybridize with the extension products. The reporter probe has a detectable label, and usually is added in excess. The unhybridized reporter probe, therefore, must be separated from the hybridized reporter probe by involving a separation step. In another method of detecting the extension products without reporter probe and a separation step, the extension products are detected by gels stained with ethidium bromide. The diagnosis can be confirmed by transferring the DNA to nitrocellulose and probing with a probe specific to the HPV type suspected of being present in the sample.

Alternately with PCR, one may take advantage of known restriction sites within the HPV DNA to demonstrate that the amplified DNA contains the expected sequence by examining the cleavage pattern(s) generated with one or more restriction endonucleases. Verifying the authenticity of the amplified sequence may be necessary for two reasons: (1) to ensure that sequences complementary to the amplifying primers are not fortuitously present in cellular DNA which does not contain HPV DNA, and (2), to identify the type of HPV present in the sample. If the sequences chosen for amplification are conserved among HPV types, then the finding of an amplified product does not implicate a particular HPV type. It also should be possible to predict the size of the amplified product based on the binding positions of the two primers. Thus, when that product is found, one reasonably can be assured that HPV is present. However, two different types of HPV may give the same or different size products. Thus, hybridization should be used to confirm the identity of the amplified sequence until confidence is built that the interpretation of the results is reliable. It should be pointed out that the PCR technique will identify only closely related, or type-specific sequences in the absence of highly homologous primers, since only a small portion of the genome is analyzed.

Another particularly useful detection technique is described in EP-A-357 011. In this method, a different reporter molecule, e.g. hapten, is attached to each primer. Following amplification, but before denaturation, duplexes can be detected by "capturing" one hapten (hapten1) with a solid phase coated with anti-hapten1. The separated complex can be detected with a conjugate of label and anti-hapten2, and label associated with the solid phase can be measured.

The Ligase Chain Reaction (LCR) amplifies sections of DNA by copying the section of DNA, and copying the copies of that section of DNA, many times over. This method is described in European Patent Application No. 0 320 308 published June 14, 1989, which is incorporated herein by reference. In this procedure, two probes (for example, A and B) complementary to immediately adjacent regions of a target sequence are hybridized and ligated. This ligated probe then is denatured away from the target, after which it is hybridized with two additional probes (A' and B') of sense opposite to the initial probes A and B. The secondary probes are themselves then ligated. Subsequent cycles of denaturation/hybridization/ligation create the formation of double-length probes of both sense (+) and antisense (-).

In LCR, the nucleic acid of the sample is provided either as single stranded DNA or as double-stranded DNA which is denatured to separate the strands. Four probes are utilized: the first two probes (A and B) are the so-called primary probes, and the second two probes (A' and B') are the so-called secondary probes. The first probe (A) is a single strand capable of hybridizing to a first segment of the primary strand of the target nucleotide sequence. The second probe (b) is capable of hybridizing to a second segment of the primary strand of the target nucleotide sequence. The 5' end of the first segment of the primary strand of the target is positioned relative to the 3' end of the second segment of the primary strand of the target to enable joining of the 3' end of the first probe to the 5' end of the second probe, when the probes are hybridized to the primary strand of the target nucleotide sequence. The third probe (A') is capable of hybridizing to the first probe, and the fourth probe (B') is capable of hybridizing to the second probe (B). The hybridized probes are ligated to form reorganized fused probe sequences. Then, the DNA in the sample is denatured to separate ligated probes from sample DNA. Successive cycles wherein the ligated probes and target DNA undergo the above-described process are performed to increase the amount of detectable DNA in the sample. The amount of cycles performed is dependent upon the sequence used and the sensitivity required of the test. Usually, the cycle can be repeated from 15 to 60 times. At least one of the probes can be conjugated to a signal generating compound.

If the four probes are conjugated to appropriate binding members, the detection of amplified product can be accomplished using standard manual or automated immunoassay procedures known to those skilled in the art. These procedures include, for example, immunochromatography, ELISA, EIA and MEIA. Hybridization also can be accomplished by following standard dot-, slot- or replica-blot procedures which are known to those in the art. The sequences can be labelled with an appropriate signal generating compound (label), which is capable of generating a measureable signal detectable by external means. The various signal generating compounds contemplated include chromogens, catalysts such as enzymes, luminescent compounds such as fluoroscein and rhodamine, chemiluminescent compounds, radioactive elements such as ³²P, and other labels known to those of ordinary skill in the art. The selection of a particular label is not critical, but it will be capable of producing a a signal either by itself or in conjunction with one or more additional substances. A variety of different indicator reagents can be formed of label and specific binding member. Either the label or a specific binding member can be varied. Examples of specific binding members which can be used as a member of the indicator reagent include antibodies, both monoclonal, polyclonal, and fragments thereof; avidin or biotin, biotin and anti-biotin, a carbohydrate or a lectin, a complementary nucleotide sequence, an effector or a receptor molecule, an enzyme cofactor or an enzyme; an enzyme inhibitor or an enzyme; also any antigenic substances, haptens, antibodies, and combinations thereof.

The test sample can be any biological material suspected of containing HPV. Thus, the test sample can be human body tissue, or a test sample which contains cells suspected of containing HPV.

The invention will now be described by way of Examples, which are meant to describe, but not to limit, the spirit and scope of the invention.

The following terms used in the examples are trademarks, tradenames or chemical abbreviations as specified:
TRIS - chemical abbreviation for [tris(hydroyxmethyl)aminomethane], used as a buffer.
EDTA - chemical abbreviation for ethylenediaminetetraacetic acid, a chelating agent.
FITC - chemical abbreviation for fluorescein isothiocyanate, a flourescent hapten derivative.
NHS-ester - chemical abbreviation for N-hydroxysuccinamide ester
MES - chemical abbreviation for [2-(N-morpholino)ethanesulfonic acid], a buffer.
TWEEN®-20 - trademark of Atlas Chemical for polyoxyethylene sorbitan monolaurate, a detergent.
BIS-TRIS - chemical abbbreviation for [bis-(2-hydroxyethyl )-amino]tris-(hydroxymethyl)methane, a buffer.
TRITON X- 100® - trademark of Rohm & Haas for nonaethylene glycol octylphenol ether, a detergent.
IMx® - trademark of Abbott Laboratories for an automated instrument for performing microparticle enzyme immunoassay (MEIA).

### EXAMPLES

### EXAMPLE 1

PCR was performed essentially following the package insert of the commercially available Gene-Amp™ kit (document No. 55635-6/89, available from Perkin-Elmer/Cetus, Emeryville, CA). The following reagents were mixed in a 0.5 mL polypropylene tube and used in performing PCR:

| Reagent | Final Concentration |
|---|---|
| Water | (to give final volume = 50 or 100 µL) |
| Reaction Buffer | 10 mM TRIS pH 8.3 |
| | 50 mM KC1 |
| | 1.5 mM MgC12 |
| | 0.01% gelatin |
| dNTP mixture | 200 µM each of dATP,dCTP,dGTP, and TTP |
| pCR1 | 1 µM |
| | |
| pCR2 | 1 µM |
| plasmid | 10 µL 1 ng/100µL |
| (or control-human placental DNA (Pooled Placental DNA, catalog D-3287, Sigma Chemical Co, St. Louis MO). | |
| DNA polymerase, | |
| Thermus Acquaticus | 25 *or* 63.9 units/1 mL |

After mixing, the reaction mixture was overlayed with 100 µL of mineral oil. The tube then was placed in an instrument capable of incubation at several temperatures, and subjected to 30 or 40 cycles of programmed temperature change. The precise cycle of temperature change used, and the instrument used, varied with the experiment, and is detailed in the descriptions of the figures in Example 3.

### EXAMPLE 2

Following the procedure of Example 1, the following sequences were found to amplify sections of papilloma virus types 6, 11, 16, 18, 31, 33, and 61 using PCR. PCR1: CAGATGTCTC TGTGGCGGCC TAGTG (ID No. 1) PCR5: AGGTGTCAGG AAAACCAAAT TTATT (ID No. 5) PCR14: GAATTAGTTA GACCATTTAA AAG (ID No. 6) PCR15: GGGGAAACAC CAGAATGGAT A (ID No. 7) IWDO:   ATCATATGCC CACTGTACCA T        (ID No. 8) Sequence IWDO is derived from a sequence disclosed in International application number PCT/US86/00629 (WO 86/05816).

TABLE 1 shows the sequences and where they map to to in the various types.

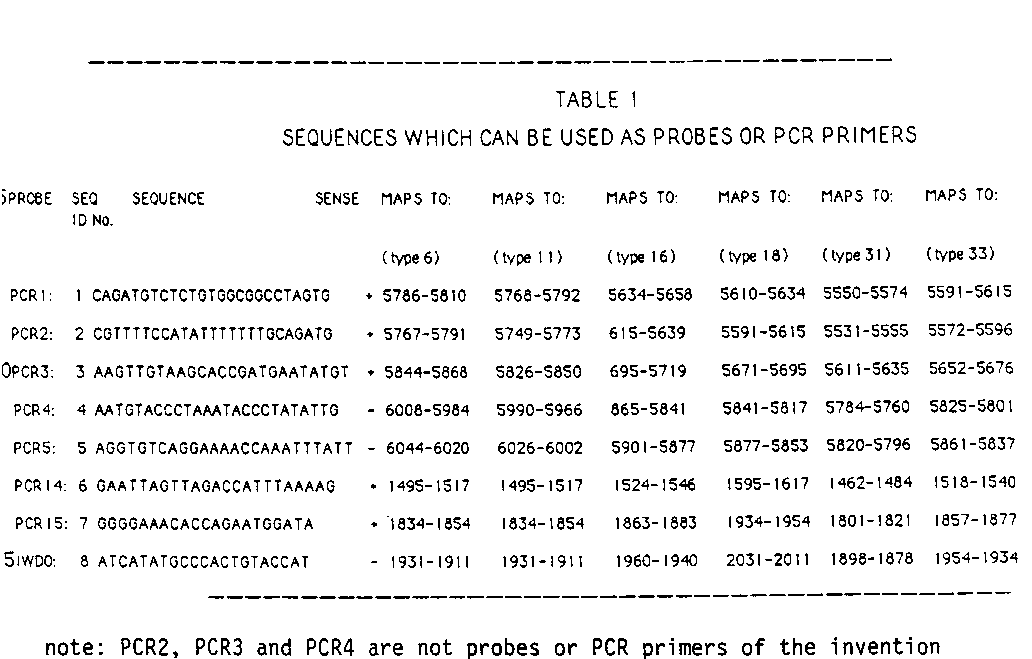

### EXAMPLE 3

Linearized plasmids containing full-length papilloma virus inserts in pGEM3 were used as targets. These were pHPV6.1 ( HPV6), pSP65.11.5 ( HPV 11), p65.16,8 (HPV16), pHPV18H(HPV18), pG3 HPV31 ( HPV31),pLNK322,HPV33 ( HPV33), and pBR322.HPV61 (HPV61). The Programmable Cyclic Reactor™ (available from Ericomp, San Diego) was used as the incubation instrument. Following PCR procedures as described in Example 1,10 µL aliquots were analyzed by electrophoresis through agarose (comprising a 5:3 ratio of NuSieve®:SeaKem® GTG, available from the FMC Corp., Rockland, ME) in a buffer comprising 0.089 M TRIS, 0.089 M borate, 2 mM EDTA, and 0.5 ppt ethidium bromide.

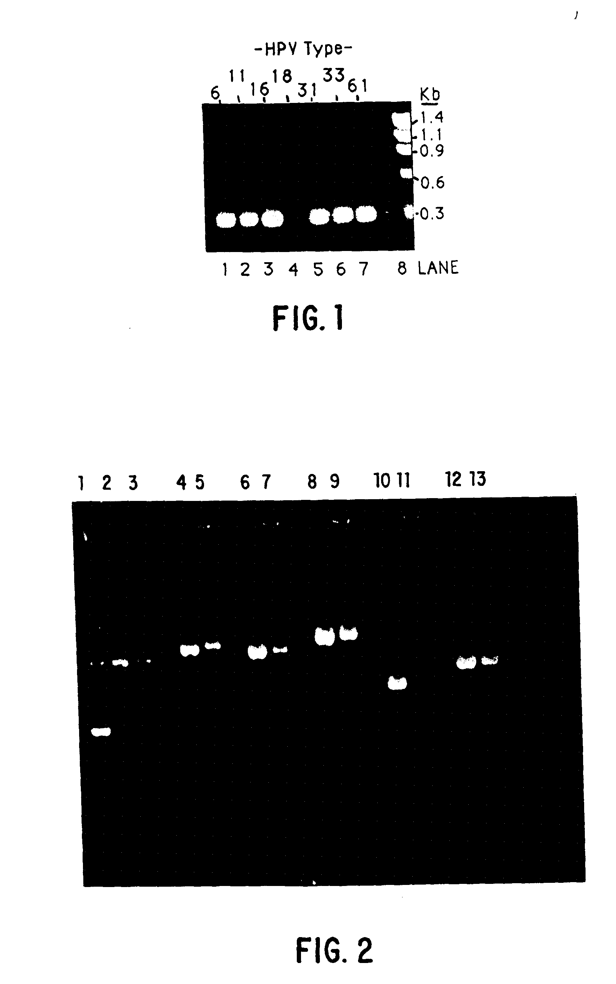

FIG. 1 is a photograph of an ethidium bromide-stained 1.2% agarose gel showing results using 63.9 units/mL DNA polymerase, in the DNA Thermal Cycler™ (Perkin-Elmer/CETUS, Emeryville, CA). The samples were heated for 5 minutes at 94°C, then subjected to 40 cycles of a temperature program of: 1 minute at 94°C, 2 minutes at 40°C, and 1.5 minutes at 72°C. The PCR primers used in this case were PCR 1 and PCR5 of Example 2. Examination of the gel following electrophoresis showed bands at the expected positions, i.e. 292 bp. Lane 1, HPV6; lane 2, HPV 11; lane 3, HPV16 ; lane 4, HPV 18; lane 5, HPV31; lane 6, HPV33, lane 7, HPV61; lane 8, pooled human placental DNA ( suspected of having HPV infection); lane 9, molecular weight markers-Hae III digest of ΦX174.

FIG. 2 is a photograph of an ethidium bromide-stained 4% agarose gel showing results using 25 units/mL DNA polymerase, in the Programmable Cycler Reactor™ ( Ericomp, San Diego, CA). Samples in this case were subjected to 30 cycles of a temperature program of: 50°C for one (1) minute, 72°C for two (2) minutes and 95°C for one (1) ) minute. In this case, the primers PCR1 , PCR2, PCR3, PCR4 and PCR5 of Example 2 were used to amplify plasmid p65,16,8(HPV 16). Examination of the gel of Figure 2 shows bands at the expected positions, i.e., PCR 1 and PCR4, 235 bp, lane 2; PCR1 and PCR5, 267 bp, lane 4; PCR2 and PCR4, 254 bp, lane 6; PCR2 and PCR5, 286 bp, lane 8; PCR3 and PCR4, 174 bp, lane 10; PCR3 and PCR5, 206 bp, lane 12; molecular weight marker, 123, 246, 369, 492,... bp ladder, lane 1. Note footnote to Table 1.

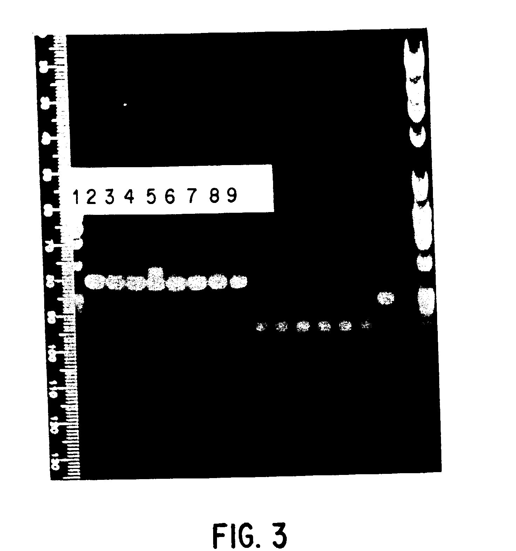

FIG. 3 is a photograph of an ethidium bromide-stained 1.2% agarose gel showing results using the same conditions as FIG. 1. In this case, PCR14 and PCR15 were used as primers in conjunction with IWDO. The expected size of the amplified PCR product of PCR 14 and IWDO is 437 bp for all of the HPV types tested. The expected size of the product of PCR 15 and IWDO is 98 bp. Products of these sizes appear in the gels, confirming that PCR14 and PCR15, used in conjunction with IWDO, will amplify HPV DNA of types 6, 11, 16, 18, 31 , 33, and 61. Lane 1, Molecular weight marker (Hae III digest of FX 174); PCR 14 + IWDO, lanes 2-9: lane 2, HPV6 ; lane 3, HPV 11 ; lane 4, HPV16 ; lane 5, HPV18 ; lane 6, HPV31; lane 7, HPV33; lane 8, HPV61 ; lane 9, human placental DNA suspected of being infected with HPV; PCR 5 + IWDO, lanes 10-17: lane 10, HPV6 ; lone 11 , HPV 11; lane 12, HPV16; lane 13, HPV18 ; lone 14, HPV31 ; lane 15, HPV33 ; lane 16, HPV61 ; lane 17, human placental DNA suspected of being infected with HPV; lane 18, molecular weight marker ( Hae III digest of FX174 and HinD III digest of 1 DNA) .

### EXAMPLE 4

The following reagents were mixed in a 0.5 mL polypropylene tube as follows for the Ligase Chain Reaction (LCR):

| Reagent | Volume | Final Concentration |
|---|---|---|
| Water | 21 µL | |
| Reaction Buffer | 10 µL | 50 mM EPPS pH7.8 |
| | | 10 mM NH₄Cl |
| | | 10 mM MgCl₂ |
| | | |
| | | 100 mM K⁺ (from all sources) |
| | | 0.001% BSA |
| | | 1 mM DDT |
| Nicotine Adenine Dinucleotide (NAD) | 0.5 µL | 100 µL |
| ProbeA (sense) | 4 µL | 5.0 x 10¹¹ molecules |
| ProbeA' (antisense, 5'-phosphate) | 4 µL | 7.5 x 10¹¹ molecules |
| ProbeB (sense, 5'-phosphate) | 4 µL | 7.5 x 10¹¹ molecules |
| Probe B' (antisense) | 4 µL | 5.0 x 10¹¹ molecules |
| Target (including human placental carrier DNA at 10 µg/mL) | 1.5 µL | 15 ng/50 µL |
| DNA ligase, Thermus therpophilus | 1 µL | |

This reaction mixture was overlayed with 30 µL of mineral oil. The tube was placed in an instrument capable of incubation at several temperatures ( e.g. thermal cycler from Coy Laboratory Products (Ann Arbor, MI) or the Programmable Cycler Reactor™ (available from Ericomp, San Diego, CA), and then subjected to several cycles of programmed temperature change. Each cycle involved incubation at 50°C for one minute and 85°C for one minute.

### EXAMPLE 5

The following procedure was used when performing the Ligase Chain Reaction ( LCR), which is described in published European Patent Application No. 0 320 308 A2. The reagents of Example 4 were utilized in the procedure as follows: Two probes (A and B) complementary to immediately adjacent to regions of a target sequence were hybridized and ligated. This ligated probe was denatured away from the target, and hybridized with two additional probes (A' and B') of sense opposite to the initial probes (A and B). The secondary probes then were ligated. Subsequent cycles of denaturation/hybridization/ligation created the formation of double-length probes of both + and - sense.

### EXAMPLE 8

The following sequences were determined to be specific for a portion of the E6 region of HPV type 16:

Probe | SEQ ID No. | Sequence | Maps to:
LCR5A | 81 | GCTGCAAACA ACTATACATG ATATAA | 157 - 182
LCR5A' | 82 | pTTATATCATG TATAGTTGTT TGCAGC | 182 - 157
LCR5B | 83 | pTATTAGAATG TGTGTACTGC AAGCA | 183 - 208
LCR5B' | 84 | TGCTTGCAGT ACACACATTC TAATA | 208 - 157

### EXAMPLE 9

Base-denatured plasmids which contained full-length papilloma virus inserts in pGEM3 were used as targets. These plasm ids were pG3HPV6(+) (HPV6), pSP 65. 11.5 (HPV11), pSP65.168 (HPV16), p63HPV18H(-)(HPV18), p63:HPV31 (HPV31), pLNK322:HPV33 (HPV33), pBR322:HPV35 (HPV35), pUC19:HPV52 (HPV52), pLNK322:HPV58 (HPV58), pUC9:HPV59 (HPV59) and PBR322:HPV61 (HPV61). All of the oligonucleotides used as probes from Example 8 had chemical labels covalently attched at the ends distal from ligation. These labels were: 5'-fluorescein-LCRSA, 3'-fluorescein-LCRSA', 3'- biotin-LCR5B and 5'-biotin-LCR5B'. Covalent attachment was performed by known methods, i.e., reaction of amine-terminated oligonucleotides with FITC or biotin-NHS-ester essentially following the procedures of Kansal et al.,Tet. Letters 29:5537-5540 (1988). The thermal cycler used was obtained from Coy Laboratory Products, Ann Arbor, MI.

Following the LCR procedure of Examples 4 and 5, the mixtures were analyzed using a prototype version of the IMₓ® instrument (Abbott Laboratories, Abbott Park, IL), following the protocol for microparticle enzyme immunoassays as follows. A 40µL aliquot of an LCR mixture was diluted 1:1 with distilled water. This diluted mixture was incubated with 50µL antifluorescein-conjugated polystyrene microparticles for five (5) minutes to form a suspension of immune complexes on the microparticles. This suspension then was transferred to an inert glass fiber matrix, to which the microparticles became attached. The matrix was washed with buffer (0.3M Nacl, 10 mM TRIS pH8, 0,1%NaN₃), Any immune complexes attached to the glass matrix was detected by using alkaline phosphatase-labeled conjugate that catalyzed the hydrolysis of 4-methylumbelliferone. The rate at which the 4-methylumbelliferone was generated on the matrix was proportional to the concentration of LCR product formed in the reaction mixture.

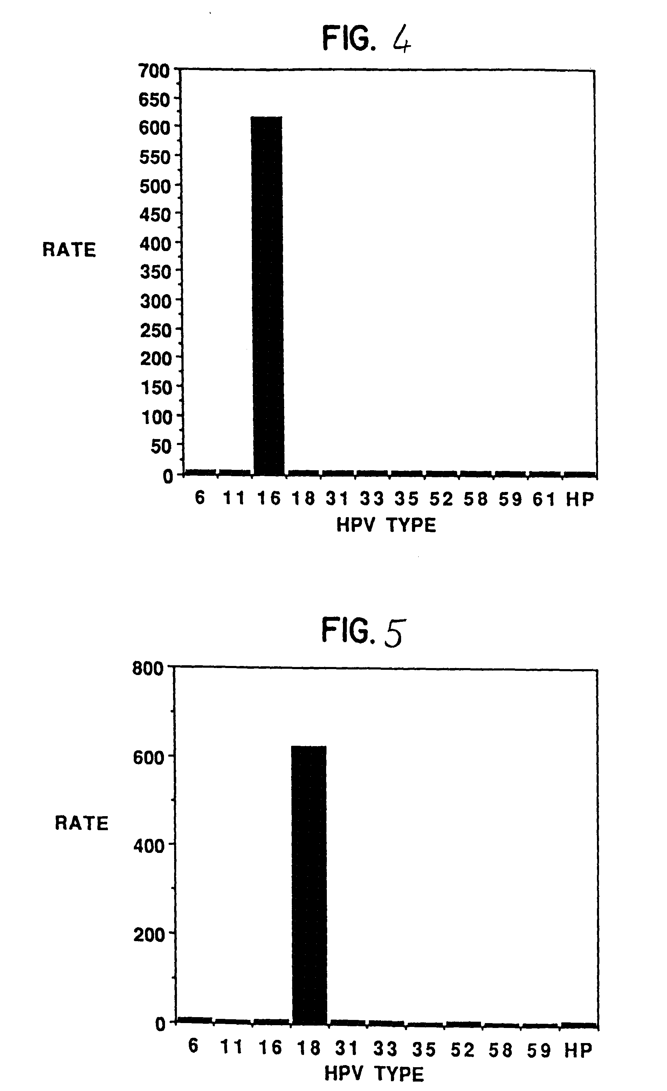

Referring to FIG. 4, the graph shows the results obtained from performing LCR on 10⁷ molecules of the targets in shown. The rate shown is the rate of generation of 4-methylumbelliferone, and is expresssed as fluorescence counts/second/second. Background signal is approximately 10 c/s/s, as shown by the amplification of human placental DNA. The only values above background are those for sample containing HPV16, and those values are about 60 times background signal.

### EXAMPLE 10

The following sequences were determined to be specific for a portion of the E6 region of HPV type 18:

Probe | SEQ ID No. | Sequence | Maps to:
LCR6A | 85 | CTTCACTGCA AGACATACAA ATAA | 172 - 195
LCR6A' | 86 | pTTATTTCTAT GTCTTGCAGT GAA | 195 - 173
LCR6B | 87 | pCCTGTGTATA TTGCAAGACA GTAT | 196 - 219
LCR6B' | 88 | TACTGTCTTG CAATATACAC AGG | 218 - 196

### EXAMPLE 11

Plasmids which contained full-length papilloma virus inserts in pGEM3 were used as targets. The plasmids used were those described in Example 9 . All of the oligonucleotides used as probes obtained from Example 10 had chemical labels covalently attached at the ends distal from ligation. The thermal cycler was obtained from Coy Laboratory Products, Ann Arbor, MI.

Following LCR procedure described in Examples 4 and 5, the mixtures were analyzed as described in Example 9 using the prototype version of the IMₓ® instrument (Abbott Laboratories, Abbott Park, IL).

Referring to FIG. 5, the graph dislays the results obtained from performing LCR on 10⁷ molecules of the targets. The rate shown is the rate of generation of 4-methylumbelliferone, and is expressed as fluorescence counts/second/second. Background signal is approximately 15 c/s/s, as shown by the amplification of human placental DNA. The only values above background are those for sample containing HPV 8, and those values are about 40 times background signal.

### EXAMPLE 12

The following sequences were determined to be specific for a portion of the E6 region of HPV type 18:

Probe | SEQ ID No. | Sequence | Maps to:
LCR7A | 89 | TATATTGCAA GACAGTATTG GAAC | 200 - 223
LCR7A' | 90 | pGTTCCAATAC TGTCTTGCAA TTTA | 223 - 200
LCR7B | 91 | pTTACAGAGGT ATTTGAATTT GCATT | 224 - 249
LCR7B' | 92 | AATGCAAATT CAAATACCTC TGTAA | 249 - 224

### EXAMPLE 13

Plasmids which contained full-length papilloma virus inserts in pGEM3 were used as targets. The plasmids were those of Example 9 All of the oligonucleotides from Example 12 which were used as probes had chemical labels covalently attached at the ends distal from ligation. The thermal cycler was as described in Example 11.

Following the LCR procedure of Examples 4 and 5, the mixtures were analyzed as described in Example 9 using the prototype version of the IMx instrument (Abbott Laboratories, Abbott Park, IL).

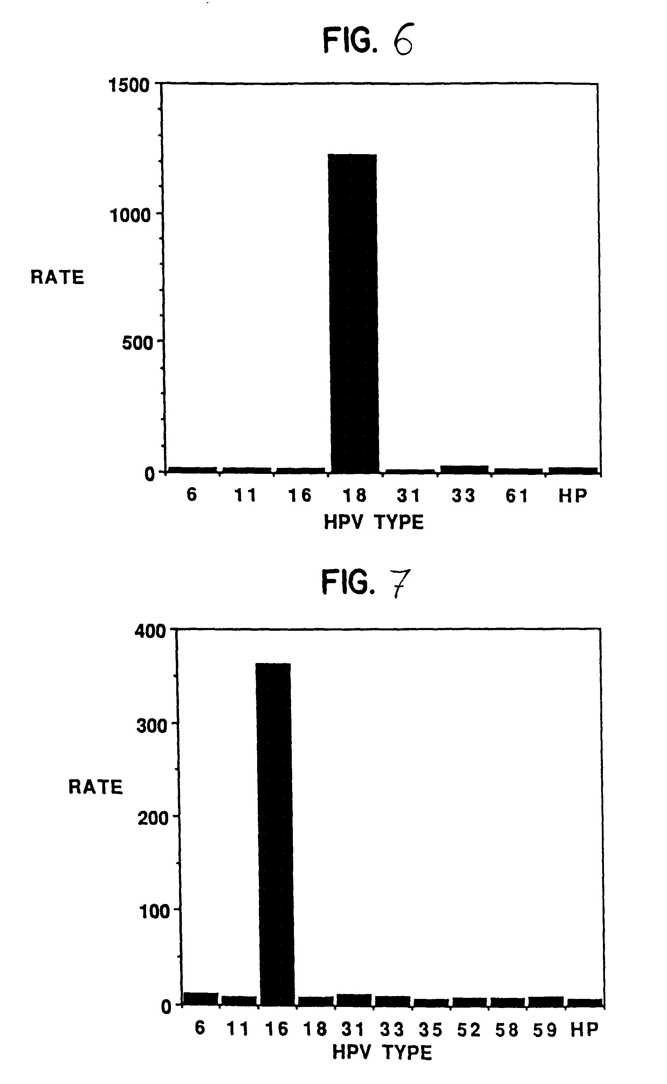

Referring to FIG. 6 , the graph shows the results obtained from performing LCR on 10⁷ molecules of the targets. The rate shown is the rate of generation of 4-methylumbelliferone, and is expressed as fluorescence counts/second/second. Background signal is approximately 15 c/s/s, as shown by the amplification of human placental DNA. The only values above background are those for sample containing HPV 18, and those values are about 80 times background signal.

### EXAMPLE 14

The following sequences were determined to be specific for a portion of the E6 region of HPV type 16.

Probe | SEQ ID No. | Sequence | Maps to:
LCR8A | 93 | GTATGGAACA ACATTAGAAC AGCA | 352 - 375
LCR8A' | 94 | pTGCTGTTCTA ATGTTGTTCC ATAC | 375 - 352
LCR8B | 95 | pATACAACAAA CCGTTGTGTG ATTT | 376 - 399
LCR8B' | 96 | AAATCACACA ACGGTTTGTT GTAT | 399 - 376

### EXAMPLE 15

Plasmids which contained full-length papilloma virus inserts in pGEM3 were used as targets. All of the oligonucleotides from Example 14 used as probes had chemical labels covalently attached at the ends distal from ligation. The thermal cycler was as described in Example 11.

Following LCR procedure of Examples 4 and 5, the mixtureswere analyzed as described in Example 9 using the prototype version of the IMₓ® instrument (Abbott Laboratories, Abbott Park, IL).

Referring to FIG. 7 , the graph details the results obtained from performing LCR on 10⁷ molecules of the targets. The rate shown is the rate of generation of 4-methylumbelliferone, and is expressed as fluorescence counts/second/second. Background signal is approximately 10 c/s/s, as shown by the amplification of human placental DNA. The only values above background are those for sample containing HPV 16, and those values are about 36 times background signal.

### EXAMPLE 16

The attached Appendix (example 16) discloses the sequences of the invention aligned to known sequences.

### EXAMPLE 16

### APPENDIX

### HUMAN PAPILLOMA VIRUS

### ALIGNMENT of TYPES 6, 11, 16, 18, 31, and 33; with CONSENSUS SEQUENCE

The appendix lists the sequences of HPV types 6, 11, 16, 18, 31, and 33. It also shows where the sequences of this invention line up with respect to these HPV sequences. In addition, the appendix shows where other sequences, known to the Inventors as of 28 September 1990, and claimed or disclosed by or unknown to others, line up with respect to these sequences.
*1. Sequences and Regions Claimed by Us;*
   - PCR =: Sequences per examples 1 through 3 (only PCR1, PCR5 PCR14 and PCR15)
   - LCR =: Sequences per examples 4 through 14 only
2. Sequences and Regions Unknown to Others and Not Claimed by Us;
   - PCR =: Sequences designated PCR other than those above JJ
   - LCR =: Sequences designated LCR other than those above
3. *Sequences and Regions Claimed by Others;*
   *(Italics represents antisense sequences)*
   - AUS =: International application number (Australians) PCT/AU88/00047 (WO 88/06634)
   - WL =: International application number (Wayne Lancaster, Wayne State University) PCT/US86/00629 (WO 86/05816)
   - BE =: European Patent Application (Belgians) 89.033834 (X= T or U)
   - C =: International application number (CETUS) PCT/US89/03747 (WO 90/C2821)
   - O =: International application number (Oncor) PCT/US89/O1318 (WO 89/09940)
   *and*
*4. Sequences and Regions Disclosed by Others.*
   - S =: Sarkar, F.H. and Crissman. J.D. *Biotechniques* **9** 180-184 (1990) *(Italics represents antisense sequences)*

6    1      gTTAATAACAATCTTgGTTTAA AAAAtAGGAGGG    ACCGAAA ACGGTTCAACCGAAAA
           ||||||||||||| |||||| |||| |||||||    ||||||| |||||||||||||||
11   1      cTTAATAACAATCTTAGTTTAA AAAAgAGGAGGG    ACCGAAA ACGGTTCAACCGAAAA
             | || | | ||||| | |||| || ||||    ||||||| ||||||||||||||
33   1     gtaaACTATAATgCcaAGTTTA AAAA AGtAGGGtGTAACCGAAA gCGGTTCAACCGAAAA
             || |||||   | | || |||   |||| |||||||||| ||||| ||||||||
16   1      actACAATAAT  tcAtGTATA AAA  ctaAGGGcGTAACCGAAA tCGGTTGAACCGAAAc
             | ||||||     ||||| |||   |||| || |||||||   | ||||||||||
31   1   TAATA ATAATAAT  ctTAGTATA AAA AAgtAGGGAGTgACCGAAA  GtGgTGAACCGAAAA
         ||||| |  |      ||||||| ||| ||  ||||||| |||||||  |    | ||||||||
18   .1 atTAATActTttaAcaattgTAGTATAtAAA AA  AGGGAGTaACCGAAAacGgtcgGgACCGAAAA

con     --taatata-ta-aa-tcttag-T-tA-AAAaaag-AGGGagtaACCGAAA-acggtt-aACCGAAAa
                                                          C4-GCCAASTTGGCTTTT
                                                          C5-GCCAGCCCTGGCTTTT
                                                          C36-CGGTTSAACCGAAAA
                                                          C37-CGGTCGGGACCGAAAA
                                                          C38-CGGTTSAACCGAAAM
                                                          C39-CGGTTCAACCGAAAM
   O15-ATTAATACTTTTAACAATTGTAGTATATAAA AA  AGGGAGTAACCGAAAACGGTCGGGACCGAAAA-O15
   O24-     ACTACAATAAT  TCATGTATA AAA  CTAAGGGCGTAACCGAAA TCGGTTGAACCGAAAC-O24
                                                          S1-CGGTCGGGACCGAAAA
                                                                 S3-ACCGAAAC

6   58 CGGTTgTATATAAA CCAGCCCtAAAAtTTAGCAaACGAGGCATTATGGAAAGTgcAaATGCCTCCAC
       ||||| |||||||| ||||||| |||| |||||| ||||||||||||||||||    | ||||||||||
11  58 CGGTTaTATATAAA CCAGCCCAAAAAaTTAGCAGACGAGGCATTATGGAAAGTaAAGATGCCTCCAC
       ||||         ||  ||   ||          ||      |||  |       | ||||   |
33  62 CGGTgcaTATAtAAAGCA aACATTTTgcagtaAGgtActGCACgACtATGTTTCAAGACaCtgAGGA
       ||||   |||| ||||||  |||||||          |  | ||  | |||||||| ||| |   ||||
16  58 CGGTTaGTATA AAAGCA gACATTTTaTGcaCcAAaAgAGAACtGCaATGTTTCCacAGGA
       ||||| ||||| ||||||        | || ||  | ||   | |   || |||||  |  | ||    ||
31  60 CGGTTgGTATATAAAGCAcataGTaTTtTGtgCaAAccTACAgacGCcATGTTcaAaaATCCtgCAGA
       ||||   |||||||||          |           | |   |||     ||     ||  |  ||||  ||
18  66 CGGT  GTATATAAA agatgtGagaaacacaCcAcaaTACtatgGCgcgcTTtgAggATCCaaCAcg

con    CGGTt-gtatatAAagcag--ca-a--at--gcaaaca-agcatt-cgatgttt-aagAtcC--c-ga
       GCC-C4                                                  AUS1-ATGCCTCCAC
       GCC-C5
       CGG-C36                                                 C67-AAATCCTGCAGA
       CGG-C37                          C68-CCTACAGACGCCATGTTCA-C68
       CGG-C38                C71-GCAGTAAGGTACTGCAC-C71
       CGG-C39                                                 O10-GGATCCAACACG
   O15-CGGT  GTATATAAA AGATGTGAGAAACACACCACAATACTATGGCGCGCTTTGAGGATCC-O15
   O24-CGGTTAGTATA AAAGCA GACATTTTATGCACCAAAAGAGAACTGCAATGTTTCCACAGGA(-O24)
       CGGTG-S1                                   S2-CCGCGCGAAACTCCTAGGTTGTGC-S2
       CGGTTAGTATA AAAGC-S3

6 125 GTCTGCAACgaCcATAGACCAGTTGTGCAAGACGTTTAATCTaTCTaTGCAtACgtTGCAAATTaAtT
11 125 GTCTGCAACAtCtATAGACCAGTTGTGCAAGACGTTTAATCTtTCTtTGCACACtcTGCAAATTCAGT
33 129 aaaACCAcGAACaTTgCAtgAtTTGTGCcAAGCaTTGgAgACAACTATACACAAcATtgAAcTACAGT
16 124 gcGACCcaGAAAgTTaCcacAgTTATGCaCAGagcTGcAAACAACTATACATGAtATAAtATTAgAaT
31 128 aaGACCtcGgAAaTTgCaTGAaCTAaGCtCGGcAtTGgAAAtAcCctacgATGAacTAAgATTgAAtT
18 131 gcGACCctacAAgcTaCcTGAtCTgtGCaCGGaAcTGaAcActtCactgcAaGAcaTAgaAaTaAccT

con g-gacCaagaa--tTacat-AgtTgtGCa-ggc-tTgaA-a-atCtatgcAt-a-aTa-aAaTaaa-T
GTCTGCAAC-AUS1 AUS7-GCAAGACGTTTAATCT-AUS7
AAGACCTC-C67 C74-ACACTCTGCAAATTCAGT
O10-GCGACCCTACAAGCTACCTGATCTGTGCACGGAACTGAACACTTCACTGCAAGACATAGAAATAACCT-O10
O24-GCGACCCAGAAAGTTACCACAGTTATGCACAGAGCTGCAAACAACTATACATGATATAATATTAGAAT-O24
S4-CTGGGTCTTTCAATGGTGTCAATA-S4

6 193 GtGTGTTTTGCAaGAATGCACTGACCACaGCAGAGATtTATtCATATGCaTATAAacACCTAAAGGTc
11 193 GCGTGTTTTGCAgGAATGCACTGACCACcGCAGAGATATATGCATATGCcTATAAgaACCTAAAGGTT
33 197 GCGTGgAaTGCAAaAAACCtTTGCaaCGAtCTGAGGTATATGAtTTTGCaTTTgcaGATTTAAcaGTT
16 192 GTGTGTACTGCAAgcAACAGTTACtgCGAcgTGAGGTATATGAcTTTGCtTTTcggGATTTAtgcATA
31 196 GTGTcTACTGCAAaggtCAGTTAacAgaAACAGAGGTATTaGAtTTTGCATTTAcAGATTTAacaATA
18 199 GTGTaTAtTGCAAgacagtaTTggaActtACAGAGGTATTtGAaTTTGCATTTAaAGATTTAtttgTg

con GtGTgtatTGCAagaa--catTgacac-a-caGAGgTaTatgaaTtTGCaTtTaaagAttTAa--gT-
AUS2-TACGTGACTGGTGGCCGTCTC-AUS2 C73-ACACCTAAAGGTC
GC-C74 AUS3-TGAGGTATATGACTTTGCTTTT-AUS3
C60-GAGGTATWTGAHTTTGC-C60 O1-CTAAAGGTT
C61-GAGATWTATKCATATGC-C61 O2-CTAAAGGTT
C69-ACAGTATTGGAACTTACAG-C69 O4-GATTTCCAA
C70-CAACAGTTACTGCGACG-C70 O6-TTATGCATA
C72-GACAGTATTGGAACTTACAG-C70 O7-TTATGCATA
S5-GTGTTTTGCAGGAATGCACTGACCA-S5 O8-AATACGTAT
O10-GTGTATATTGCAAGACAGTATTCGAACTTACAGAGGTATTTGAATTTGCATTTAAAGATTTATTTGTG-O10
O11-TTATTTGTG
O12-TTATTTGTG
O13-AATAAACAC
O17-CTAAAGGTC
O18-CTAAAGGTC
O20-GATTTCCAG
O24-GTGTGTACTGCAAGCAACAGTTACTGCGACGTGAGGTATATGACTTTGCTTTTCGGGATTTATGCATA-O24
O25-TTATTTGTG

6 261 cTGTttCGAGgCggCTaTCCaTaTGCAGCcTGcGCgTGcTGCcTAGAAtTtCAtGGaAAAATaAACCA
11 261 GTGTggCGAGACaaCTtTCCcTTTGCAGCgTGTGCcTGtTGCTTAGAAcTgCAAGGgAAAATTAACCA
33 265 GTATATAGAGAgGGaAATCCATTTGgAaTATGTaAactgTGTTTgcgGTTcTtATCTAAAATTAGTGA
16 260 GTATATAGAGAtGGgAATCCATATGctGTATGTgAtAAATGTTTAAaGTTTTATTCTAAAATTAGTGA
31 264 GTATATAGgGACGacAcACCACAcGgaGTgTGTacaAAATGTTTAAgaTTTTATTCaAAAgTaAGTGA
18 267 GTgTATAGaGACagtAtACCcCAtGctGcaTGccatAAATGTaTAgatTTTTATTCtAgAaTtAGaGA

con gT-TataGaGacggcaatCCatatGcag-aTGtg--aaaTGttTagaatTttattctAaAaTtAgtgA
C-44CTCTGYCGWWAGGTAWACGW-C44 JJ1-aattagnga
C-45CTCTGTCATATGGCGTACGA-C45 AUS8-GTGA
C-46CCCTGCTGTGTGGTGTGCCT-C46 S6-GT
C-47CYCTGCYGWWWGGTAWACSW-C47
C-48CYCTGYYGWWAGGTAWACGW-C48
C-49CYCTGYYGWDWGGTAWACSW-C49
C56-MGAGACRGCWWTCCATWTG-C56
C57-MGAGACRGSWWTCCATWTG-C57
C58-MGAGACRGVWWTCCATWTG-C58
C59-AGAGACAGTATACCGCATG-C59
GTGTGGCGAGACAACTTTCCCTTTGCAGCGTGTGCCTGTTG-O1
GTGTGGCGAGACAACTTTCCC-O2
O3-CAACTTTCCCTTTGCAGCGTGTGCCTGTTG-O3
CACACCGCTCTGTTGAAAGGGAAACGTCGCACACGGACAAC-O4
GTATATAGAGATGGGAATCCA-O6
GTATATAGAGATGGGAATCCATATGCTGTATGTGATAAATG-O7
CATATATCTCTACCCTTAGGTATACGACATACACTATTTAC-O8
O9-ACCCTTAGGTATACGACATACACTATTTAC-O9
O10-GTGTATAGAGACAGTATACCCCATGCTGCATGCCATAAATGTATAGATTTTTATTCTAGAATTAGAGA-O10
GTGTATAGAGACAGTATACCG-O11
GTGTATAGAGACAGTATACCCCATGCTGCATGCCATAAATG-O12
CACATATCTCTGTCATATGGGGTACGACGTACGGTATTTAC-O13
O14-GTCATATGGGGTACGACGTACGGTATTTAC-O14
O17-CTGTTTCGAGGCGGCTATCCA-O17
O18-CTGTTTCGAGGCGGCTATCCATATGCAGCCTGCGCGTGCTG-O18
O19-GCCGATAGGTATACGTCGGACGCGCACGAC-O19
GACAAAGCTCCGCCGATAGGTATACGTCGGACGCGCACGAC-O20
O24-GTATATAGAGATGGGAATCCATATGCTGTATGTGATAAATGTTTAAAGTTTTATTCTAAAATTAGTGA-O24
GTGTATAGAGACAGTATACCG-O25
O26-CAGTATACCCCATGCTGCATGCCATAAATG-O26

6 329 ATATAGACACTTTgATTATGCTGgATATGCAaCaACAGTtGAAGAAGAAACtAAacAAGAcATcTTAg
11 329 ATATAGACACTTTAATTATGCTGcATATGCAcCTACAGTAGAAGAAGAAACcAAtgAAGATATtTTAa
33 333 ATATAGACATTATAATTATtCTGTATATGGAAaTACATTAGAACAAacAgttAAaAAACCTTTaaaTG
16 328 gTATAGACATTATtGTTATAGTtTGTATGGAACAACATTAGAACAgcaAtacAACAAACCgTTgTGTG
31 332 ATTTAGAtggTATaGATATAGTGTGTATGGAACAACATTAGAAAAAtTgACaAACAAAGGtaTATGTG
18 335 ATTaAGAcatTATtcAgActcTGTGTATGGAgacACATTgGAAAAAcTaACtAACAct GGgtTATaca

con aTatAGAcatTaTaattAt-cTgt-TATGgAacaACAtTaGAA-Aa-aaactAAcaaag-t-Tat-tg
atatagacatt-JJ1
GTATAGACATTAT-AUS8
C50-ATAHSACAYATACSTTGWTGTMATCTT-C50
C51-ATAHSACAYATACSTTGWTGTMATC-C51
C52-ATAHSACAYATACSTTGWTGTMAT-C52
C53-CTGAGACACATACCTCTGTGTGTAACC-C53
C54-CTGAGACACATACCTCTGTGTGTAA-C54
C55-CTGAGACACATACCTCTGTGTGTA-C55
O10-ATTAAGACATTATTCAGACTCTGTGTATGGAGACACATTGGAAAAACTAACTAACACTGGGTTATACA-O10
O24-GTATAGACATTATTGTTATAGTTTGTATGGAACAACATTAGAACAGCAATACAACAAACCGTTGTGTG-O24
TATATCTGTGAAATTAATACGAC-S6

6 397 AcGTGcTAATTCGgTGcTACCTGTGTCACAAaCCGcTGTGTGAAgTAGAAAA ggTAAAaCAtATAcT
11 397 AAGTGTTAATTCGtTGTTACCTGTGTCACAAgCCGTTGTGTGAAaTAGAAAAA cTAAAgCAcATAtT
33 401 AAaTaTTAATTAGGTGTATTATaTGTCAAAgaCCtTTGTGTCCTcAAGAAAAAaAAcGACATgTGGAT
16 396 ATTTGTTAATTAGGTGTATTAacTGTCAAAagCCacTGTGTCCTGAAGAAAAgCAAAGACATcTGGAc
31 400 ATTTGTTAATTAGGTGTATaAcGTGTCAAAgACCGTTGTGTCCAGAAGAAAAACAAAGACATtTGGAT
18 403 ATTTaTTAATaAGGTGccTgcgGTGcCAgAaACCGTTGaaTCCAGcAGAAAAACttAGACAccTtaAT

con AttTgtTAATtaGgTGtat--tgTGtCAaAaaCCgtTGtgTccagaAGAAAAaca-agAcatct--at
AUS4-AATTAATCCACATAAT-AUS4 AUS9-GATTTATTTG
AUS5-TGTCATAACCTTGAATGTCT-AUS5
O10-ATTTATTAATAAGGTGCCTGCGGTGCCAGAAACCGTTGAATCCAGCAGAAAAACTTAGACACCTTAAT-O10
O24-ATTTGTTAATTAGGTGTATTAACTGTCAAAAGCCACTGTGTCCTGAAGAAAAGCAAAGACATCTGGAC-O24

6 464 aaccAAGGCgCGgTTCATAAA gCTAAATtgtacGTGGAAGGG TCGcTG
11 464 gggAAAGGCaCGcTTCATAAAA CTAAATaaCcaGTGGAAGGG TCGTTG
33 469 ttAAAcaAAcGATTTCATAATAT TtcGGGTCGtTGGGCAGGGCGcTGTgcGgCgTGTTG
16 464 AAAAAGcAAaGATTCCATAATATA aGGGGTCGGTGGACcGGtCGaTGTATGtCtTGTTG
31 468 AAAAAGaAACGATTCCACAACATAG GaGGAaGGTGGACaGGaCGtTGCATagCaTGTTG
18 471 gAAAAacgACGATTtCACAACATAGctgggcactataGAgGccaGtgccattcgTGCtgcaaccGagc

con aaaaAa--acgatTtCAtAA-atag------ctaaaggacg-tgGgcagggcg-tgcatggct-Gttg
TGGTGTATAGA-AUS9 AUS6-AAATGTATAGATTTTTATTC-AUS6
C65-CAACCGAGC
O10-GAAAAACGACGATTTCACAACATAGCTGGGCACTATAGAGGCCAGTGCCATTCGTGCTGCAACCGAGC-O10
O24-AAAAAGCAAAGATTCCATAATATA AGGGGTCGGTGGACCGGTCGATGTATGTCTTGTTG-O24

6 512 CcTACACTGC TGGACAACATGCATG GAAGACaTGT
11 512 CtTACACTGC TGGACAACATGCATG GAAGACtTGT
33 528 gaggtcccgACGTAGAGAAACTGCactgtgAcgTGTAAAAacgcCATGagagGACACaagcC
16 523 cagatcatcAAGAaCACGTAGAGAAAC CCAgcTGTAA tCATGCATGGAGAtACAC C
31 527 GagAAGACCtCGTActGAAAC CCAagTGTAA aCATGCgTGGAGAaACAC C
18 539 acgacaGgaAcGACtcCaacgacgcAgagaaacaCaAgtataAtattAaGtaTGcAtggACctaaggC

con --ga--gagaagaccacgta-aga-Actgca---ccaggtgtAaaacatgcaTGgagagAcacaaggc
C64-GAACACGTAGAGAAAC CCAG-C64
ACGACAGGA-C65
C66-GAGGTCCCGACGTAGAGAA-C66
O10-ACGACAGGAACGACTCCAACGACGCAGAGAAACACAAGTATAATATTAAGTATGCATGGACCTAAGGC-O10
O24-CAGATCATCAAGAACACGTAGAGAAAC CCAG-O24

6 547 TACCCTAAAGGA TATtGTAtTAGACCTGCAaCCTCCaGACCCTGTAGGGTTACATTGCTATG
11 547 TACCCTAAAGGA TATaGTAcTAGACCTGCAgCCTCCTGACCCTGTAGGGTTACATTGCTATG
33 590 aACgTTAAAGGA ATATGTtTTAGA TTTatATCCTGAaCCAACTGAcCTATACTGCTATG
16 579 TACaTTGCAtGA ATATaTGTTAGA TTTGCAACCaGAGaCAACTGAtCTCTACTGTTATG
31 577 TACgTTGCAAGAC TATgTGTTAGA TTTGCAACCtGAGgCAACTGACCTCCACTGTTATG
18 607 aACaTTGCAAGACattgtaTtgcatTTAGAgccccaaaAtgaaattcCggtTGACCTtCtaTGTcAcG

con tAC-tT--AgGAc-----at-tgt-tTAGAcctt---catcc-ga-cCa--tGaccTacacTG-tAtG
BE16-ACCAGAGACAACXGAXCXCXACXGX-BE16
BE18-GXXAGAXXXGCAACCAGAGACAACXGAXCXCXAC-BE18
O10-AACATTGCAAGACATTGTATTGCATTTAGAGCCCCAAAATGAAATTCCGGTTGACCTTCTATGTCACG-O10
C89-G
C90-G

6 609 AGCAATTAGtAGACAGCTCAGA AGATGA GGTGGACgAaGTGGACggACAAGAttCACAACCT
11 609 AGCAATTAGaAGACAGCTCAGA AGATGA GGTGGACaAGGTGGACaaACAAGAcGCACAACCT
33 649 AGCAATTAAgTGACAGCTCAGAtGAGGATGAaGgctTGGACcGGCCAGATGGACAA GCACAACCa
16 638 AGCAATTAAaTGACAGCTCAGAgGAGGAGGATGaaATAGAtgGTCCAGCTGGACAA GCAGAACCG
31 636 AGCAATTAccCGACAGCTCAGAtGAGGAGGATGtcATAGAcaGTCCAGCTGGACAA GCAGAACCG
18 675 AGCAATTAagCGACtcagagGAaGAaaAcGATGaaATAGA tggagttaatcatcaacatttAcCaG

con AGCAATTAa--GACagctcaGAtga-gAtGAtga-aT-GAc-gg-c-gatggacaagacgcacAaCcg
AGCAATTAGWAGAC-C89 BE8-GACGAAGXGGACGGACAAGAXXC-BE8
AGCAATTAARYGAC-C90 BE9-GA---GGXGGACGAAGTGGACGGACAAGATTCACAACC-BE9
BE13-XGA---GGXGGACAAGGXGGACAAAC-BE13
BE14-AGAAGAXGA---GGXGGACAAGGXGGACAAACAAGACG-BE14
BE15-CAGAACCG
BE17-ACAAGCAGAACCG
C62-CGAAGTGGACGGACAAGAT-C62
C63-CAAGGTGGACAAACAAGACG-C63
O10-AGCAATTAAGCGACTCAGAGGAAGAAAACGATGAAATAGA TGGAGTTAATCATCAACATTTACCAG

6 671 TTAAaACAACATTtCCAAATAgTGACCTGTTG CTGTGGATGTGAC AGCAACGTtCGA
11 671 TTAACACAACATTACCAAATAcTGACCTGTTG CTGTGGATGTGAC AGCAACGTcCGA
33 714 GcCACAGCtgATTACtAcATTGTAACCTGTTGT caCActTGTaAC ACCACaGTTCGt
16 703 GACAgAGCCcATTACAATATTGTAACCTTTTGTTG CAAGTGTgACT CTACgCTTCGg
31 701 GACAcAtCCaATTACAATATCGTtACCTTTTGTTGT cAGTGTAAGT CTACACTTCGt
18 741 ccCgacgagccgaACcAcAaCGTcACacaaTGTTGTgtatgtgtTGTAAGTgtgaagCcAgAaTTgag

con g-cacag--cattaCcA-At-gT-ACctgtTGttgt-ctg--g-TGT-Act-----accAcagTtcg-
GACAGAGCCCAX-BE15 BE19-AGXGXGACX------CXACGCXXCGG
GACAGAGCCCA-BE17 BE20-XXGCAAGXGXGACX------CXACGCXXCGG
BE24-XXGXAAGXGXGAAGCCAGAAXXGAG
BE25-AXGXGXXGXAAGXGXGAAGCCAGAAXXGAG
O10-CCCGACGAGCCGAACCACAACGTCACACAATGTTGTGTATGTGTTGTAAGTGTGAAGCCAGAATTGAG-O10

6 728 CTGGTTGTGcAGTGtACAGAaacAGACATCAGAgAAgTgCAAcAgCTTcTGtTGGGaACACTAAAcAT
11 728 CTGGTTGTGgAGTGcACAGAcgGAGACATCAGAcAAcTACAAgAcCTTtTGcTGGGCACACTAAAtAT
33 771 TTaTGTGTcaAcACtACAGcaaGtGACcTaCGAACcaTACAgcAaCTacTtATGGGCACAgTgAATAT
16 760 TTGTGcGTACAaAGCACACAcGTAGACATTCGtACtTTGgAAGAcCTGTTAATGGGCACAcTaGGAAT
31 758 TTGTGtGTACAgAGCACACAAGTAGAtATTCGcAtATTGCAAGAGCTGTTAATGGGCtCAtTtGGAAT
18 809 cTagtaGTAgAaAGCtCAgcAGacGAccTTCGagcATTcCAgcAGCTGTTtcTGaaCaCccTgtcctT
con -Tg--tGTacAgaGcaCAgaag-aGAcaTtcGaacatTgcAa-AgCTgtT-aTGggcaCacTaaa-aT
XXG-BE19 BE29-AGCAAGXGACCXACGAACCAXACA-BE29 C42-CCCGTGTGAYYYDTA
XXGXGCGXAC-BE20 C43-CTTGTGGGACAGGAA
CXAGX-BE25
BE30-AGXACAGCAAGXGACCXACGAACCAXACAGCAACX-BE30
O10-CTAGTAGTAGAAAGCTCAGCAGACGACCTTCGAGCATTCCAGCAGCTGTTTCTGAACACCCTGTCCTT-O10

6 796 aGTGTGTCCCATCTGCGC AC CgAAgaCcTAACAAcGATGGCGGACGATTCAGGTACAGAAAAT
11 796 TGTGTGTCCCATCTGCGC AC CaAAACCaTAACAAgGATGGCGGACGATTCAGGTACAGAAAAT
33 839 TGTGTGCCCtAcCTGTGC ACAacAAtAAACATCAtCtAcaATGGCcGATcCTGaAGGTACAaAtGgg
16 828 TGTGTGCCCCAtCTGTTCT CAgAAACcATAATCTACcATGGCTGATCCTGCAGGTACcAATGGGGAa
31 826 cGTGTGCCCCAaCTGTTCT aCtAgACtGTAA CTACAATGGCTGATCCAGCAGGTACAGATGGGGA
18 877 tGTGTGtCCgtggTGtgC atCccagCaGTAAgCaACAATGGCTGATCCAGaAGGTACAGAcGGGGA
con tGTGTG-CCcatcTGtgCtaca-aaacaataatcaaCaAtg---G-t---g--gg---ta-ag-ggat
C40-CACACRGGGTAGACRCG-C40 C75-ATGGCKGAYCCTGMAGGTAC-C75
C41-CACACAGGCACCACACG-C41 C76-ATGGCKGAYGATTCAGGTAC-C76
ACACAC-C42 C77-ATGGCKGAYCCTTCAGGTAC-C77
ACACAC-C43 C81-TACCGMCTRGGACKTCCATG-C81
C82-TACCGMCTRCTAAGTCCATG-C82
C83-TACCGMCTRGGAAGTCCATG-C83
O10-TGTGTGTCCGTGGTGTGC ATCCCAGCAGTAAGCAACAATGGCTGATC-O10

6 859 GAGGGGTCtGGGTGTACAGGATGGTTTATGGTAGAAGCtATAGTgcAaCACcCaACAGG TAC
11 859 GAGGGGTCGGGGTGTACAGGATGGTTTATGGTAGAAGCcATAGTAGAGCACACtACAGG TAC
33 906 GctGGGAtGGGGTGTACTGGtTGGTTTgAGGTAGAAGCaGTcaTAGAGAgAAgAACAGG aGA
16 895 GaGGGtACGGGATGTAATGGaTGGTTTTATGTAGAgGCtGTAgTgGAaAaAAAAACAGG GGA
31 891 GGGgACGGGATGcAATGGtTGGTTTTATGTAGAAGCaGTAaTtGACAgAcAgACAGG GGA
18 943 GGGcACGGGtTGtAAcGGcTGGTTTTATGTAcAAGCtaTtgTaGACAaAaAaACAGGagatgtaat

con gagGGgacgGGgTGtA-tGGaTGGTTTta-GTAgAaGCt-TagTagA-aaaaaaACAGG--------a
C78-TGTAMWGGMTGGTTTTATGT-C78
C79-TGTAMWGGMTGGTTTGAGGT-C79
C80-TGTAMWGGMTGGTTTATGGT-C80
C84-ACATKWCCKACCAAAATACA-C84
C85-ACATKWCCKACCAAACTCCA-C85
C86-ACATKWCCKACCAAATACCA-C86

6 921 ACAAATATCAGAcGATGAGGAtGAGGAGGTGGAGGACAGTGGGTATGACATGGTGGACTTTATTGATG
|||||||||||| |||||||| ||||||||||||||||||||||||||||||||||||||||||||||
11 921 ACAAATATCAGAAGATGAGGAaGAGGAGGTGGAGGACAGTGGGTATGACATGGTGGACTTTATTGATG
| || |||||||||||||| || | || |||||||| || | | || ||||| ||||
33 968 TaATATtTCAGAAGATGAGGAtGAAAcaGcaGATGACAGTGGcacgGATTTacTAGAgTTTATAGATG
| ||| ||||| || ||| | |||| | ||| | || ||||| |||| |||||||
16 957 TgcTATaTCAGAtGACGAGAAcGAAAAtGacAGTGATACaGGtGAaGATTTGGTAGAtTTTATAGtaA
|| ||||| ||||| || ||| | |||||||| || || ||| |||| || ||||| | |
31 951 caacATTTCAGAGGACGAAAATGAAgACAGtAGTGATACtGGGGAGGATATGGTtGAcTTTATTGAcA
| ||||||||||||| |||| | || ||| ||||||||| || |||||||| |
18 1009 atcagaTgacGAGGACGAAAATG caACAG AcACaGGGtcGGATATGGTaGAtTTTATTGAtA

con a-aaat-tcaGA-GA-GAg-AtGaa-a-g-ggatgAcA-tGGgtagGAtaTggTaGAcTTTATtGat-

6 989 A CAGcaATATTACA CAcAATTCacTGGAAGCACAGGCATTGTTTAAcAGGCAGGAGGCG
| ||| |||||||| || ||||| |||||||||||||||||||||| |||||||||||
11 989 A CAGgcATATTACA CAAAATTCtGTGGAAGCACAGGCATTGTTTAATAGGCAGGAGGCG
| | | |||| | | | | || ||| | |||||||||||||| ||||| | |
33 1036 ATtcTAtgGAAaATAgtATACAGGCAGAcACAGAGGCAGCcCgGGCATTGTTTAATAtACAGGAAGgG
|| || || || | ||||||||| |||||| |||| | || ||||||| | ||||||||
16 1025 ATgaTAATGAtTATttaAcACAGGCAGAAACAGAGACAGCACAtGCgTTGTTTAcTGCACAGGAAGCa
|| ||||| || | ||||||||| ||||||||||||| || |||||| |||||||||||
31 1019 ATtgTAATGtATAcaacAAtCAGGCAGAAgCAGAGACAGCACAGGCATTGTTTCATGCACAGGAAGCg
|| | |||||||| |||||||||||||||||||| ||||| ||||| |
18 1071 cacaaggaacATtttgtgAaCAGGCAGAgctAGAGACAGCACAGGCATTGTTcCATGCgCAGGAgGtc

con attataatgcatatataataCAggcagA--cagaG-cAGCaCagGCaTTGTTtaat-c-CAGGA-Gcg

6 1048 GAcaCcCATTATGCGACTGTGCAGGACCTAAAACGAAAGTATTTAGGtAGTCCATATGTtAGTCCTAT
|| | |||||||||||||||||||||||||||||||||||||||| ||||||||||| ||||||||
11 1048 GAtGCTCATTATGCGACTGTGCAGGACCTAAAACGAAAGTATTTAGGcAGTCCATATGTaAGTCCTAT
|| | | | ||| ||||| | ||||||||||||| | |
33 1104 GAgGATgATTtAaATGCTGTGtgtGcaCTAAAACGAAAGT TTGCCgc
| | || | |||| || | ||||||||||||| | | |
16 1093 aAacAACATagAGATGCaGTaCAGGTTCTAAAACGAAAGT AT TtGGTAGTCCa
| ||||| ||| || || |||||||||||||||||| || | ||||||||
31 1087 gAggAACATGCAGAgGCtGTGCAGGTTCTAAAACGAAAGT ATgTaGGTAGTCCt
| | ||||| | | |||| ||| |||||||||||||
18 1139 cAcaAtgATGCAcAaGtgtTGCAtGTTtTAAAACGAAAGT ttgcaggaggcagcacaga

con gA-gatcATt-agaggctgTgcagGttcTAAAACGAAAGTatttagg-agtcca--tgtga-tgcc-t
BE1-XAAAACGAAAGX-BE1
BE2-AGGACCXAAAACGAAAGXAXXXAG-BE2
BE3-AGGXXCXAAAACGAAAGXAXXXGG-BE3
BE4-AXGXXXXAAAACGAAAGXXXGCAG-BE4

6 1116 AAaCAcTaTAGCcgAgGCAGTgGAAAGTGAaATAAGTCCACGaTTgGACGCCATTAAACTTACAAgAC
|| || | |||| | ||||| |||||||| |||||||||| || ||||||||||||||||| ||
11 1116 AAGCAaTgTAGCTaATGCAGTaGAAAGTGAgATAAGTCCACGgTTaGACGCCATTAAACTTACAAcAC
| | | | ||| | || || ||| | | || |
33 1151 ATGttcacaAagTGcTGCgGagGAcgtTGTtGAtcGTgCTgcaAacCCgtGtAgAAcgtCTATtAaTA
| | | | ||| || | | | || | || |||| ||
16 1146 cTtAGTGATATTAG TGGaTGTGTaGAcaATAATATTAGTCCtaGaTTAAAAGCTATATGTA
| ||||||||||| | | ||||| || ||||||||||||| | ||||||||||||| |
31 1141 tTAAGTGATATTAG TaGtTGTGTGGATtATAATATTAGTCCACGGTTAAAAGCTATATGcA
| ||||| |||||| | | ||||||||||||| ||| ||||
18 1198 aaAcagtccATTAGgggagcggctggagGTGGATacagAgtTaAGTCCACGGTTAcAAGaaATATctt

con a-aca-tatAttagaggcagtggaa-gtGtggatagt--t-taagtcc--g--taaaagctAta-gta

6 1184 AGCCAAAAAAGGTAAAGCGACGGCTGTTTcAAACcaGGGAAcTAACGGACAGTGGATATGGCTATTCT
||||||||||||||||||||||||||||| |||| ||||| ||||||||||||||||||||||||||
11 1184 AGCCAAAAAAGGTAAAGCGACGGCTGTTTgAAACAcGGGAAtTAACGGACAGTGGATATGGCTATTCT
| ||| | | | ||| | || || ||||| ||||||||| || ||
33 1219 AAaAtAAAgAAtGcAcAtacaGAAAAcGaAAAATAgaTGAgcTAGAAGACAGCGGATATGGCAATACT
| | ||| || | | | |||| | | | || ||| |||||||||| |||||||||||
16 1207 TAGAAAAAcAAAGTAgAgCtGCAAAAaGgAGAtTATTTGAAagcGAAGACAGCGGGTATGGCAATACT
||||||| | |||| | | |||||| | ||| | |||||| ||||||||||||||||||||||
31 1202 TAGAAAATaAcAGTAAAACaGCAAAAcGaAGACTcTTTGAAcTtcCAGACAGCGGGTATGGCAATACT
|| | | | |||| |||||| | | || ||| | | |||| || || |||||| | ||
18 1266 TAaAtAgTgggcagAAAAagGCAAAAaGgcGgCTgTTTacAaTatCAGAtAGtGGcTATGGCtgTtCT

con -a-aaAaaaag-g-Aaa--ag-aaaa-g-a-aatatttgaacta-caGAcAG-GGaTATGGC-aT-CT

JJ3-tatggctattct
C87-ATACCGTTAWGA
C88-ATACCGAYAWGA

6 1252 GAAGTGGAAGCTGgaacgggAACG CAGGTAGAGAAACA TGGCG
||||||||||||| |||| |||||||||||||| |||||
11 1252 GAAGTGGAAGCTG cAACG CAGGTAGAGAAACA TGGCG
||||||||| || | |||| |||| | |
33 1287 GAAGTGGAAACT CAGCAGAT GGTA cAACA GGTAG
|||||||||||| |||||||| | ||| |||||
16 1275 GAAGTGGAAACT CAGCAGAT G tTACA GGTAG
||||||||||| |||||||| | |||| |||||
31 1270 GAAGTGGAAAC gCAGCAGAT G gTACA GGTAG
||||||||| | ||||| |||| ||||
18 1334 GAAGTGGAAgC aacaCAGATtcaggtaacTACAaatggcgaacatggcggcaatgtatGTAG

con GAAGTGGAA-Ctg----gca-caGataggtagag--ACA----------------------GtaG
gaagtggaagctgnnnnncnacagat-JJ3
CTTCACCT-C87
CTTCACCT-C88

6 1295 taCCGGAAAATGG GGGAGATGGTCAGGAAAaGGA
||||||||||| ||||||||||||||||| |||
11 1289 A cCCGGAAAATGG GGGAGATGGTCAGGAAAgGGA
| ||||||| | | | || ||
33 1321 A — AagtcAAAATGG cgACAC AaActtaaAtGActtaGA
| | | |||| | | | | |
16 1306 A A gggcGccatgagactgAAACACcAtgtagtcAgtAtagtGg
| |||||
31 1301 A GGAG CAAC AAACA AC
|||| |||| |||| ||
18 1396 tggcggcagtacGGAGgctatagaCAACgggggcacagagggcAACA AC

con a------------ggag----aacgcaaaatgg----aga--gaaacacgagatggtcaggaaaggga

6 1329 CACAGGaAGGGACATAGAGGG GGaGGAACATAcAGAGGCGGAAGCgcccacaaACAGtgtaC
|||||| |||||||||||||| || |||||||| ||||||||||| |||| |
11 1323 CACAGGgAGGGACATAGAGGGTgagGgGGTGGAACATAgAGAGGCGGAAGCagtagacGACAGcaccC
| | ||| || | | | || | | || || |
33 1358 atCtAGTGGGGtgGGgGAtGaTtcaGAaGTaAGctGTgagacaaatGtAGaTagctGTGAAA
||||||| || | | || | | | | ||||||
16 1349 tggAAGTGGGGgtGGttgcagTcagtAcaGTAGTGGaagtggGGGAGagGgTgtTAGTGAAAGAcAcA
|||| | |||||| |||| ||||||| || | |
31 1317 AttAAGT tgtaATgGTAGTG ACGGGA cAcATAGTGAAcGAgAgA
| | |||| || | | | || |
18 1445 A gcagtgtagacggTacaAGTG AC aAtAgcaatAtaGAaAat

con a-caagtagggacagaga-ggt-agga-g--agtgataga-cgg--gaagca---agtgAaaga-a--

6 1391 GgGAGCATGCAGgCACAgCAGGAATAT TgGAATTgtTAAAATGTAAaGATtTAC GggCagCATT
| |||||||||| |||| ||||||||| | ||||| |||||||||| ||| ||| | | ||||
11 1391 GaGAGCATGCAGACACAtCAGGAATAT TAGAATTacTAAAATGTAAgGATATAC GAtCtACATT
|| |||||| || ||| | | | | | |||| | | | |
33 1420 atgttACgttgCAGGAA AT TAGtAATGTTCTAcAtAgTAGTAATACAAAAGCAAatAT
|| || | ||||| ||| | | ||||||| |||| ||| ||
16 1417 cTatAtgcCaAACACcacttacAA ATATTTTaAATGTaCTAAAAACTAGTAATGCAAAgGCAGCaAT
| | | ||||| | |||| || | || |||||||||| |||| || || || ||
31 1361 aTgAAaCtCCAACAC GtA ATATATTgcAaGTGTTAAAAACTAGCAATGgtAAAGCtGCTAT
| || | ||| | | | || | |||||||| | |||| ||| |||||
18 1487 gTaAAtCcaCAAtgtaccataGcAcAatTAaaagActTGTTAAAAgtaAaCAATaaacAAGgaGCTAT

con gtgaa--t-caa-c-ca-caggaAtAtattagaaatgtt-tAaaaa--ag-aaTacaaaagcagc-aT

6 1455 ACtTGGTAAGTTTAAAGAaTGCTTTGGGCTGTCtTTTaTaGATTTAATTAGGCCATTTAAAAGTGATA
|| ||||||||||||||| |||||||||||||| ||| | ||||||||||||||||||||||||||||
11 1455 ACaTGGTAAGTTTAAAGAcTGCTTTGGGCTGTCaTTTgTtGATTTAATTAGGCCATTTAAAAGTGATA
| ||| |||||||| || ||| | ||| || ||| | || |||||||||||||||
33 1478 ATTAtaTAAATTTAAAGAGgcCTATGGaaTaAGTTTTaTgGAATTAGTAAGACCATTTAAAAGTGATA
||| |||||||||||| || || | |||||| ||||||||||||||||||||| |||
16 1484 GTTAGcaAAATTTAAAGAGTTATAcGGgGTgAGTTTTtcaGAATTAGTAAGACCATTTAAAAGTAATA
||||| ||||||||||| ||||| || || |||||| ||| || | | || |||||| |||| ||||
31 1422 GTTAGgtAAATTTAAAGAaTTATATGGtGTAAGTTTTAtgGAAcTAaTTAGgCCATTTcAAAGcAATA
||||| ||||||||| |||||| || |||| || || |||| ||| |||| |||
18 1555 GTTAGcagtATTTAAAGAcacATATGGgcTAtcaTTTAcaGAttTAgTTAGaaatTTTaAAAGtgATA

con -ttaggtaaaTTTAAAGA-t--TatGGgcT---tTTTataGA-tTA-TtAG-ccaTTTaAAAGtgATA
JJ4-ttagttagaccatttaaaagtgata

6 1523 aAACaACATGTtaGATTGGGTGGTaGCAGGgTTTGGTATACATCATAGCATAtCAGAgGCATTTCAA
11 1523 gAACcACATGTgCcGATTGGGTGGTtGCAGGATTTGGTATACATCATAGCATAGCAGAtGCATTTCAA
33 1546 AAACAAgcTGTaC[?]GATTGGTGTATaaCAGGATaTGGAATTAgTCCatcagTAGCAGAaAGTTTAAAA
16 1552 AAtCAACgTGTtgcGATTGGTGTATtGCTGCATTTGGAcTTACACCcAgtaTAGCtGAcAGTaTAAAA
31 1490 AAAgCACaTGTACtGATTGGTGTgTAGCTGCgTTTGGAGTTACAggtACAgTtGCAGAAGGATTTAAA
18 1623 AAAcCACgTGTACaGATTGGgtTacAGCTataTTTGGAGTaAacccaACAaTaGCAGAAGGATTTAAA

con aAac-AcaTGTacaGATTGG--t-tagC-ggaTtTGGaaT-aatccta-aaTagCaGAaggatTtaAA
aaacaacNtgtNcagattgg-JJ4

6 1591 AAaTTAATTGAGCCATTAAGTTTATATGCACATATACAATGGCTaACAAATGCATGGGAATGGTAtT
11 1591 AAgTTAATTGAGCCATTAAGTTTATATGCACATATACAATGGCTtACAAATGCATGGGAATGGTAcT
33 1614 gtATTAATTaAACAgcATAGTTTgTATaCtCATtTACAATGTTTAACtTGcGataGaGGAATaaTAaT
16 1620 ACACTATTaCAACAATATTGTTTaTATTtaCAcaTtCAAAGTTTAGCATGTTCaTGGGAATGGTTgT
31 1558 ACcCTATTgCAACCATATTGTTTgTATTgCCATtTaCAAAGTTTAGCATGTTCcTGGGcATGGTTAT
18 1691 ACaCTAaTaCAgCCATtTataTTaTATgcCCATaTtCAAtGTcTAGacTGTaaaTGGGGagTatTaAT

con aca-TAaTtcA-Ccat-tagtTTaTATgcaCAtaTaCAAtGt-Ta-catgtgcatGgGGaaTggTaaT

6 1659 gTTAGTATTAtTAAGaTTTAAAGTAAATAAaAGtAGAaGTACCGTtGCACGTACAcTtGcaACGcTAT
11 1659 ATTAGTATTAATAAGgTTTAAAGTAAATAAgAGCAGATGTACCGTgGCACGTACAtTaGGTACGTTAT
33 1682 ATTAtTgTTAATtAGATTTAggTGTAGcAAAAACAGgTtAACAGTaGCAAAAcTAaTGaGTAAtTTAT
16 1688 GTTAcTaTTAGTaAGATaTAAATGTGGAAAAAATAGAgaAACAATTGAAAAATTgcTGtcTAAAcTAT
31 1626 GTTAaTgCTtGTGAGATtTAAATGTGcAAAAAATAGAaTAACAATTGAAAAATTaTTAgaaAAATTAT
18 1759 aTTAgccCTgtTGcGtTacAAATGTGgtAAgAgTAGAcTAACAgTTGctAAAggtTTAagtAcgTTgT

con -TTAgtatTa-TaaGaTttAaatgt-gtAAaA-tAGa-taACagTtGcaaaa-tatTaggtA-gtTaT

6 1727 TAAATATACCTGAAAAcCAaATGTTAATaGAGCCaCCaAAAATACAAAGTGGtGTtgcAGCCCTGTAT
11 1727 TAAATATACCTGAAAAtCAcATGTTAATtGAGCCtCCtAAAATACAAAGTGGcGTAcGAGCCCTGTAT
33 1750 TATcaATACCTGAAAcaTGTATGgTtATAGAGCCaCCAAAATTACGgAGccaAaCAtGtGCATTGTAT
16 1756 TATGTgTgTCTcCAAtgTGTATGaTgATAGAGCCtCCAAAATTgCGTAGtACAGCAGCAGCATTATAT
31 1694 TgTGTaTATCTaCAAaTTGTATGTTAATTCAGCCACCcAAATTaCGTAGcACAGCTGCAGCATTATAT
18 1827 TacacgTAcCTgaAAcTTGTATGTTAATTCAaCCACCaAAATTgCGaAGtAgtGtTGCAGCAcTATAT

con TatataTacCTgaAAattgtATGtTaAT-gAgCCaCCaAAAtTaCgaAGtagag--gcaGCa-T-TAT

6 1795 TGGTTTcGtACAGGtATaTCAAATGCcAGTACAGTTATAGGGGAaGCaCCaGAATGGATAACaCGCCA
11 1795 TGGTTTAGgACAGGcATtTCAAATGCaAGTACAGTTATAGGGGAgGCgCCgGAATGGATAACgCGCCA
33 1818 TGGTTTAGAACAGcaATgTCAAAcATTAGTGAtGTacAAGGtacaACaCCtGAATGGATAgAtAGACt
16 1824 TGGTATAaAACAGGtATaTCAAAtATTAGTGAaGTgTATGGaGAcACgCCAGAATGGATAcAAAGACA
31 1762 TGGTAcAGAACAGGAATgTCAAAcATTAGcGAtGTATATGGtGAaACACCAGAATGGATAgAAAGACA
18 1895 TGGTAtAGAACAGGAATaTCAAAtATTAGtGAaGTAatgGGaGAcACACCtGAgTGGATAcAAAGACt

con TGGT-tagaACAGgaATaTCAAAtattAGtgaaGTaa-aGG-gaaaCaCCaGAaTGGATA-aaaGaCa
BE32-AXAXCAAAXAXXAGXGAAGX-BE32 JJ6-tggataNaaagaca

6 1863 aACaGTTATTGAACAcgGgTTGGCaGACAGTCAgTTTAAATTAACaGAAATGGTGCAGTGGGCgTATG
11 1863 gACcGTTATTGAACATAGtTTGGCTGACAGTCAATTTAAATTAACTGAAATGGTGCAGTGGGCATATG
33 1886 AACtGTTTTACAACATAGcTTTAATGATAaTAtATTTGAtTTAAgTGAAATGGTACAGTGGGCATATG
16 1892 AACAGTATTACAACATAGTTTTAATGATtgTACATTTGAaTTATCaCAgATGGTACAATGGGCcTAcG
31 1830 AACAGTATTACAgCATAGTTTTAATGAcAcaACATTTGATTTGTCcCAAATGGTACAATGGGCATATG
18 1963 tACtaTtaTACAaCATgGaaTagATGAtAgcAatTTTGATTTGTCagAAATGGTACAATGGGCATtTG
con aACagTt-TacAaCAtaGttTt-atGA-agtaaaTTTgA-TTa-cagAaATGGTaCA-TGGGCaTatG
aacNgttatacaacatagtttNgatgat-JJ6

6 1931 ATAATGAcATaTGcGAgGAgAGTGAaATtGCATTTGAATATGCACAaaGgGGAGAtTTTGAtTCtAAT
11 1931 ATAATGAtATtTGtGAaGAaAGTGAgATaGCATTTGAATATGCACAgCGTGGAGAcTTTGAcTCcAAT
33 1954 ATAAcGAgtTAacgGACGATAGTGAcATTGCATATtAtTATGCACAACTTGCAGAtTCaAATAGTAAT
16 1960 ATAATGAcaTAgTaGACGATAGTGAAATTGCATATAAATATGCACAATTgGCAGACACTAATAGTAAT
31 1898 AcAATGAtgTtATgGATGATAGTGAAATTGCcTATAAATATGCACAATTAGCtGACAGTgATAGTAAT
18 2031 AtAATGAgcTgAcaGATGAaAGcGAtATgGCaTtTgAATATGCcttATTAGCaGACAGcaAcAGcAAT

con AtAAtGA-aTaa--GA-GAtAGtGAaATtGCaT-TgAaTATGCacaatt-GcaGAc--t-AtagtAAT

6 1999 GCAcGaGCaTTTTAAATAGcAATATGCAGGCaAAATATGTgAAAGATTGTGCAAcTATGTGtAGACA
11 1999 GCAaGgGCcTTTTAAATAGTAATATGCAGGCtAAATATGTAAAAGATTGTGCAATTATGTGcAGACA
33 2022 GCtgcTGCaTTTTAAAAAGTAAcTCACAaGCAAAAATaGTAAAGGAcTGTGgAATAATGTGTAGACA
16 2028 GCAaGTGCcTTTcTAAAAAGTAATTCACAGGCAAAAATtGTAAAGGATTGTGcAACAATGTGTAGACA
31 1966 GCAtGTGCaTTTTAAAAAGTAATTCgCAGGCAAAAATaGTtAAAGATTGTGgAACAATGTGTAGACA
18 2099 GCAgcTGCcTTTTAAAAAGcAATTgcCAaGCtAAAtattTaAAAGATTGTGccACAATGTGcAaACA

con GCa-gtGC-TTTtTAAAaAGtAAttcgCAgGCaAAA--tgTaAAaGAtTGTGcaAcaATGTGtAgACA

6 2067 TTATAAACATGCAGAAATGAggAAGATGTCTATaAAACAATGGATaAAacATAGGGGTtCTAAAaTaG
11 2067 TTATAAACATGCAGAAATGAaaAAGATGTCTATtAAACAATGGATtAAgtATAGGGGTaCTAAAgTtG
33 2090 TTATAAAaAAGCAGAAAAAcgtAAAATGTCaATagGACAATGGATAcAAagTAGATGTGAaAAAacAa
16 2096 TTATAAACGAGCAGAAAAAaaACAAATGagtATGaGtCAATGGATAAAAtaTAGATGTGAtAggGTAg
31 2034 TTATAAACGAGCAGAAAAACGACAAATGtccATGgGACAGTGGATtAAAagTAGATGTGAcAAAGTta
18 2167 TTATAggCGAGCccAAAAACGACAAATGaatATGtcACAGTGGATacgAttTAGATGTtcaAAAaTag

con TTATAaac-aGCagAAAaa-ga-AaATGtctATgagaCAaTGGATaaaataTAGatGTg-tAaa-tag
JJ11-tggataaaatatagatgtNctaaaatag

6 2135 AagGcacAGGaAAtTGGAAaCCAATTGTaCAaTTcCTAcGACATCAAAAtATAGAATTcATTCCtTTT
11 2135 AcaGTGtAGGtAAcTGGAAgCCAATTGTgCAGTTtCTAAGACATCAAAACATAGAATTTATTCCATTT
33 2158 ATGATGGAGGaAATTGGAgaCCAATaGTaCAGTTgTTAAGATATCAAAACATtGAATTTAcagCATTT
16 2164 ATGATGGAGGTGATTGGAaGCAAATtGTtAtGTTTTTAAGgTATCAAggtgTAGAgTTTATGTCATTT
31 2102 gTGAcGaAGGTGAcTGGAGGgAcATAGTaAAGTTTTTAAGATATCAACAAATAGAaTTTgTGTCATTT
18 2235 aTGAaGggGGaGAtTGGAGaccaATAGTgcAaTTccTgcGATAcCAACAAATAGAgTTTaTaaCATTT
con atgatggaGG--AtTGGA--ccaAT-GTacagTTt-TaaGatAtCAAaa-aTaGAaTTtat--CaTTT
atgatggaggaaattgga-JJ11 JJ12-cattt

6 2203 TTAActAAAtTtAAATTATGGCTGCACGGtACGCCaAAAAAAAAcTGcATAGCCATaGTAGGcCCtCC
11 2203 TTAAGcAAAcTaAAATTATGGCTGCACGGaACGCCcAAAAAAAAtTGTATAGCCATTGTAGGgCCACC
33 2226 TTAgGTGCATTtAAAAagTTTTTaaAAGGtATACCaAAAAAAAgcTGTATgcTAATTTgTGGaCCAGC
16 2232 TTAaCTGCATTAAAAAgaTTTTTgcAAGGcATACCtAAAAAAAAtTGcATaTTAcTATATGGTGCAGC
31 2170 TTAtCTGCATTAAAgctgTTTTTAAAAGGAgTgCCaAAgAAAAAcTGTATtTTAaTAcATGGTGCAcC
18 2303 TTAggaGCcTTAAAatcaTTTTTAAAAGGAacccCCcAAaAAAAAtTGTtTagTAtTttgTGGacCAgC

con TTAa-tgcatTaAAattaTtttT--AaGGaa-gCCaAAaAAAAa-TGtaTagtaaT-t-tGG-cCa-C
ttaagtgcattaaaattattttgcaaggNacNccNaaaaaaaa-JJ12

6 2271 aGAtACTGGGAAaTCGTaCTTTTGtATGAG TTTAATaAgcTTTcTaGGaGGtACAGTTATTAGTcAT
11 2271 tGAcACTGGGAAGTCGTgCTTTTGcATGAG TTTAATtAAGTTTTTgGGgGGaACAGTTATTAGTTAT
33 2294 aAAtACAGGaAAGTCATatTTTGGaATGAG TTTAATacAGTTTTTaaAAGGGTgTGTTATATCaTgT
16 2300 TAAcACAGGTAAATCATtaTTTGGtATGAG TTTAATGAAaTTTcTgCAAGGGTcTGTAATATgtTtT
31 2238 TAATACAGGTAAATCATATTTTGGAATGAGccTTATTGAgCTTTtTACAAGGATgTaTAATATCATaT
18 2371 aAATACAGGaAAATCATATTTTGGAATGAGttTTAT acaCTTTaTACAAGGAgcagTAATATCATtT

con -aAtACaGG-AAaTCaTatTTTGgaATGAG-tTTAataaacTTTtTacaaGGatc-gT-ATat--taT

6 2338 GTaAATTCCaGCAGCCATTTtTGGtTgCAaCCgtTAgtaGATGCtAAgGTaGCATTgTTaGATGATGC
|| |||||| ||||||||||| ||| | || || || ||||| || || ||||| || ||||||||
11 2338 GTtAATTCCtGCAGCCATTTcTGGcTaCAGCCAcTAaCgGATGCAAAAGTgGCATTaTTgGATGATGC
|| ||||| || || || ||| | |||||| || | |||||||| | | | | |||||||
33 2361 GTAAATTCTAAAAGtCAcTTTTGGTTgCAGCCATTAtCAGATGCAAAAATAGGAATGaTAGATGATGt
|||||||||||||| || ||||||||| || |||||| |||||||| ||||||||| ||| |||||||||
16 2367 GTAAATTCTAAAAGcCATTTTTGGTTACAACCATTAGCAGATGCcAAAATAGGtATGTTAGATGATGC
| ||||||| ||||| ||||||||||||||||||| | || ||||| |||||||| |||||||||||||
31 2306 GcAAATTCaAAAAGTCATTTTTGGTTACAACCAcTgGCtGATGCTAAAATAGGCATGTTAGATGATGC
| ||||| | |||||||||||||| |||| | | ||| |||| | | |||||||||||||||
18 2438 GtgAATTCcActAGTCATTTTTGGTTggAACCgtTaaCaGATaCTAAggTgGcCATGTTAGATGATGC

con GtaAATTCcaaaAG-CAtTTtTGGtT-cAaCCatTagcaGATgCtAAa-TaG-aaTgtTaGATGATGc

6 2406 aACACAgCCATGTTGGAtATATATGGATACATATATGAGAAAttTgTTAGATGGTAATCCTATGAGtA
||||| |||||||||| |||||||||||||||||||||||| | |||||||||||||||||| |
11 2406 cACACAACCATGTTGGACATATATGGATACATATATGAGAAAccTaTTAGATGGTAATCCTATGAGcA
|| | | | ||||||||||||| ||| || ||||||||| |||||||| ||| || |
33 2429 aACgCcAatAaGTTGGACATATATAGATGAtTAcATGAGAAATGCgTTAGATGGAAATgaAATTTCaA
|| |||||| || |||||||| | | |||||||| || ||||||||| | |||| |
16 2435 TACAgtGCCcTGTTGGAAcTAcATAGATGAcaAttTAAGAAATGCATTgGATGGAAATttAGTTTCTA
|||| ||| |||||| | || ||||| | | || |||||||| | ||||| || || ||||
31 2374 TACAACGCCaTGTTGGcAtTAtATAGAcAAtTAccTAcGAAATGCAcTAGATGGCAAcCCtGTATCTA
|| || | |||||| || | || | || | ||||||| |||||||||| || || ||
18 2506 aACgACcaCgTGTTGGacaTActTtGAtAccTAtaTgaGAAATGCgtTAGATGGCAAtCCaaTAagTA

con aACaccgccatGTTGGacaTAtaTaGAta--tAtaTgaGAAAtgc-tTaGATGG-AAtcc-aT---tA
JJ15-gttggacatatatNgatacNtatatgagaaatgcgttagatgg-JJ15

6 2474 TtGAcAGAAAgCATAaAGCATTgACATTAATTAAaTGTCCACCtCTgCTaGTaACgTCcAAcATAGAt
| || ||||| |||| |||||| |||||||||| |||||||| || || || || || || |||||
11 2474 TAGATAGAAAACATAGAGCATTAACATTAATTAAgTGTCCACCgCTaCTgGTTACaTCAAATATAGAc
||||| |||||||| |||||| | | || ||||||||| || || |||| ||||||| | |
33 2497 TAGATGTgAAACATAGgGCATTAGTgCAAtTAAAATGTCCACCACTgCTtcTTACcTCAAATAcAAAT
| ||||| || ||||| |||| || ||| |||||||| || | | |||| || || | |||
16 2503 TgGATGTAAAGCATAGAcCATTgGTaCAAcTAAAATGcCCTCCATTATTaATTACATCTAACATtAAT
| ||||||||||||| | | || | || ||||||| ||||| ||||| ||||||||||| || |||
31 2442 TaGATGTAAAGCATAAAgCtTTAATgCAgtTAAAATGTCCTCCtTTATTgATTACATCTAATATAAAT
| ||| |||||| ||| | ||||| || ||||||||||||| || | | || | |||||| ||
18 2574 TtGATagAAAGCAcAAAcCaTTAATaCAacTAAAATGTCCTCCaaTAcTacTaACcaCaAATATAcAT

con TaGAt--aAAgCAtA-agCaTTaatacaa-TaAAaTGtCC-CCacTacTa-TtACatCaAAtAtaaAt
6 2542 ATTActAAAGAaGAtAAATAtAAgTATTTACATAcTAGAGTaACaACATTTACATTTCCAAATCCATT
|||| ||||| || ||||| || |||||||||| |||||| || ||||||||||||||||||||||
11 2542 ATTAGCAAAGAgGAgAAATAcAAATATTTACATAGTAGAGTtACcACATTTACATTTCCAAATCCATT
||| ||| | || |||||||||||||||| | || |||| |||| |||||||||
33 2565 GCaGGCACAGAcTCTAGATGGCCATATTTACATAGTAGATTaACaGTATTTgaATTTaaAAATCCATT
|| || ||||| ||||| ||||| |||||||||| |||||| || ||| |||| ||| ||
16 2571 GCtGGTACAGATTCTAGgTGGCCtTATTTACATAaTAGATTGGTGGTgTTTACATTTCCtAATgagTT
|| |||| ||| || ||||| || |||||| ||| ||||||| ||||||||||| ||| ||
31 2510 GCAGGTAAGGATgAcAGATGGCCATAccTACATAGcAGAcTGGTGGTtTTTACATTTCCAAATcCATT
||| |||||| | ||||||||||| || | || ||| | || ||| |||||||||| ||||
18 2642 cCAGcaAAGGATaAtAGATGGCCATAttTAgAaAGtAGAaTaacaGTaTTTgaATTTCCAAATgCATT

con gc-ggtAaaGAtgatAgaTggccaTAttTAcAtAgtAGA-TaacagtaTTTacATTTccaAATccaTT

6 2610 CCCtTTTGACAGAAATGGGAATGCAGTgTATGAACTgTCAaATaCAAACTGGAAATGTTTtTTTGAAA

11 2610 CCCcTTTGACAGAAATGGGAATGCAGTaTATGAACTATCAGATGCAAACTGGAAATGTTTcTTTGAAA

33 2633 CCCATTTGAtGAAAATGGtAAcCCAGTGTATGcAaTAAATGATGAAAAtTGGAAATCCTTTTTCTCAA

16 2639 TCCATTTGACGAAAACGGAAATCCAGTGTATGAgcTtAATGATAAgAACTGGAAATCCTTTTTCTCAA

31 2578 TCCATTTGACAAAAACGGAAATCCAGTATATGAAtTAAgTGATAAAAACTGGAAATCCTTTTTCTCAA

18 2710 TCCATTTGAtAAAAAtGGcAATCCAGTATATGAAaTAAaTGAcAAAAAtTGGAAATgtTTTTTtgaAA

con -CCaTTTGAcaaAAAtGG-AAtcCAGT-TATGaacTaaatgAtaaaAAcTGGAAAT--TTtTT---AA

6 2678 GACTGTCGTCaAGCCTAGACATTcAGGATTCtGAGGA CGAGGAA GATGGAAGCAATAGCCAA

11 2678 GACTGTCGTCCAGCCTAGACATTgAGGATTCAGAGGA CGAGGAA GATGGAAGCAATAGCCAA

33 2701 GGACGTGGTgCAaATTAGATTTaataGAGGAAGAGGA CAAGGAAAACcATGGAGGaAATATCagc

16 2707 GGACGTGGTcCAGATTAAgTTTGCACGAGGAcGAGGA CAAGGAAAACGATGGAGACTCTTTgcCA

31 2646 GGACGTGGTgCAGATTAAATTTGCACGAGGAAGAGGA CAAAGAAAACGATGGAGACTCTTTCtCA

18 2778 GGACaTGGTcCAGATTAgATTTGCACGAGGAAGAGGAagatgcAGAcAcCGAaGGAaACcCTTTCggA

con GgacgTgGTccAgatTAgattTgcacGAggaaGAGGA---c-agGAaaacgAtGGA--ca-T-tcc-a

6 2740 GCGTTTAGATGCGTGCCAGGAaCAGTTGTTAGAACTTTATGAAGAAAACAGTAcTGAccTACACAAAC

11 2740 GCGTTTAGATGCGTGCCAGGAtCAGTTGTTAGAACTTTATGAAGAAAACAGTAtTGATaTACACAAAC

33 2766 ACGTTTAAATGCagtgCAGGAgAAAATACTAGAtCTTTAcGAAGcTGATAaaACTGATtTACcatcAC

16 2772 ACGTTTAAATGTGTGTCAGGACAAAATACTAacACATTATGAAAATGATAGTACAGAcCTACGTGAcC

31 2711 ACGTTTAAATGTGTGTCAGGACAAAATAtTAGAACATTATGAAAATGATAGTAAAcgaCTttGTGAtC

18 2846 ACGTTTAAgTtgcgtgCAGGACAAAATcaTAGAcCAcTATGAAAATGAcAGTAAAgacaTagacagcC

con aCGTTTAaaTgcgtg-CAGGAcaAaaTatTAgaaC-tTAtGAA-atgA-AgtAc-gaccTacacaaaC

6 2808 AtgTatTGCATTGGAAATGCATgaGAcatGAAAGTGTATTAtTAtAtAAAGCAAAACAAATGGGCCTa

11 2808 AcATTaTGCATTGGAAATGCATACGAtTGGAAAGTGTATTAcTAcAcAAAGCAAAACAAATGGGCCTg

33 2834 AaATTGAaCATTGGAAACtgATACGCaTGGAgTGTGCTTTATTgTAtAcAGCCAAACAAATGGGATTT

16 2840 ATATAGACTATTGGAAACAcATgCGCCTaGAATGTGCTaTtTatTAcAAgGCCAGAGAAATGGGATTT

31 2779 ATATAGACTATTGGAAACAtATtCGaCTtGAATGTGtAtTAaTgTATAAAGCAAGAGAAATGGGAATA

18 2914 AaATAcAgTATTGGcAACtaATaCGttggGAAaaTGcAaTAtTcTtTgcAGCAAGgGAAcatGGcATA

con AtaTagag-ATTGGaAAc-cATacGactgGAa-gTG-atTatt-tataaaGCaA-a-AAatgGG--Ta

6 2876 AGCCACATaGGaaTgCAAGTAGTgCCACCATTAAagGTGTCcGAagCaAAAGGACATAATGCcATTGA
11 2876 AGCCACATcGGgtTaCAAGTAGTaCCACCATTAActGTGTCAGAGACtAAAGGACATAATGCtATTGA
33 2902 tcACATtTatGCCACCAgGTGGTGCCttCtTTGttaGcATCAAAGACcAAAGCATTTCAAGtAATTGA
16 2908 aAACATATTAACCACCAaGTGGTGCCAaCacTGgCtGTATCAAAGAatAAAGCATTACAAGCAATTGA
31 2847 CAcAgTATTAACCACCAGGTGGTGCCAGCgTTGtCaGTATCAAAGgccAAAGCcTTACAAGCTATTGA
18 2982 CAgAcatTaAACCACCAGGTGGTGCCAGCcTataacaTtTCAAAaagtAAAGCacataAAGCTATTGA

con aaccatataa-ccacCA-GTgGTgCCa-Cattgac-gtaTCaaAgactAAAGca--t-AaGctATTGA
JJ18-tcaaagactaaagcacataaagcNattga

6 2944 AATGCAAATGCATTTAGAATCaTTAttAAggACTgAGTATaGTaTGGAACCgTGGACATTACAaGAaA
11 2944 AATGCAAATGCATTTAGAATCcTTAgcAAAAACTCAGTATgGTgTGGAACCtTGGACATTACAgGAcA
33 2970 ACTaCAAATGgCaTTAGAgACATTAagTAAATCACAGTATAGTAcaagcCAaTGGACATTgCAAcAaA
16 2976 ACTgCAAcTaACGTTAGAAACAaTAtATAACTCACAATATAGTAATGAaaAgTGGACATTaCAAgAcg
31 2915 ACTaCAAATGAtGTTgGAAACAtTAaATAACACTgAATACAAAAATGAGGAcTGGACAaTGCAgcAaA
18 3050 ACTgCAAATGgcccTacAAggccTtgcacAaAgTcgATACAAAAccGAGGAtTGGACAcTGCAagAcA

con AcTgCAAaTg-c-tTagAaacatTa---aaaactca-TAtagta--gaaca-TGGACAtT-CAagA-a
actgcaaatgg-JJ18

6 3012 CaAGTTATGAAATGTGGCaAACACCACC tAAACGcTGtTTTAAAAAACgGGGcAAaACTGTaGAaGT
11 3012 CcAGTTATGAAATGTGGCTAACACCACC CAAACGgTGcTTTAAAAAACAGGGAAAtACTGTgGAgGT
33 3038 CaAGCTTaGAgGTGTGGCTttgTGaACCACC AaaATGTTTAAAAAACAaGGAgAaACAGTaactGT
16 3044 ttAGCCTTGAAGTGTATTTAACTGCACCAAC AGGATGTaTAAAAAAACATGGATATACAGTgGAaGT
31 2983 CAAGtCTTGAACTGTATTTAACTGCACCTAC AGGgTGTTTAAAAAAACATGGATATACtGTAGAgGT
18 3118 CAtGcgagGAACTaTggaatACaGaACCTACtcactgcTTTAAAAAA ggTGGccAaACaGTAcAaGT

con caaG-t-tGAa-TgTggctaac-gcACCaacaa-g-tgttT-AAAAAacatGGa-A-AC-GTagaaGT

6 3079 tAAATTTGA TGGCTGTGcAaACAATacAATGGAtTATGTGGTATGGACAgATgTgTAtgTGCAGG
11 3079 aAAATTTGA TGGCTGTGAAgACAATgtAATGGAgTATGTGGTATGGACAcATATATAccTGCAGG
33 3105 GCAATaTGA caatGACAAAaAaAATACAATGGATTATACAAACTGGggtgAaATATATATTataG
16 3111 GCAgTTTGATGG aGACATATgCAATACAATGCATTATACAAACTGGAcAcATATATATATTTGTG
31 3050 GCAaTTTGATGG tGAtGTAcACAAcACcATGCATTATACtAACTGGAaAtTTATATAccTaTGTa
18 3185 atAtTTTGATGGcaacaAaGacaAttgtAtgAcctATgtagCatgggacAgtgTgtatTAtaTgacTg

con gcAaTtTGAtggcaacgatgaaaacaatacaAtggAttat-caaactggacagatataTAtaTg--tg

6 3144 ACAAtGACaCcTGGGTAAAgGTgcaTAGTatgGTAGATGCtAAGGGtATATATTAcACATGTGGACAA
11 3144 ACAAcGACtCATGGGTAAAaGTaACTAGTtccGTAGATGCcAAGGGcATATATTATACATGTGGACAA
33 3170 AgGAAGAtaCATGtACTATGGTtACaGGgaAAGTAGATTATAtaGGTATgTATTATATACATaActGt
16 3176 AAGAAGcatCAgtaACTGTGGTaGAgGGtCAAGTTGAcTATtAtGGTtTaTATTATGTtCATGAAGGA
31 3115 tAGAtGGccaATGtACTGTtGTgGAaGGgCAAGTTAATTgTAAGGGcaTtTATTATGTACATGAAGGA
18 3253 atGcaGGaacATGggacaaaaccGctacctgtGTaAgTcacAgGGGatTgTATTATGTAaAgGAAGGg
con a-gaaGacacatgg-cta-ggt-g-t-gt-aaGTagattataagGGtaTaTATTAt-tacatgaagga

6 3212 TTTAAAACATATTATGTAAAcTTTgtaAAAGAGGCAgAAAAGTATGGgAGcACCAAaCATTGGGAAGT
11 3212 TTTAAAACATATTATGTAAATTTTAAtAAAGAGGCAcAAAAGTATGGTAGtACCAAtCATTGGGAAGT
33 3238 gaAAAggtATATTTTaaAtATTTTAAAGAgGATGCtGcAAAGTATtcTAAAACacAAaTgTGGGAAGT
16 3244 atAcgAACATATTTTGTgcAgTTTAAAGAtGATGCAGAAAAATATaGTAAAAaTAAAgTATGGAAGT
31 3183 cAtAtAACATATTTTGTAaAtTTTAcAGAaGAgGCAaAAAAATATGGGActggTAAAAaATGGGAAGT
18 3321 tAcAacACgTtTTaTaTAgAaTTTAaAagtGAatgtgAAAAATATGGGAacacaggtAcgTGGGAAGT
con t-taaaacaTaTT-TgtaaAtTTTaaa-aaGAggcagaAAA-TATgg-Aa-ac-aaaaa-TGGGAAGT

6 3280 ATGTTATGGCAGCACAGTTAT ATGTTCTCCTGC ATCTGTATCTAGCACTacACaAGAAGTAT
11 3280 ATGTTATGGCAGCACAGTTAT ATGTTCTCCTGC ATCTGTATCTAGCACTgtACgAGAAGTAT
33 3306 ACATGtGGGTGGTCAGGTAAT tgTTTGTCCTAC gTCTaTATCTAGCA ACCA AaTAT
16 3312 tCATGCGGGTGGTCAGGTAAT atTaTGTCCTACATCTGTgTTTAGCAGCA ACGA AgTAT
31 3251 gCATGCGGGTGGTCAGGTAATTG TTTtTCCTgaATCTGTaTTTAGCAG TGACGA AaTAT
18 3389 aCATtttGGgaaTaAtGTAATTGattgTaaTgactctatgtgcagTAcCAG TGACGAcacggTAT
con acaT---GGt-gt-agGTaATtg-at-tt-Tcctgcatc-tct-t--c-AGcactgac-aagaagTAT
BE21-CGGTAT

6 3342 CCATTcCTGAA tCTACTACATACACCCCCGCACAGACC tCcaCCCT tGTGTCCtCaaGC AC
11 3342 CCATTgCTGAA CCTACTACATACACCCCCGCACAGACCacCgcCCCTacaGTGTCCgCctGC AC
33 3362 CCACTaCTGAAACTgcTgACATACA gACAGAC AAC gaTaacCGACC AC
16 3371 CCTCTcCTGAAAtTATTaggcagCA cttgGCC AACcACcCCgCCgCGACCcATAC
31 3310 CCT tTGCTggGATTGTTAcAaAGCTACcaacaGCC AACaACACCaCCaCAtCGaATTC
18 3454 CC gcTaCTcaGcTTGTTAaAcAGCTAC AgcACACCcCCtCAcCGtATTC
con CCact-cTgaaa---ttgacatacAcccacgcacagacc--c--caacaac-cctcc-Caacc-ataC
CC---GCXACXCAGCXXG-BE21

6 3403 CA aGGAAGACGcaGTGcaaaCGCCGCCTAGGAAaCG
|| |||||||| ||| ||||||||||||| ||
11 3406 CAC GGAAGACGGcGTGtcggCGCCGCCTAGGAAgCG
||| || | |
33 3410 CACAAGC agcggccAAACgacGACGAC cTgCAGacACCA
|| |||| |||| |||||| | ||| ||||
16 3426 CA AAGCCgtCGCCTTGGGCACC GAAG AAACacaGACGAC TATcCAGCGACCAAGA
|| || || ||||||||||||| |||| |||||| || |||||||||||
31 3368 CA AAACCtgCGCCTTGGGCACCaGtGAAGgtgTgCGGCgggcGACGACGTCTActaAGCGACCAAGA
|| ||| || |||||||| | ||| | |||| ||||||
18 3503 CA gcACCgtgtCCgTGGGCACC GcaAAGaccTaCGGC caGACGTC
BE10-GGCGXGXCGGCGCCGCCXAG-BE10

con CAcaaaccgtcgccttgggcacc-g-gaaggcgtacgaagac-gacgacgtcc-cc-ag--accaaca

6 3439 AGCACGaggagtccaACaGTCCcCTtgCAACgCCtTGTGTGTGGCCcACATtgGAcCCGTGGACAGTg
|||||| || |||| || |||| || ||||||||||| |||| || ||||||||||
11 3442 AGCACG tggACCGTCCaCTaaCAACaCCcTGTGTGTGGCCaACATCaGAtCCGTGGACAGTA
|| | | ||| | | | || |||||| ||
33 3449 CAGACACCGCCcAgCCCCT tacaaAgcTGTTctGTGCA gaCccCgCCtTGGACAaTA
||||||||| | ||||| | | |||| | | |||||| |
16 3481 tCAGAGCCAGACACCG GAAACCCCTgCCACaCCActAAGTTGTTGCAcaGaGACTCaGTGGACAGTG
|||||||||| | ||||| || |||| ||| |||||||||| | ||||| ||||||||||
31 3435 aCAGAGCCAGAGcaC aGAAACaCCCaCCACcCCAacAAGTTGTTGCGAgGCGACTCcGTGGACAGTG
| | || || || | | | | | || || | |||||| ||
18 3548 GgctgctAcACgaCCtggACaCtgtggAcTcGcgGaGAaGCagCattGTGGAC cTG

con acaaagccaga--ccgc-aaaCccct-c-acaccatgt-tttggtgcacagcggctccgTGGACagTg

6 3507 gAAaCcACAACcTCaTCACTaAC AATcACgACcAGCACCAAA GAcGG AACAACaG
|| | ||||| || ||||| || ||| || || ||||||||| || || |||||| |
11 3504 cAAtCaACAACATCGTCACTgAC AATtACAACAAGCACCAAA GAaGG AACAACTG
|| | | ||| || |||||||| | | | | |
33 3506 gAACAgcaCgtACTGCAACTAACtGC aCAAACAAGCAgCGGA cTgtGTGT AgTTc
|| | |||||| |||| || || |||| |||| | ||| | |
16 3548 cTCCAaTcCtcACTGCAttTAACAGCT CACACAAAggACGGA tTaaCTGT AaTag
|| | | | ||||| |||||| | | |||| |
31 3502 TCAACTgtggggTTaTCaGTGCAGCT gcatgCACAAAccAAACAA GGgCTGTCaGTtGTcc
||||| || || |||||||| ||||| || || || || ||
18 3604 TCAAC ccacTTcTCgGTGCAGCTacacctacaggcaacAACAAagacGGaaacTCtGTaGTgg

con c--aac-c--cactgc-actaaCagctaat-c-aacaagcacca-Aa------gggtgtcaaca-t-g

6 3562 TaACAGTtCAGCTACGCCTATAGTGCAAtTtCAAGGTGAaTCCAATTGTTTAAAgTGTTTAGATATA
| ||||| |||||||||||||||||||| | |||||||| ||||||||||||||| |||||||||||||
11 3559 TcACAGTGCAGCTACGCCTATAGTGCAAcTgCAAGGTGAtTCCAATTGTTTAAAATGTTTAGATATA
| || |||| ||||||||||| | ||||||| || ||| |||||||||||||| |||||
33 3561 TAACgtTGCA CCTATAGTGCATTTAAAAGGTGAaTCaAATAGTTTAAAATGTTTAAGATA
|||| | || || ||||| |||||||||||||||| | |||| ||||||||||||||||||
16 3603 TAACACTACA CCcATAGTACATTTAAAAGGTGATGCtAATAcTTTAAAATGTTTAAGATA
| |||||| || ||| |||| |||||||||||||||||| |||| ||||||||||||||||||
31 3563 TgcaACTACA CCTATAATACAcTTAAAAGGTGATGCAAATAtaTTAAAATGTTTAAGATA
| ||||| ||||||||||| |||||||||| ||| | ||||||||||||| | ||
18 3668 TaacACTAC gCCTATAATACAtTTAAAAGGTGAcagAAAcAgtTTAAAATGTTTAcGgTA

con TaacacTaCagctacgCCtATAgT-CAttTaaAAGGTGAttcaAAtagtTTAAAaTGTTTaaGaTAta
JJ20-catttaaaaggtgaNtcNaatagtttaaaatgtttaagatata

6 3630 GgCTaAATGACAgAcAcAgACATTTaTTTGAtTTAatATCaTCAACGTGGCAcTGGGCCTCctCaaAG
| || ||||||| | | | |||||| ||||| |||   ||| ||||||||||| ||||||||   |  ||
11 3627 GaCTgAATGACAaATATAAACATTTGTTTGAaTTAgcATCtTCAACGTGGCATTGGGCCTCacCtgAG
| [?] |||||| | |||| |   |   || || || ||||||||| ||   |
33 3621 cAGATTaAAAcctTATAAAgAgTTGTATAgTTCtaTGTCaTCcACcTGGCATTGGACCaGtgAcAAc
||||| |||   ||   ||||||| | |  |||| || || |||||||||||   |  | ||
16 3663 TAGATTtAAAAAgcATtgtacATTGTATAcTgCAGTGTCgTCTACATGGCATTGGACAgGacAtAAT
|||  |   |||  ||   |||||||   |||||| |||||||||||||||||| |   ||
31 3623 TAGgcTGtcAAAAtATAaacAATTGTATgaAcAAGTGTCATCTACATGGCATTGGACAtGtacagAT
|| ||  |||| |||   |   |||  | |  | ||||| || ||||||||||||
18 3728 cAGatTGcgAAAAcATAgcgAccacTATagAgAtaTaTCATCcACcTGGCATTGGACA     ggtgc

con g-agattt-aaaaa-Ata-aca-ttgTaT-a-t-a-t-TCaTC-AC-TGGCAtTGGaC-tg-cc-aa-
gcagatt-JJ20

6 3698 GCACCACATAAA cATGCcATTGTAACtgTAACAT   ATgatAGTGAGGAACAAaGgCAaCAgTTTT
||||||||||||  |||| ||||||||  ||||||   ||   |||||||||||| | || || ||||
11 3695 GCACCACATAAAA ATGCAATTGTAACatTAACAT   ATAgcAGTGAGGAACAACGtCAgCAaTTTT
|   ||||| ||| |||||||||  ||||||   |   || ||||||   |   ||||
33 3688 aaAAAtagTAAAA ATGgAATTGTAACtgTAACATtTGtaAcTGAAcAGCAACAAC   AaatgTTTT
|||   ||||| || |||||| ||  | |||| ||  | ||||  |||||   |   ||||
16 3730 GtAAAACATAAAA gTGCAATTGTtACacTtACATATGatAGTGAATgGCAAC   GtGACcAaTTTT
| |||||||||||  ||| ||||| ||  | ||||||   |||  ||  |||   | ||| | ||||
31 3690 GGAAAACATAAAAA TGCtATTGTaACctTAACATATataAGTacATCACAA   AGAGACgAtTTTT
|  ||   |||||  |  ||  | ||  |||||||   |||  | |||||   |||   | ||||
18 3791 aGgcAAtgaAAAAAcaGgaATacTgACtgTAACATAccatAGTgaAaCACAA   AGAacaaAaTTTT

con g-a-aacatAAAaaatGcaATtgTaACtgTaACATatgatagt-aa-agcAAcaaag--aacaaTTTT

6 3762 TAgAtgtTGTAAAAATACCcCCtACCATTAGcCA cAAacTGGGaTTTATGTCAcTgCAccTATTGTA
|| |   ||||||||||| || |||||||| ||  ||  |||| ||||||||| | ||  |||||||
11 3759 TAaAcAgTGTAAAAATACCACCcACCATTAGgCAT AAGgTGGGgTTTATGTCAtTaCATTTATTGTA
||   |  ||||||||||||||   |   |   || |||   | |  ||||||
33 3752 TAggTAccGTAAAAATACCACC tACTgTgcaAAT AAG   TACTGGATTTAT
|   |   || |||||||||   ||| |   | |   |||||||||||
16 3794 TgtcTcaaGTtAAAATACCA AAaACTaTtaCAGT   GTC   TACTGGATTTAT
|   |   || ||||||||  || ||  |  ||||   |||   |||||
31 3754 TAAATACTGTaAAAATACC tAAcACagTatCAGT   GTCaacaggatatatgactATTTA
||||||||||
18 3856 TAAATACTGT

con TaaatactGTaaaaataccaccaaaca-tagcaat-aaggtcgg-tttatgt-actg-atttattgta

6 3829 AtttgtatatatgtaaAtgtgTaaATATATGgTATtgGTGTAatacaActgTACaTGTATGGAaGTgG
|   |   |   ||||||| |||  |||||   |   ||| |||||||| || |
11 3826 A   cCATTAcaccTgtATATATG TATAtGTGTA CATAACATACgTGTATGGAgGTaG
|||||   || ||||| ||| |||  |  ||||
33 3801   G aCATTA   TAaGTGTA CATcACAagCcaaTATG
|
16 3843   G   tCt
|
31 3812   gCc
|
18 3866   tgcaattccagatagtgtacaaatattggtgggataCa

con a--------g--catta----t--atatatggtatatgtgta--cataacaaacatgtatggaagtcg

6 3897 TGCCTGTACAAATaGCTGCAGgAACAACcAgcACATTcATAcT        GCCTGTTaTaATTGCAT
|||||||||||||| ||||||| |||||| |  ||||| ||| |        ||||||| | |||||||
11 3881 TGCCTGTACAAATTGCTGCAGcAACAACTAcAACATTGATATT        GCCTGTTGTTATTGCAT
|||||| |  || ||| |||||||        | |||| |  |
33 3833 TGCTGC TaAtTgtATAtAACcATGATATTtgTTTTTG taTTaTGTTTTaTaTT
| | | | | | | | |||||| || ||| | |
16 3847 atATGA caAAtcTtgatacTgcATccAcAaCATTAcTGgcgTGCTTTTTG CTTTGCTTT GTGTG
|||| || | | | |||| || ||||||||| ||||||||| |||||
31 3815 TaATGATtgAActaAatattTcTAcagtaAgCATT gTGCtaTGCTTTTTG CTTTGCTTTTGTGTG
| | || || | | || | || | | ||||| ||||||||||||
18 3904 TgAcaATgtAAtacAtatgcTgTAgtaccAatATgttatCacTtaTTTTTtatTTTGCTTTTGTGT

con tgac-atacaa-ttgctgc-tgaacaaccA-cA--tt-ata-TgctttttggccTtt-cTtttgtgtt
O21-CTGCAGGAACAACCAGCACATTCATACT        GCCTGTTATAATTGCAT

6 3957 TTGttGTATGTtTTgTTAGcATcaTACTTATtgTATggATATCTGAgTTTaTtGTgTAcACATCTGTG
||| |||||| || |||| || ||||||| ||| |||||||| ||| | || || ||||||||
11 3941 TTGcaGTATGTaTTcTTAGtATtgTACTTATaaTATTAATATCTGAtTTTgTaGTaTATACATCTGTG
| | | | | || | |||||| | || ||| | | ||
33 3886 gT     TTTtaTGctTaTcCtTATTAtTACGTCCtTTAATAcTTTCcaTTTCTACcTATgCtTggTTG
| ||| || | | | ||||| ||||||| | | | || | ||||| || | | ||
16 3911 CT     TTT GTGTGTCTgCcTATTAATACGTCCgCTgcTttTGTCTGTGTCTACATAcaCATCATTA
|| ||| |||||||| | |||||||| || | |||||||||| ||| || || ||
31 3880 CTactaTTT GTGTGTCT     tgTcATACGTCCaCTtgTgcTGTCTGTGTCggtATAtgCAaCAcTA

18 3971

con -tgctgtttg-tgtgt-tgcat--ta-tacgtccatt-atatttct-tttctgtatatacatc--tg
O21-TTGTTGTATGTTTTGTTAGCATCATACTTATTGTATGGATATCTGAGTTTATTGTGTACACATCTGTG-O21

6 4025 CTaGTACTAACACTgCTTTTATATTTaCTaTTGTGGCTgcTATTAACAACCCCcTT GCAATTtTTcc
|| ||||||||||| ||||||||||| || |||||||| ||||||||||||| || |||||| ||
11 4009 CTGGTACTAACACTTCTTTTATATTTGCTTTTGTGGCTttTATTAACAACCCCTTT GCAATTcTTTT
||||| | | | | | ||||||| | | ||||| |||| ||||
33 3950 CTGGT  gTTGGTATTgcTgcTtTggGtgTTTGTGG        gAtCtCCTTTaaaAATT TTTT
| | |||||||| | | | ||
16 3974 aTAaT  ATTGGTATT        acTaTTgTGGaTAACaGCAgCCTCTgCgTTTaG
|| | ||| | | | || ||| | | ||| ||||| | || |
31 3943 cTAtT  ATTaaTtgT        gaTtTTaTGGgTtAttGCAaCCTCTcCaTTacG

18 3971

con ctagtac-tt-atttttttatatttgcttttgtggcttttatgaa-aac-cc-ttc-caatttt--
O21-CTAGTACTAACACTGCTTTTATATTTACTATTGTGGCTGCTATTAACAACCCCCTT GCAATTTTTCC-O21

6 4092 TACTAACtCTactTGTGTGtTAcTgTCCcGCaTTgTATATACACtacTAtATTGT        taccacA
11 4076 TACTAACaCTgtgTGTGTGCTATTTCCTGCcTTTATATACACataTACATTGTgcaaacgcAAcaA
33 4005 T              tTGCTATTT  GTTgtTTTTATAT tTA    CCAaTGaTgTgtATtAATtt
16 4021 gTGTTTtaTTGTATATATtaTATTT  GTT      TATA   TA    CCATTATTTTAATaCATAC
31 3990 tTGTTT   TTGTATATAT  gtTgT  GTT      TATA   TAtattCCATTATTTgTAATtCATAC
18 3971
con     tactaacactgtatat-tgctattttcgttc-ttttatatactata-tccattgtttttaat-cata-
   O21-TACTAACTCTACTTGTGTGTTACTGTCCCGCATTGTATATACACTACTATATTGT        TACCACA-O21

6 4154 cAgcaaTGATGcTAACaTGTCAaTTtAATGATGGaGAT  ACcTGGcTGggTtTGTGGTTGTTatgTG
11 4144 TAATGgTGATGtTAACcTGTCACTTaAATGATGGtGAT  ACATGGtTGTtTcTGTGGTTGTTtacTG
33 4051 TCATGCACAgcaTAtgacacaACaagAgTAATGTATAT  ACATGtaTaTATTGTTtGTATATAtgTG
16 4075 ACATGCAC      gcTTTTTAAttacaTAATGTATATGTACATAaTgtaATTGTT ACATATA
31 4044 ACATGCA      tctTTTTTAA              GTCAAcAgTaaCTTTTTT AC
18 3971                    AtgcatgtatgtgtgctGcCAtgtcccgCTTTTgccAtctgtctgta

con     -catgcacatg-taac-t-t-Aattaaataatggagatgtacatggttg-tTtt-tg-t-t-tatgtg
   O21-CAGCAATGATGCTAACATGTCAATTTAATGATGGAGAT  ACCTGGCTGGGTTTGTGGTTGTTATGTG-O21

6 4220 CcTTTaTTGTAGggaTgtTgGGgTTaTTaTT      gaTgCAcTAtAGaGCTGTACAaGGggaTaAAc
11 4210 CATTTgTTGTAGcTGTacTtGGATTGTTGTT      acTaCATTAcAGgGCTGTACATGGTacTgAAA
33 4117 CA   caTGGTGgTGTtTTAacATTGTTGTT      gTTATTTT      AgtTTTTTTTTTTTGTA
16 4132   atTgTTGTATACcaTaActtactaTtTTttctTtTTTATTTTcaTatAtaaTTTTTTTTTTGT
31 4081     TtGTGTATAC                    TgTTgtTTgTatTgGTattggTaTTggTaTTgg
18 4018 tgtgtgcGTaTgcAtgggtattggtatttgtgtatatTgtggTaataacGTcccctgccacagcaTtc

con     cattt-tt-gtg-a-t-ttag--tt-tt-tt-t-tt-ttt-ttt--a--g-t-t-tttttt-ttt-t-
   O21-CCTTTATTGTAGGGATGTTGGGGTTATTATT      GATGCACTATAGAGCTGTACAAGGGGATAAAC-O21

6 4283 AcACcAAATGTaagAAGTGTAA  CAAAC aCAActgTAaTGatGATTATGTaacTATGcattATacT
11 4273 AaACTAAATGTgctAAGTGTAAATCAAACcgCAAtacTAcTGTgGATTATGTGTATATGtcacATggT
33 4171 ttACTAA          TAAAT        AccTTTATaTttagcaGTGTAT
16 4196                    TTgTTTgtTTgTTTTTTA
31 4124 tattggTATtggTaTaaTaaacTTTTTaCTTtTTTTTTA
18 4086 acagtaTATgtaTtTtgTttttTaTTgccCaTgTTacTattgcatatacatgctatattgtctttaca
con     a-actaaatgtattaagtgtaatt-t--cc-t--tttT-atgttgattaagtgtatatg---tatact
   O21-ACACCAAATGTAAGAAGTGTAA  CAAAC ACAACTGTAATGATGATTATGTAACTATGCATTATACT-O21

6 4348 acTgATGGtGATTAT   aTaTAtATGAAtTAGAGTAAACCgTTTTTATAtttgtaacaGTGTAtGc
          | |||| ||||||   | || ||||| ||||||||||| |||||||||         ||||| |
11 4341 gaTaATGGaGATTATG   TgTAcATGAAcTAGAGTAAACC TTTTTATAcagtgtgtgGTGTAcGt
                  ||||||
33 4206         tATTATG

16 4214                                                          ataaactgTTATTA
                                                                         ||||||
31 4164                                                                  TTATTA
                                                                          |  |
18 4154 gtaattgtataggttgttttatacagtgtattgtacattgtatattttgttttataccttttaTgcTt

con     g-taatggagattatg---tatacatgaa-tagagtaaacc-tttttatatt-ttaat-gt-tatt-
    O21-ACTGATGGTGATTAT   ATATATATGAATTAGAGTAAACCGTTTTTATATTTGTAACAGTGTATGC-O21

6 4413 TttgTATAccATggcacAtagTAGGGCcCGacGACGcAAgCGTGCGTCAGCtACACAgCTATATCAAA
       |   ||||  ||    |   |||||| ||  |||| || ||||||||||| ||||| ||||||||||
11 4405 TagtTATA tATAatgAAACcTAGGGCACGcaGACGtAAaCGTGCGTCAGCcACACAACTATATCAAA
                 | |  ||||      || |  |     | ||||| || || ||||||||||| ||||
33 4213          AGACACAAACGaTCTaCAAGgCGCA  AgCGTGCATCtGCaACACAACTATAcCAAA
                  |||||||||| ||| |||  ||||  | |||||||| || || ||||| ||  |||
16 4228 CttaacaATGCGACACAAACGtTCTgCAAAACGCACaAAACGTGCATCgGCTACcCAACTtTATaAAA
        |    |||||  |||||| ||| |||||||||| |||||||| || ||||| ||| | ||| |||
31 4170 C     cATGCGgtcCAAACGcTCTaCAAAACGCACTAAACGTGCgTCtGCTACaCAATTaTATcAAA
              |  |   ||| |      |   | | |                  |
18 4222 tttgtattTttGtaatAAAaGtatggtAtccCaCcgTgccgcacgacgcaaacgggctTcggtaactg

con     tttgtatat-aga-acaAacgt-c-gcaagacgc-gtaaacgtgc-tc-gctacacaactatatcaaa
    O21-TTTGTATACCATGGCACATAGTAGGGCCCGACGACGCAAGCGTGCGTCAGCTACACAGCTATATCAAA-O21

6 4481 CATGtAAactCACTGGaACATGcCCCCCAGATGTAATTCCTAAgGTgGAgCAcAacACcATTGCAGAT
       |||| ||   |||||| ||||| |||||||||||||||||||| || || || |   || ||||||||
11 4472 CATGCAAGGCCACTGGtACATGtCCCCCAGATGTAATTCCTAAAGTtGAACAtAcTACtATTGCAGAT
        ||||||||||||| || || || || || ||||| ||||||||||| |||    | ||| || ||||||
33 4268 CATGCAAGGCCACAGGcACcTGcCCACCcGATGTTATTCCTAAAGTgGAAGGaAgTACcATaGCAGAT
        |||||||     |||| || || ||||| ||  |||| ||||| || ||||| |  || || || ||
16 4296 CATGCAAAcagGCAGGTACaTGTCCACCtGACaTTATACCTAAgGTtGAAGGcAaaACtATTGCtGAa
        |||| |||   |||||||| |||||| | ||| ||||||||||   | |||    | || ||||| ||
31 4233 CATGtAAAgcAGCAGGTACtTGTCCAtCaGACgTTATACCTAAaaTaGAACaTActACcATTGCaGAC
          |   |   | || |||        |          |  |||| |  | |  | ||        |   |
18 4290 acTtatAtaaAaCAtGTAaacaatCtggtacatgTccACCTgAtgTtGttCcTAaggtggagGcacC

con     caTgcaAagccaCagGtAcatgtcCaccagatgttat-CCTaAagTtGaacatAataccattGcagat
    O21-CATGTAAACTCACTGGAACATGCCCCCCAGATGTAATTCCTAAGGTGGAGCACAACACCATTGCAGAT-O21

6 4549 CAAATATTAAAATGGGGAAGtTTgGGGGTgTTTTTTGGaGGGTTgGGTATaGGcACgGGttCcGGcAc
11 4540 CAAATATTAAAATGGGGAAGcTTAGGGGTTTTTTTGGTGGGTTAGGTATTGGtACAGGggCTGGTAg
33 4336 CAAATtcTtAAATATGGcAGTTTAGGGGTTTTTTTGGTGGtTTAGGTATTGGcACAGGcTCTGGTtC
16 4364 CAAATATTAcAATATGGaAGTATGGGTGTaTTTTTGGTGGGTTAGGaATTGGaACAGGgTCgGGTAC
31 4301 CAAATATTAaggTATGGtAGTATGGGTGTtTTTTTGGTGGGTTgGGtATTGGgtCcGGCTCtGGTAC
18 4358 acgtTAgcAgataAaatattgcaatGgtcaagccTTGGTataTTtttgggTGGacttGGCataGGTAC

con caaaTattaaaatatggaagttt-gGggtttttTTGGtgggTTaggtattGG-acaGGctctGGtac
O21-CAAATATTAAAATGGGGAAGTTTGGGGGTGTTTTTTGGAGGGTTGGGTATAGGCACGGGTTCCGGCAC-O21

6 4617 TGGgGGTCGTaCtGGcTATgTtCCCTTacaAActTCTgCaAAaCCTtCTATTACTaGtGGGCCtatgG
11 4608 TGGcGGTCGTgCaGGgTATaTACCCTTgGGAAgcTCTCCcAAgCCTGCTATTACTgGgGGGCCagCaG
33 4404 AGGtGGAaGgACTGGcTATgTACCtaTtGGtACtgacCCaCCtACAGCTgCAAtccCctTGCagCCTa
16 4432 AGGcGGACGCACTGGgTATaTtCCatTgGGaACAaGgCCTCCcACAGCTaCAGAtaCAcTTgctCCTg
31 4369 TGGgGGTCGCACTGGaTATgTcCCtcTtaGtACACGtCCTtCTACAGtaTCtGAggCAagTaTaCCTa
18 4426 TGGcaGTgGtACaGGgggTcgtaCagggtacAttCcattgggTgggcgtTCcaAtaCAgtggTggaTg

con tGGcgGtcGtaCtGGgtaTgttcC-ttgggaAct-ctcc--ctacagctactaatacag-gcc-cctg
BE11-GAAGCXCXCCCAAGCCXGCXAXX-BE11
BE12-TATATACCCTTGGGAAGCXCXCCCAAGCCXGCXAX-BE12
O21-TGGGGGTCGTACTGGCTATGTTCCCTTACAAACTTCTGCAAAACCTTCTATTACTAGTGGGCCTATGG-O21

6 4685 CtCGTCCtCCtGTGgTgGTGGAGCCTGTgGCCCCTTCgGATCCaTCtATTGTGTCtTTAATTGAaGAa
11 4676 CACGTCCgCCaGTGcTTGTGGAGCCTGTTGCCCCTTCcGATCCcTCcATTGTGTCcTTAATTGAgGAg
33 4472 TACGTCCtCCgGTtACTGTAGAcaCTGTTGGaCCTTtaGActCgTCTATAGTGTCaTTAATaGAAGAA
16 4500 TAAGACCcCCttTaACaGTAGAtCCTGTgGGcCCTTctGAtCCtTCTATAGTtTCTTTAGTgGAAGAA
31 4437 TTAGACCaCCAgTTAgCaTtGAcCCTGTaGGtCCcTTGGACCCCTCTATAGTAagTcTTGTTGAAGAA
18 4494 TTgGtCCtaCAcgTccCccaGtggtTaTtGaaCCtgTGGgCCCCaCagacccAtcTaTTGTTacAttA

con t-cGtCCtcCagttac-gtaGagccTgTtGgcCCtt-gGa-cCctCtatagtgtcttTa-Ttgaagaa
BE26-CGXCCXCCGGXXACXGXAGAxA-BE26 JJ22-TCTATTGTGTCNTTAATNGAAGAA
BE27-GXCCXCCGGXXACXGXAGACACX-BE27 O22-GGATCCATCTATTGTGTCTTTAATTGAAGAA
BE28-XCCXCCGGXXACXGXAGACACXGXXGGACCXXXAG-BE28
O21-CTCGTCCTCCTGTGGTGGTGGAGCCTGTGGCCCCTTCGGATCC-O21

6 4753 TCgGCaATcATTAAcGCaGGgGCgCC TGAaaTtGTgCCCCC TgCACAcGGTGGgTTTAC
11 4744 TCtGCTATTATTAAtGCtGGTGCACC TGAggTgGTaCCCCC TACACAgGGTGGcTTTAC
33 4540 ACaAGTTTTATaGAgGCaGGTGCACCA GccCCaTCtATTCC TACACcATCAGGtTTga
16 4568 ACTAGTTTTATTGATGCTGGTGCACCAaCatCTGtaCCtTCcATTCCcCCagatgtATCAGGaTTTag
31 4505 tCTGGaaTTgTTGATGTTGGTGC ccCTGCTCCTAtaCCacacCCTCCTacaACATCTGGGTTTGA
18 4562 ataGaggactccagTGTgGtTaCatcaggTGCaCCTAggCCtacgttTaCTggcACgTCTGGGTTTGA

con -ctggtattatt-atGctGgtgCacca-ctgctgc-at--c--cccctcct-caccatctGGgTTT-a
TCTAGTNTTATTAATGCAGGTGCACC-JJ22
BE5-CAXXAACGCAGGGGCGCC------XGAA-BE5
BE6-GGCAAXCAXXAACGCAGGGGCG-BE6
BE7-GCAAXCAXXAACGCAGGGGCGCC------XGAAAXXGXGCC-BE7
O5-GTACCCCC TACACAGGGTGGCTTTAC
O22-TCGGCAATCATTAACGCAGGGGCGCC TGAAATTGTGCCCCC TGCACACGGTGGGTTTAC-O22

6 4812 aATtACATCcTCTGAAaCaACTACcCCTGCaATaTTgGATGT aTCaGTT ACtAgTCACACTA
11 4803 TATaACATCATCTGAAtCgACTACACCTGCtATTTTaGATGT gTCTGTT ACcAaTCACACTA
33 4599 TgTTACTACATCTGCAGATACTACACCTGCaATTATTAATGTttcaTCTGTTggggAgtcatCTATTc
16 4636 TATTACTACtTCaaCtGATACcACACCTGC tATATTA GATattaAtAatACTGTTA
31 4570 cATTgCTACaaCtGCaGAcACaACACCTGC aATTTTA GAT gtaACaAgTGTTA
18 4630 tATaaCatCtgCgGgtacaACtACACCTGCggtTTTggatatcacaccttcgtctacctCtgtgtcTA

con taTtaC--CatCtgcag--ACtACaCCTGCaatttTt-atgt--catctgtt--tac-act--ta-Ta
O5-TATAACATCATCTGAATCGACTACACCTGCTATTTTAGATGT GTCTGTT ACCAATCACACTA-O5
O22-AATTACATCCTCTGAAACAACTACCCCTGCAATATTGGATGT ATCAGTT ACTAGTCACACTA-O22

6 4874 CtACTA GTaTaTTTagAAATCCtgTcTTTACAGAACCtTCTGTAAcACAaCCCCAACCACCcGTG
11 4865 CcACTA GTgTgTTTcaAAATCCCcTgTTTACAGAACCgTCTGTAATACAgCCCCAACCACCTGTG
33 4667 aaACTATTtCTACACATttAAATCCCACaTTTACTGAACCATCTGTAcTACAcCCTCCAgCgCCTGCA
16 4692 ctACTgTTaCTACACATaAtAATCCCACTTTcACTGAcCCATCTGTATTGCAGCCTCCAACACCTGCA
31 4623 gCACACATgAaAATCCTACTTTTACTGATCCATCTGTATTGCAGCCTCCtACACCTGCA
18 4698 tttccacaacCAatttTaccAATCCTgCaTTTtCTGATCCgTCcaTtaTtgAagtTCCacaAaCTGgg

con ctactatta-taca--TaaaAATCC-ac-TTtaCtGAaCCaTCtgTaatacAgcctCcaccacCtGc-
O5-CCACTA GTGTGTTTCAAAATCCCCTGTTTACAGAACCGTCTGTAATACAGCCCCAACCACCTGTG-O5
O22-CTACTA GTATATTTAGAAATCCTGTCTTTACAGAACCTTCTGTAACACAACCCCAACCACCCGTG-O22
O27-CTGCA

6 4939 GAGGCtAaTGGaCAtATAtTaATtTCTGCaCCcACtgTAACgTCaCAcCcTaTAGAgGAaATTCCttT
||||| | ||| || ||| | || ||||| || ||   |||| || || | | |||| || |||||  |
11 4930 GAGGCCAgTGGtCAcATAcTtATaTCTGCCCCaACaaTAACaTCcCAACaTgTAGAAGACATTCCAcT
|| |||  ||| ||   |   |  | ||| |||| ||   | |    | |||   |     ||| |||| ||| |
33 4735 GAAGCCtcTGGaCATTTTATAtTTTCTTCCCCtACTgTTAGcACACAAAgTTATGAAaACATaCCAAT
||| |      || ||||||| | |||| ||   | |||  |||| ||||| | ||||||| | || || ||
16 4760 GAAACtggAGGgCATTTTAcACTTTCATCATCcACTATTAGtACACATAATTATGAAGAAATtCCTAT
|||||    ||| |||||    ||||||||||||  ||||||| |||||||||||||| ||||| |||||
31 4682 GAAACatCAGGTCATTTAcTACTTTCATCATCatCTATTAGcACACATAATTATGAGGAAATACCTAT
||      ||||| || || |  ||       |  |  |   | | ||||||    |||||||||||||| |
18 4766 GAggtggCAGGTaATgTAtTtgTTggtaCccCtaCatcTgGaACACATgggTATGAGGAAATACCTtT

con GA-gc--c-GGtcAttTa-ta-TttcttC-cC-aCtattag-aCaCAtaattatGA-gAaAT-CCtaT
O5-GAGGCCAGTGGTCACATACTTATATCTGCCCCAACAATAACATCCCAACATGTAGAAGACATTCCACT-O5
O22-GAGGCTAATGGACATATATTAATTTCTGCACCCACTGTAACGTCACACCCTATAGAGGAAATTCCTTT-O22
O27-GAAGCCTCTGGACATTTTATATTTTCTTCCCCTACTGTTAGCACACAAAGTTATGAAAACATACCAAT-O27

6 5007 AGAtACTTTTGTgGTATCaTCTAGTGATAGcGGtCCTACATCCAGTACcCCTgTTCCTgGTaCTgcaC
||| |||||||| ||||| ||||||||||| || |||||||||||||| ||| ||||| || ||     |
11 4998 AGAcACTTTTGTTGTATCCTCTAGTGATAGTGGaCCTACATCCAGTACtCCTcTTCCTcGTgCTtttC
|| || |||||||| ||| |        ||||        ||||| || || ||  ||||   |   ||   |
33 4803 GGATACcTTTGTTGTTTCCACAgACagTAGTAatGTAACATCaAGCACgCCCATTCCAGGGTCTCGCC
|||||| ||| |||||  |||| ||  ||  |  |||||      ||||| ||||| ||||||||||||
16 4828 GGATACATTTATTGTTagCACAAACccTAAcAcaGTAACtAGTAGCACACCCATaCCAGGGTCTCGCC
|||||||||||||||     || ||     | | |     |||| |||||||||||||| ||||||    ||||
31 4750 GGATACATTTATTGTTTCTACTAAtaaTGAaAAcaTAACaAGTAGCACACCCATtCCAGGGGTGCGCC
| ||||||  |   ||||  ||        || |      |  ||||| || ||   | ||      ||||| |
18 4834 acAaACATTTgcTtcTTCTggTAcggggGAggAacccAttAGTAGtACcCCatTgCCtactGTGCGgC

con -gAtACaTTTgttgtttccactaatgata---aac-aAca--tAG-AC-CCcaTtCC-gg-gctcgcC
O5-AGACACTTTTGTTGTATCCTCTAGTGATAGTGGACCTACATCCAGTACTCCTCTTCCTCGTGCTTTTC-O5
O22-AGATACTTTTGTGGTATCATCTAGTGATAGCGGTCCTACATCCAGTACCCCTGTTCCTGGTACTGCAC-O22
O27-GGATACCTTTGTTGTTTCCACAGACAGTAGTAATGTAACATCAAGCACGCCCATTCCAGGGTCTCGCC-O27

6 5075 CTCGGCCTCGtGTGGGccTaTATAGTCGTGCaTTgCAcCAGGTgCAGGTTACaGACCCtGCaTTTCtt
|||||||||| |||||   | ||||||||||| || || ||||| |||||||| ||||| || ||| |
11 5066 CTCGGCCTCGgGTGGGTTgTATAGTCGTGCcTTaCAgCAGGTACAGGTTACgGACCCcGCgTTTTTg
|| | | ||  | ||||| ||||||||          |        ||||||||   |        |
33 4871 CTGTGGCACGCCTtGGTTTATATAGTCG      CAAtACcCAACAGGTTA AGGTTGTtGACCCTGC
| ||||||||||| || |||||||||||      ||   || |||||||||| | ||||| ||||||||
16 4896 CaGTGGCACGCCTAGGaTTATATAGTCG      CACaACACAACAGGTTA AAGTTGTaGACCCTGC
|||||  |||| |||||||||      | |||||||| || |  ||||| | || ||  |
31 4818 GTcctGCACGTtTAGGgTTATATAGT      AaGGCtACACAACAAGTaA AAGTTaTTGAtCCaaC
||   ||| ||     | | || |||      | |||     ||||||||      |||    | | ||
18 4902 GTgtaGCAgGTccccGccTtTAcAGT      AgGGCctacCAACAAGT gtcAGTggcTaAcCCtga

con ct-tggCacGtct-gG-tTaTAtAGTcgtgc-atg---a--caaCAgGTtaca-gttgttga-cctgc
O5-CTCGGCCTCGGGTGGGTTGTATAGTCGTGCCTTACAGCAGGTACAGGTTACGGACCCCGCGTTTTTG-O5
O22-CTCGGCCTCGTGTGGGCCTATATAGTCGTGCATTGCACCAGGTGCAGGTTACAGACCCTGCATTTCTT-O22
O27-CTGTGGCACGCCTTGGTTTATATAGTCG      CAATACCCAACAGGTTA AGGTTGTTGACCCTGC-O27

6 5143 TCCACtCCtCAaCGcTTaaTtACaTAT GAtAACCCTGTaTATGAA GGgGAgGATG
11 5134 TCCACgCCaCAgCGaTTggTAACTTAT GAcAACCCTGTcTATGAA GGaGAaGATG
33 4932 TTTTtTAACatCgCCTcaTAAACTTATaACATATGATAATCCTGCATtTGAAaGctTtGAccctGAaG
16 4957 TTTTgTAACcaCTCCcAcTAAACTTATTACATATGATAATCCTGCATATGAAgGTaTAGATGtgGAta
31 4879 GTTTCTTAgtgCTCCAAaacAgCTAATTACATATGAaAACCCTGCcTATGAAacTgTAaATGCtGAaG
18 4963 GTTTCTTAcacgTCCAtcctcttTAATTACATATGAcAACCC gGCctttG

con ttttct-accactcctta-taacttATtacatatGAtAAcCCtgcatatgaaagt-taga-gc-gatg
O5-TCCACGCCACAGCGATTGGTAACTTAT GACAACCCTGTCTATGAA GGAGAAGATG-O5
O22-TCCACTCCTCAACGCTTAATTACATAT GATAACCCTGTATATGAA GGGGAGGATG-O22
O27-TTTTTTAACATCGCCTCATAAACTTATAACATATGATAATCCTGCATTTGAAAGCTTTGACCCTGAAG-O27

6 5198 TtAGTgTACAATTTAgtCATGAtTCTA TaCACAATGCACCTGATGAgGCtTTTATGGAcATa
11 5189 TaAGTTTACAATTTAccCATGAgTCTA TcCACAATGCACCTGATGAaGCaTTTATGGATATT
33 5000 AcACATTACAATTTcaaCATAgTGATA TatcaccTGCTCCTGATCCTGACTTTcTaGATATT
16 5025 AtACATTATAtTTTTCtagTAaTGATAatagtaTTAATATAGCTCCaGATCCTGACTTTtTgGATATa
31 4947 AatCTTTATACTTTTC caATAcatCgCaTAATATAGCcCCTGATCCcGACTTTcTaGATATT
18 5013 AgcCTgTggACacTaCattaacattTgatcCtCgTAgTgatGttCCTGATtCaGAtTTTaTgGATATT

con a-actttTacAattTac-cataattaTaat-ctcttaataatGctCCtGATcc-GacTTTaTgGAtATt
O5-TAAGTTTACAATTTACCCATGAGTCTA TCCACAATGCACCTGATGAAGCATTTATGGATATT-O5
O22-TTAGTGTACAATTTAGTCATGATTCTA TACACAATGCACCTGATGAGGCTTTTATGGACATA-O22
O27-ACACATTACAATTTCAACATAGTGATA TATCACCTGCTCCTGATCCTGACTTTCTAGATATT-O27

6 5260 ATTcGttTgCAcAGACCtGCcATtgCGTCCcGACGtGGcCTTGTGCGgTacAGTCGCATTGGaCAACG
11 5251 ATTaGAcTACATAGACCaGCTATaACGTCCaGACGgGGTCTTGTGCGTTTTAGTCGCATTGGgCAACG
33 5062 ATTGCATTACATAGGCCtGCTATtACatCTcGtaGacaTacTGTGCGTTTTAGTAGAgTaGGTCAAAA
16 5093 gTTGCtTTACATAGGCCaGCatTaACCTCTaGgcGtAcTggcaTTAGgTAcAGTAGAaTtGGTAATAA
31 5009 ATaGCaTTACATAGGCCTGCccTtACCTCacGtaGgAacACTGTTAGaTATAGTAGAcTAGGTAATAA
18 5081 ATccgtcTACATAGGCCTGCttTaACaTCcaGgcGtgggACTGTTcGcTtTAGTAGAtTAGGTcAacg

con aTtg--tTaCAtAGgCCtGCtaT-aC-TCc-G-cGtggtactgT-cG-T-tAGTaGaaT-GGtcAa--
JJ24-TACATAGGCCTGCTATAACNTCCAGNCGTGGTNNTGTGCGNTTTAGTAGA-JJ24
O5-ATTAGACTACATAGACCAGCTATAACGTCCAGACGGGGTCTTGTGCGTTTTAGTCGCATTGGCAACG-O5
O16-ACTGTGCGTTTTAGTAGAGTAGGTCAAAA-O16
O22-ATTCGTTTGCACAGACCTGCCATTGCGTCCCGACGTGGCCTTGTGCGGTACAGTCGCATTGGACAACG-O22
O27-ATTGCATTACATAGGCCTGCTATTACATCTCGTAGACATACTGTGCGTTTTAGTAGAGTAGGTCAAAA-O27
O28-GTAGACATACTGTGCGTTTTAGTAGAGTAGGTCAAAA

6 5328 GGGGTCtATGcACACtCGCAGcGGAaAgCAcATAGGgGCCCGCATtCATTATT
|||||| ||| |||| ||||| ||| | || ||||| |||||||| |||||||
11 5319 GGGGTCcATGtACACaCGCAGTGGAcAACAtATAGGtGCCCGCATACATTATT
| | | | || |||||||| |||| || || || | ||||||||||
33 5130 AGccACACTtaAaACTCGCAGTGGtAAACAaATtGGaGCTAGaATACATTATTATcAgGATTTAAGTc
| ||||| ||||| ||||| ||| || || |||| ||||||||||| | |||||||||
16 5161 ACAAACACTaCGtACTCGTAGTGGaAAAtCTATaGGTGCTAaGGTACATTATTATTATGATTTAAGTA
|||||| | || ||||||||||| |||| ||||| | ||| ||||||||||||||| | ||||
31 5077 ACAAACTtTGCGcACTCGTAGTGGTgCtaCTATtGGTGCaAGGGTgCATTATTATTATGATATtAGTA
|||| || || || || ||| | || ||||| ||||| || | |||| |||||||| |||
18 5149 ggcAACTaTGtttACcCGcAGcGGTaCacaaATaGGTGCtAGGGTtCAcTtTTATcATGATATaAGTc

con -g-aaCtaTgcacACtCGcAGtGG-aaacatATaGGtGCtagg-TaCAtTaTTatcatgatataagta
O5-GGGGTCCATGTACACACGCAGTGGACAACATATAGGTGCCCGCATACATTATT(-O5)
O16-AGCCACACTTAAAACTCGCAGTGGTAAACAAATTGGAGCTAGAATACATTATTATCAGGATTTAAGTC-O16
O22-GGGGTCTATGCACACTCGCAGCGGAAAGCACATAGGGGCCCGCATTCATTATT(-O22)
O27-AGCCACACTTAAAACTCGCAGTGGTAAACAAATTGGAGCTAGAATACATTATTATCAGGATTTAAGTC-O27
O28-AGCCACACTTAAAACTCGCAGTGGTAAACAAATTGGAGCTAGAATACATTATTATCAGGATTTAAGTC-O28

6 5381 TTtAtGAtATTTCACCtaTTgCACAgGCTGCAGAaGAAATAGAAaTGCACCCTCTtGTGG
|| | || |||||||| || |||| |||||||| ||||||||| |||||||||| ||||
11 5372 TTcAgGAcATTTCACCagTTaCACAAGCTGCAGAgGAAATAGAAcTGCACCCTCTAGTGG
| | ||| | | || || ||| | | |
33 5198 CTATTG TgcCtttAGAcCACaccgTgCcAAATgaACAAtaTgAATtAcAgcCTttaCaTgAtacT
|||||| | ||| | | || | | | | | | || | |
16 5229 CTATTGATCCTGCAGaAGAAAtagaatTACaAAcTatAacAccTtCtaCAtAtACTACcACTTCacaT
|||| ||||||||| |||| | | | | ||||| ||||| |
31 5145 gTATTaATCCTGCAGgtGAAAgTATTGAAaTGCAaCCTTTAGgggCgTCTGCaACTACtACTTCtacT
|||| ||| | ||| ||||||| |||| ||||||| ||||| |
18 5217 cTATTgcaCCTtCcccaGAAtaTATTGAAcTGCAgCCTTTAG taTCTGC caCggag

con ctattgatc-t-c---agaacacat--taca-aagct-caa--g-aatc--aa-ctaccc--tcg---
(O5-)TTCAGGACATTTCACCAGTTACACAAGCTGCAG-O5
O16-CTATTG TGCCTTTAGACCACACCGTGCCAAATGAACAATATGAATTACAGCCTTTACATGATACT-O16
O22- TTTATGATATTTCACCTATTGCACAGGCTGCAG-O22
O27-CTATTG TGCCTTTAGACCACACCGTGCCAAATGAACAATATGAATTACAGCCTTTACATGATACT-O27
O28-CTATTG TGCCTTTAGACCACACCGTGCCAAATGAACAATATGAATTACAGCCTTTACATGATACT-O28

6 5441 CTGCAcAggATGAtACaTTTGATATTTATGCTGAAtCtTTTGAaCCTggCatTaACCCTacCCAACAc
||||| | |||| || ||||||||||||||||||| | ||||| ||| | | ||||| ||||||
11 5432 CTGCAgAaaATGAcACGTTTGATATTTATGCTGAAcCATTTGAcCCTatCccTgACCCTgtCCAACAT
|| | | | || | | | |
33 5263 tCtaCaTCgtCTtaTaGTATTAATgATGG tTTgTATGATgTTTATGC TgaCgAtGT
| | || || | ||||||| |||| || |||||| ||||||| | |
16 5297 gCAgCcTCacCTacTTcTATTAATaATGGA TTaTATGATaTTTATGCaGATgacttTattACAGA
| | | | | | | |||| || || ||| | ||| |
31 5213 ttAAATGAtggCTTaTaTGAcATTTATGCAGA CAcTGATtTTaCTGTGGATacACCTgCcACAcA
||||| ||| | ||| || |||||||| || || | | ||| | ||| |
18 5273 gacAATGA CTTgTtTGAtATaTATGCAGAtgaCAtgGAcccTgCaGTGccTgtACCatCgcgttc

con -t-aa--a-a---tat-T-ttAt-taTg-acag-ac-atgatatt--tgc---taccctt-ccaa---
O16-TCTACATCGTCTTATAGTATTAATGATGG TTTGTATGATGTTTATGC TGACGATGT-O16
O27-TCTACATCGTCTTATAGTATTAATGATGG TTTGTATGATGTTTATGC TGACGATGT-O27
O28-TCTACATCGTCTTATAGTATTAATGATGG TTTGTATGATGTTTATGC TGACGATGT-O28

6 5509 cCTGTTACAaatatatcagAtaCaTATtTaACtTCCACACCTAATACagTTaCACAAcCGTGGGGTAA
|||||||||          |  | ||| | || ||||||||||||||  || ||||| ||||||||||
11 5500 tCTGTTACA          cAgtCtTATCTtACcTCCACACCTAATACCcTTtCACAAtCGTGGGGTAA
|  |    || |          |   | |     |  |   |    ||
33 5319 ggaTaaTgtAcaCaCcccAAtgCaacaCTCATacAgtaCgTTtgCAaCaacaCgTACcaGcAATGTgt
|  |  |  |  | |   |          | | |   | ||  ||          ||   |||
16 5362 TAcTtcTaCAaCCcCggtAcCatctgtacCCTCtACatCTTTaTCAGgtTATaTTCCTgCAAATACaA
|| |  |  |  |  |          || |  |  || | || |  ||| | ||| ||||||| |
31 5278 TAaTgtTTCCcCTtCtacTgCTgtAcAgTCCaCatCTgCTgTgTCTGCCTATgTaCCTACAAATACcA
|| |   |||  | |   |  |  | | ||  |  ||          ||||||| |      ||  |||  |
18 5338 TAcTaccTCCttTgCattTttTaaAtAtTCgcCcaCTatatctTCTGCCTcTtcctaTAgtAATgtaA

con ta-tttt-ca---tctc--attca----tcacctac--c-ttatcagcct-tc-ca-tagtaatgtaa
O16-GGATAATGTACACACCCCAATGCAACACTCATACAGTACGTTTGCAACAACACGTACCAGCAATGTGT-O16
O27-GGATAATGTACACACCCCAATGCAACACTCATACAGTACGTTTGCAACAACACGTACCAGCAATGTGT-O27
O28-GGATAATGTACACACCCCAATGCAACACTCATACAGTACGTTTGCAACAACACGTACCAGCAATGTGT-O28

6 5577 cACCACAGTtCCATTGTCAcTtCCTAaTGACctGTTTtTaCAaTCTGGcCCTGAtATAACTTTTCCTA
|||||||| ||||||||| | |||| ||||  |||| | || ||||| ||||| ||||||||||||
11 5559 tACCACAGTcCCATTGTCAATcCCTAgTGACTGGTTTGTgCAGTCTGGgCCTGACATAACTTTTCCTA
||  |  ||   ||          ||| | |||    | |  |   |  |  | |||  |
33 5387 CtATACCTTTaaATacaGgATttgATAcTCCTGTTaTGTCtGGcCCTGATATACCtTccCcTTTAtTT
| || |||||   |   | ||   ||| |||| |  | || || ||||||||||      | || |
16 5430 CaATtCCTTTtgGTggtGcATacaATATTCCTtTAgTaTCaGGtCCTGATATACCcATtaATaTAaCT
|  | ||  |  ||   |  |   |||||  || | || || |||||| |||| ||  |    |
31 5346 CtGTgCCacTAAGTaCaGgTTtGAcATTCCcaTATTtTCtGGgCCTGATgTACCtATagAgCATgCA
| || ||  |||   |   ||  ||  |  | ||  |||    | || ||||||   |       | ||| |
18 5406 CgGTcCCttTAAcctCctcTTggGAtgTgCCtgTATacaCgGGtCCTGAT  AttacattAcCATctA

con ca-taCctttaaatt-tg-aTtcgatat-cCtgt-tt-tc-ggtCctGat-taccataacattt-cta
O16-CTATACCTTTAAATACAGGATTTGATACTCCTGTTATGTCTGGCCCTGATATACCTTCCCCTTTATTT-O16
O27-CTATACCTTTAAATACAGGATTTGATACTCCTGTTATGTCTGGCCCTGATATACCTTCCCCTTTATTT-O27
O28-CTATACCTTTAAATACAGGATTTGATACTCCTGTTATGTCTGGCCCTGATATACCTTCCCCTTTATTT-O28

6 5645 CTGCAcCTATGGGAACACCCTTTAGTCCTGTAACTCCTGCTTTACCTACAGGCCCTGTTTTcATTACA
||||| ||||||||||||||||||||||||||||||||||||||||||||||||||||||| ||||||
11 5627 CTGCAtCTATGGGAACACCCTTTAGTCCTGTAACTCCTGCTTTACCTACAGGCCCTGTTTTTATTACA
|      |  |              |||| |  | |||  ||| |||   | | |  || || |
33 5455 CcCacAtCTaGccCATTtgT          TCCTATttCgCCTttTTTTCCTtttGACACcATTgTTGTaGAc
|  | ||   ||||  |          ||||||   ||   |  |||      | || ||| |||  ||
16 5498 gaCcaAgCTccTTCATTAaT          TCCTATaGttCCagggTCTCCACAAtAtACaATTaTTGcTGAT
|     ||    | |          |||| | |  ||     | ||||||    | ||| ||| ||||
31 5414 CctaCACaGgtTTtCCCATT          TCCTtTGGCCCCTaCaaCgCCACAAgtGTCTATTtTTGTTGAT
|   |  |  |  |  ||||||          ||   ||||||| |  |  ||||    || ||||  | |  ||
18 5472 CtacCtCtGtaTggCCCATTgtatcaCCcacGGCCCCTgCctCtaCACA    GTaTATTggTaTacAT

con ct-c-act-tgtg-ac-a-ttttagtCCtatagctCCtgctt-tcC-caag-c-ctaTTttt-ttgat
BE22-CCCAXXGXAXCACCCACGGCCC-BE22
BE23-CCCAXXGXAXCACCCACGGCCCCXGCCXCXACACA-BE23
O16-CCCACATCTAGCCCATTTGT          TCCTATTTCGCCTTTTTTTCCTTTTGACACCATTGTTGTAGAC-O16
O27-CCCACATCTAGCCCATTTGT          TCCTATTTCGCCTTTTTTTCCTTTTGACACCATTGTTGTAGAC-O27
O28-CCCACATCTAGCCCATTTGT          TCCTATTTCGCCTTTTTTTCCTTTTGACACCATTGTTGTAGAC-O28

6 5713 GGTTCTGgaTTtTATTTGCATCCTgCATGGTAtTTTGCACGtAaACGCCGTAAACGTATTCCCTTATT
||||||| || |||||||||||| |||||| |||||||| | ||||||||||||||||||||||||
11 5695 GGTTCTGACTTcTATTTGCATCCTACATGGTAcTTTGCACGcAGACGCCGTAAACGTATTCCCTTATT
||| |||||||   ||| |||||||  |   |   ||  |||  |   | ||||||||| |||| |  ||
33 5518 GGTGCTGACTTTgtTTTACATCCTAGTTATTtATTTACGtcGcaGgCGTAAACGTTTTCCATATTT
|  | |||||||  ||||||||||||||||| || |||||      | |||||||||| |||||||
16 5561 GcaGGTGACTTTATTTACATCCTAGTTATTAcATGTTACGAaaaCGACGTAAACGTTTACCATATTT
|  ||||| |||||||| || |||||||||||| |||||| |    |||||||||||| || |||||||
31 5477 GGgGGTGATTTTTATTTGCAcCCTAGTTATTATATGTTAaaAcgtCGACGTAAACGTGTAtCATATTT
||     ||| |||||||    ||      |||||| |  |          ||||||||||| | |||||
18 5537 GGtacacATTaTTATTTGtggCCattaTATTATtTtaTtcctaagaaACGTAAACGTGTtcCcTATTT

con GgtgctgacTtttaTTTgcatCCtag-TatTat-Ttttacgta-acgaCGTAAACGT-TtcC-TatTT
JJ25-CGTAAACGTNTTCCCTATTT
PCR2-CGTTTTCCATATTT
O27-GGTGCTGACTTTGTTTTACATCCTAGTTATTTATTTTACGTCGCAGGCGTAAACGTTTTCCATATTT-O27

6 5781 TTTTtCAGATGT GGCGGCCTAGCGACAGCACAGTATATGTGCCTCCTCCtAACCCTGTATCC
|||| |||||||          |||||||||||||||||||||||||||||||| |||||||||||
11 5763 TTTTACAGATGT GGCGGCCTAGCGACAGCACAGTATATGTGCCTCCTCCcAACCCTGTATCC
||||||||||||          |||||||||| ||   |||||| ||  ||||||||        ||||||||
33 5586 TTTTACAGATGTCcgTgTGGCGGCCTAGTGAGGCCACAGTgTACcTGCCTCCT GTaCCTGTATCT
|||| ||||||||  | |||| ||||||||||||||| || ||| ||||||||   || || ||||||
16 5629 TTTTtCAGATGTCTCTtTGGCtGCCTAGTGAGGCCACTGTCTACTTGCCTCCT GTCCCAGTATCT
|||| ||||||||||| |||| |||||| ||||| |||||||||| || |||   |||||||| |||
31 5545 TTTTaCAGATGTCTCTGTGGCGGCCTAGcGAGGCTACTGTCTACTTaCCACCT GTCCCAGTGTCT
|||| |||||| || ||||||||||||| ||    ||| || ||  | ||||||      | ||| |
18 5605 TTTTgCAGATGgCTtTGTGGCGGCCTAGtGAcaaTACcGTaTAtcTtCCACCT ccttCtGTGgCa

con TTTTaCAGATGtctctgtGGCgGCCTAG-GA--ccACaGTaTA--TgCCtCCTcc-gtccCtGTatCt
TTTTNCAGATGTCTNTGTGGCGGCCTAGTGA-JJ25
PCR1-CAGATGTCTCTGTGGCGGCCTAGTG-PCR1
TTTTGCAGATG-PCR2
O27-TTTTACAGATGTCCGTGTGGCGGCCTAGTGAGGCCACAGTGTACCTGCCTCCT GTACCTGTATCT-O27

6 5843 AAaGTTGTTGCCACGGATGCtTATGTTActCGCACCAACATATTTTATCATGCCAGCAGTTCTAGACT
|| ||||||||||||||||| |||||||  ||||||||||||||||||||||||||||||||||||
11 5825 AAgGTTGTTGCCACGGATGCgTATGTTAaaCGCACCAACATATTTTATCATGCCAGCAGTTCTAGACT
|| |||||   ||| ||||  |||||    ||||| | ||| | |||| ||||   | ||||| |||||
33 5651 AAaGTTGTcAGCACtGATGAATATGTgtCtCGCACAAgCATtTATTATtATGCtGGtAGTTCCAGACT
|| ||||| ||||| ||||||||||| | ||||||| ||| |||||| |||| || |  ||||||||
16 5694 AAgGTTGTAAGCACGGATGAATATGTTgCACGCACAAACATATATTATCATGCAGGaAcaTCCAGACT
|| |||||||||||||||||||||   |||| || |||||||||||||| ||||| |    | || ||
31 5610 AAAGTTGTAAGCACGGATGAATATGTaACACGaACcAACATATATTATCAcGCAGGCAGtgCTAGgCT
| ||||||||  || ||||| ||||| || |  || | ||||| |||||| || |||||  ||||  |
18 5670 AgAGTTGTAAatACcGATGAtTATGTgACtCccACaAgCATATtTTATCAtGCtGGCAGctCTAGatT

con AaaGTTGTaagcACgGATGaaTATGTtac-CgcAC-AaCATaT-TTATcAtGC-gGcAgttCtAGacT
JJ26-GTTGTNANCACGGATGANTATGTTACTCGCACAA-JJ26
PCR3-AAGTTGTAAGCACCGATGAATATGT-PCR3
LCR1A-AAGTTGTAAGCACGGATGAATATGT-LCR1A
LCR1B-TGCACGCACAAACATATATTATCA-LCRB
LCR1B'-ACGTGCGTGTTTGTATATAATAGTA-LCRB'
O27-AAAGTTGTCAGCACTGATGAATATGTGTCTCGCACAAGCATTTATTATTATGCTGGTAGTTCCAGACT-O27
6 5911 tCTTGCaGTGGGACATCCtTATTttTCcATaAAA cggGcTAA C AAAA CtGTTGTgC

11 5893 cCTTGCTGTGGACATCCATATTacTCTATcAAAAA    aGtTAA    C      AAAA CAGTTGTAC
33 5719 tCTTGCTGTTGGcCATCCATATTTTCTATTAAAAAtCCTAcTAA    CgctAAAAAAtTATTgGTAC
16 5762 aCTTGCAGTTGGaCATCCcTATTTTcCTATTAAAAAACCTAACAAT    AAcAAAA    TATTaGTtC
31 5678 gCTTACAGTaGGcCATCCATATTaTtCcATacctAAAtCTGACAATccTAAaAAAA    TAGTTGTaC
18 5738 atTaACtGTtGGtaATCCATATT        TtagggttcCTGcaggTggTggcAAtAagcagGaTaTtC

con     -cTtgC-GTtGGacATCCaTATTttctaTtaaaaacctgctaat--caacaaaAaa-tagttgTaC
          JJ27-GTTGGACATCCATATTTT-JJ27
    O27-TCTTGCTGTTGGCCATCCATATTTTCTATTAAAAATCCTACTAA    CGCTAAAAAATTATTGGTAC-O27

 6 5967 CAAAGGTGTCaGGATATCAATAcAGgGTaTTTAAGGTgGTGTTaCCAGATCCTAACAAaTTTGCATTg
11 5949 CAAAGGTGTCtGGATATCAATATAGaGTgTTTAAGGTaGTGTTgCCAGATCCTAACAAgTTTGCATTa
33 5784 CcAAAGTATCAGGcTTgCAATATAGGGTtTTTAGGGTcCgTTTACCAGATCCTAATAAaTTTGGATTT
16 5824 CtAAAGTATCAGGATTACAATAcAGGGTATTTAGaaTaCaTTTACCtGAcCCcAATAAgTTTGGtTTT
31 5743 CaAAGGTgTCAGGATTACAATATAGGGTATTTAGGGTtCgTTTACCaGAtCCAAAcAAATTTGGaTTT
18 5800 CtAAGGTtTCtGcATacCAATATAGaGTATTTAGGGTgCagTTACCtGAcCCAAAtAAATTTGGtTTa

con     CaAAgGTgTCaGgaT--CAATAtAGgGTaTTTAgggT-c--TTaCCaGAtCCtAA-AAaTTTGgaTTt
                    JJ28-CAATATAGGGTATTTAGGGTNCNGTTACC-28 30-AATAAATTTGGATTN
                    PCR4-GTTATATCCCATAAATCCCATGTTAA-PCR4PCR5-TTATTTAAACCAAAA
    O27-CCAAAGTATCAGGCTTGCAATATAGGGTTTTTAGGGTCCGTTTACCAGATCCTAATAAATTTGGATTT-O27

 6 6035 CCTGAcTCgTCtCTtTTcGAtCCCACaACACAaCGTTTAGTATGGGCaTGCACAGGccTaGAGGTgGG
11 6017 CCTGAtTCaTCCCTgTTTGACCCCACTACACAgCGTTTAGTATGGGCgTGCACAGGgtTgGAGGTAGG
33 5852 CCTGACACCTCCTTTTATAACCCtGATACACAaCGaTTAGTATGGGCaTGTGTAGGccTTGAaaTAGG
16 5892 CCTGACACCTCaTTTATAATCCaGATACACAgCGgcTgGTTTGGGCCTGTGTAGGTgTTGAGGTAGG
31 5811 CCTGATACaTCTTTTTATAATCCTGAAACtCAACGcTTAGTTTGGGCCTGTGTTGGTtTaGAGGTAGG
18 5868 CCTGATACtagTaTTTATAATCCTGAAACaCAACGtTTAGTgTGGGCCTGTGcTGGagTgGAaaTtGG

con     CCTGA-aC-tcttTtTataAtCCtga-ACaCAaCGttTaGTaTGGGCcTGtg-aGG--T-GAggTaGG
        CCTGACACCTCNNTTTATAAT-JJ30
        GGACTGTGGA-PCR5
    O27-CCTGACACCTCCTTTTATAACCCTGATACACAACGATTAGTATGGGCATGTGTAGGCCTTGAAATAGG-O27

  6 6103 CAGGGGaCAgCCaTTAGGtGTgGGTGTaAGTGGaCATCCtTTcCTAAAtAAATATGATGATGTtGAAA
         |||||| || || ||||| || ||||| ||||| ||||| || ||||| |||||||||||||| ||||
 11 6085 CAGGGGtCAaCCtTTAGGCGTTGGTGTtAGTGGgCATCCaTTgCTAAACAAATATGATGATGTaGAAA
          || || || || |||||||||||  | ||||| ||||| ||  ||||||||| ||||||    ||||
 33 5920 TAGaGGgCAGCCATTAGGCGTTGGCATaAGTGGtCATCCTTTATTAAACAAATTTGATGACACtGAAA
         | | || ||||||||||| || ||||| ||||| |||||||||||||| ||||| |||||||| ||||
 16 5960 TCGtGGtCAGCCATTAGGTGTgGGCATTAGTGGcCATCCTTTATTAAATAAATTgGATGACACaGAAA
         ||| || |||||||||||||| || |||||||| ||||| |||||||||||||| |||||||| ||||
 31 5879 TCGcGGgCAGCCATTAGGTGTaGGtATTAGTGGtCATCCATTATTAAATAAATTtGATGACACTGAAA
          || || ||||| |||||||| ||  ||||||| |||||||| |  |||||||| |||||||||||||
 18 5936 cCGtGGtCAGCCtTTAGGTGTtGGccTTAGTGGgCATCCATTtTatAATAAATTaGATGACACTGAAA

con      --G-GGtCAgCCaTTAGGtGTtGG-aTtAGTGG-CATCC-TTattaAAtAAATttGATGAcactGAAA
     O27-TAGAGGGCAGCCATTAGGCGTTGGCATAAGTGGTCATCCTTTATTAAACAAATTTGATGACACTGAAA-O27
  6 6171 AT   tcaGGGagTGGTGGTAAcCCTGGaCAGGATAAcAGGGTTAATGTAGGTATGGATTATAAACAA
         ||      |||  ||||||||| ||||| |||||||| ||||||||||||||||||||||||||||||
 11 6153 ATaGTggtGGGTATGGTGGTAAtCCTGGTCAGGATAATAGGGTTAATGTAGGTATGGATTATAAACAA
            ||     ||||  |||  | || |||   ||||||||||    | ||   |||||||||||||||
 33 5988 ccGGTAacaaGTATcCTGGAcAaCCgGGTGctGATAATAGGGAATGTtTATCcATGGATTATAAACAA
           | ||     ||| | | | |  | ||||  |||||||| |||||| |||| ||||||||| |||||
 16 6028 AtGCTAgTgctTATGCaGcAaATgCaGGTGtgGATAATAGaGAATGTATATCtATGGATTAcAAACAA
         |  ||| |   ||||| |    | | ||    |||||||| ||||||||||| ||||||||| |||||
 31 5947 AcTCTAATagaTATGCcGgTggTcCTGGcactGATAATAGGGAATGTATATCaATGGATTATAAACAA
           ||  ||       |   |  |  |      ||   ||||||   | | ||  | |||||||| ||
 18 6004 gtTCccATgccgccaCgtcTaaTgTtctgagGAcgtTAGGGAcaaTgTgTCtgTaGATTATAAgCAg

con      at-ctaatgggtatgctggtaatcctggtgagGAtaatAGgGaa--TgTatctaTgGATTAtAAaCAa
     O27-CCGGTAACAAGTATCCTGGACAACCGGGTGCTGATAATAGGGAATGTTTATCCATGGATTATAAACAA-O27


  6 6236 ACaCAatTATGcATGGTtGGaTGTGCcCCcCCtTTgGGcGAgCATTGGGGTAAaGGTAaACAgTGTaC
         || ||  |||| ||||| || ||||| || || || || || ||||||||||| |||| ||| ||| |
 11 6221 ACcCAGcTATGTATGGTgGGcTGTGCtCCaCCgTTAGGtGAACATTGGGGTAAgGGTAcACAaTGTtC
         || ||| ||||| |  | || |||   || ||   ||| |||||||||||||| |||      ||| |
 33 6056 ACACAGTTATGTTTAcTTGGaTGTAAgCCtCCaAcAGGGGAACATTGGGGTAAAGGTgttgCtTGTAC
         ||||| || |||||| |||| || || || || | ||||||||| ||||| |||||     | |||||
 16 6096 ACACAATTGTGTTTAaTTGGTTGCAAACCACCTATAGGGGAACAcTGGGGcAAAGGatccCCaTGTAC
         |||||| |||||||| ||||||||||||||||||| || || || ||||| |||||    || ||||
 31 6015 ACACAAcTGTGTTTAcTTGGTTGCAAACCACCTATTGGaGAgCAtTGGGGTAAAGGtAgTCCTTGTAg
         |||||  | ||| |  | || ||    ||  ||||||| || || |||| |||||| | | ||||||
 18 6072 ACACAgtTaTGTaTttTgGGcTGtgccCCtgCTATTGGgGAaCAcTGGGcTAAAGGcAcTgCTTGTAa

con      ACaCA-tTaTGt-Ta-TtGG-TGt---CCacCtataGGgGAaCAtTGGGgtAAaGGtactcctTGTac
     O27-ACACAGTTATGTTTACTTGGATGTAAGCCTCCAACAGGGGAACATTGGGGTAAAGGTGTTGCTTGTAC-O27

6 6304 tAATACacCTGTACAggcTGGTGACTGCCCgCCcTTaGAACTTATTACCAGTGTTATACAGGATGGcG
11 6289 aAATACctCTGTACAaaaTGGTGACTGCCCcCCgTTgGAACTTATTACCAGTGTTATACAGGATGGgG
33 6124 TAaTGCAGcACcTgCcaaTGATTGTCCACCtTTAGAACTTATAAAtACTaTTATTgAGGATGGTG
16 6164 cAATgtTGCAGTAAatCCaGGTGATTGTCCACCATTAGAgTTAATAAAcACAGTTATTCAGGATGGTG
31 6083 tAAcaaTGCTaTtACcCCtGGTGATTGTCCtCCATTAGAATTAAAAAAttCAGTTATaCAAGATGGgG
18 6140 atcgcgTcCTtTatCaCagGGcGATTGcCCcCCtTTAGAAcTtAAAAAcaCAGTTtTggAAGATGGtG
con -aata-tgCtgtac--cctggtGAtTG-CC-CC-TTaGAacTtAtaAacac-gTTaTacAgGATGGtG
JJ36-GATGGTG
O27- TAATGCAGCACCTGCCAATGATTGTCCACCTTTAGAACTTATAAATACTATTATTGAGGATGGTG-O27

6 6372 AtATGGTTGAcACAGGCTTTGGTGCTATGAATTTTGCtGAtTTgCAgACCAATAAATCaGATGTTCCt
11 6357 AcATGGTTGAtACAGGCTTTGGTGCTATGAATTTTGCAGAcTTaCAaACCAATAAATCgGATGTTCCc
33 6189 ATATGGTgGAcACAGGaTTTGGTtgcATGGATTTTAaAACATTgCAGGCTAATAAAAGTGATGTTCCt
16 6232 ATATGGTTcATACtGGCTTTGGTGCTATGGAcTTTACTACATTACAGGCTAAcAAAAGTGAaGTTCCa
31 6151 ATATGGTTGATACaGGCTTTGGaGCTATGGAtTTTACTgCtTTACAAGAcACTAAAAGTaAtGTTCCt
18 6208 ATATGGTaGATACtGGaTaTGGtGCcATGGAcTTTAgTaCaTTgCAAGAtACTAAAtGTgAgGTaCCa
con AtATGGTtgAtACaGGcTtTGGtgctATGgAtTTact-caTT-CA-gc-AatAAA-gtgAtGTtCCt
ATATGGTTGATACAGGCTTTGGTGCTATGGA-36 37-CATTNCANGCNAATAAANGTGATGTTCCT
O27-ATATGGTGGACACAGGATTTGGTTGCATGGATTTTAAAACATTGCAGGCTAATAAAAGTGATGTTCCT-O27

6 6440 aTTGAcATaTGTGGcACTacaTGtAAATATCCaGATTATTTaCAAATGGCTGCAGACCCaTATGGTGA
11 6425 cTTGATATTTGTGGaACTgtcTGCAAATATCCtGATTATTTgCAAATGGCTGCAGACCCTTATGGTGA
33 6257 aTTGATATTTGTGGcAgTAcaTGCAAATATCCAGATTATTTaAAAATGaCTagtGAgCCTTATGGTGA
16 6300 cTGGATATTTGTAcaTCTATTTGCAAATATCCAGATTATaTTAAAATGGTgtCaGAaCCATATGGCGA
31 6219 TTGGAcATTTGTAAtTCTATTTGTAAATATCCAGATTATcTTAAAATGGTTGCtGAgCCATATGGCGA
18 6276 TTGGAtATTTGTcAgTCTATTTGTAAATATCCtGATTATtTacAAATGtcTGCaGAtCCtTATGGgGA
con -T-GAtATTtTGTgg--cTatttG-AAATATCCaGATTATtTa-AAATGgctgcaGA-CC-TATGGtGA
NTNGATATTTGT-JJ37 JJ38-AAATATCCAGATTATTTANAAATGG-JJ38
O27-ATTGATATTTGTGGCAGTACATGCAAATATCCAGATTATTTAAAAATGACTAGTGAGCCTTATGGTGA-O27

6 6508 TAGaTTaTTTTTTTtTcTaCGgAAGGAACAAATGTTTGCcAGACAtTTTTTAAcAGGGCtGGcgagG

11 6493 TAGgTTgTTTTTTaTtTgCGAAAGGAACAAATGTTTGCtAGACACTTTTTTAATAGGGCcGGTACtG

33 6325 TAGtTTATTTTTcTtTcTtCGAcGtGAACAAATGTTTGTaAGACACTTTTTTAATAGGGCTGGTACat

16 6368 cAGcTTATTTTTTTATTTACGAAGGGAACAAATGTTTGTtAGACATTTaTTTAATAGGGCTGGTACtG

31 6287 TACaTTATTTTTTTATTTACGtAGGGAACAAATGTTTGTaAGGCATTTTTTTAATAGAtCAGGcACgG

18 6344 TtCcaTgTTTTTTTgcTTACGgcGtGAgCAgcTtTTTGctAGGCATTTTTggAATAGAgCAGGtACta

con tag-tTaTTTTTtTattTaCGaaggGAaCAaaTgTTTG-tAGaCAtTTtTttAAtAGggCtGGtactg
WO 86/05816-GAGG
O27-TAGTTTATTTTTCTTTCTTCGACGTGAACAAATGTTTGTAAGACACTTTTTTAATAGGGCTGGTACAT-O27

6 6576 TGGGGGAACCTGTGCCTGATacaCTtaTaaTtAAgGGtaGTggaAAtcGcaCgTCTGTAGggAGTAGT

11 6561 TGGGGGAACCTGTGCCTGATGACCTGTTggTaAAAGGggGTaatAAcAGatCaTCTGTAGctAGTAGT

33 6393 TaGGaGAggCTGTtCCcGATGACCTGTACATTAAAGGtTCaGGaACTACTGCcTCTaTtcaaAGcAGT

16 6436 TTGGTGAAaatGTaCCaGAcGAtTTATACATTAAAGGCTCtGGgTCTACTGCaAaTTTAGCcAGttca

31 6355 TTGGTGAAtCgGTcCCTactGACTTATATATTAAAGGCTCcGGTTCaACaGCTACTTTAGCtAaCaGT

18 6412 TgGGTGAcaCtGTgCCTcaatcCTTATATATTAAAGGCaCaGGTatgcCtGCTtCacctGgcAgCtGT

con TgGGtGAa-ctGTgCCtgatgac-Tata-aTtAAaGGctctggtactactgC-tct-tagc-Ag-agt
TGGGGGAACCTGTGCCTGATACACTTATAATTAAGGGTAGTGGAAATCGCACGTCTGTA-WO86/05816
LCR2A-ACCTGTTGGTAAAAGGGGGTAATAA-LCR2A
LCR2A'-GGACAACCATTTTCCCCCATTATT-LCR2A'
LCR2B-CAGATCATCTGTAGCTAGTAGT
LCR2B'-GTCTAGTAGACATCGATCATCA
LCR3A-ATTTATACATTAAAGGCTCTGGGTC-LCR3A
LCR3A'-AAATATGTAATTTCCGAGACCCAG-LCR3A'
LCR3B-TACTGCAAATTTAGCCAGTTCA
LCR3B'-ATGACGTTTAAATCGGTCAAGT
LCR4A-CCTTATATATTAAAGGCACAGGTAT-LCR4A
LCR4A'-GAATATATAATTTCCGTGTCCATA-LCR4A'
LCR4B-GCCTGCTTCACCTGGCAGCTGT
LCR4B'-CGGACGAAGTGGACCGTCGACA
O27-TAGGAGAGGCTGTTCCCGATGACCTGTACATTAAAGGTTCAGGAACTACTGCCTCTATTCAAAGCAGT-O27

6 6644 ATaTATGTtaAcACcCgAGcGGCTCtTTGGTGTCcTCtGAGGCaCAATTgTTTAATAAgCCATATTG
          || |||||  | || || || ||||| |||||||| || ||||| ||||| |||||||| ||||||||
11 6629 ATTTATGTaCAtACaCCTAGTGGCTCATTGGTGTCTTCaGAGGCTCAATTATTTAATAAaCCATATTG
          || | | |  || |||||||| ||| ||||  ||||| ||  |||| ||||||||||| ||||||||
33 6461 gcTTtTTTTCCcACtCCTAGTGGaTCAATGGTTACTTCcGAatCTCAgTTATTTAATAAgCCATATTG
          || |||||| || |||||||| || |||||||| || ||  | ||  |||| ||||| || |||||
16 6504 AaTTATTTTCCTACACCTAGTGGtTCtATGGTTACcTCtGATGCcCAAATATTcAATAAACCtTATTG
          |  || ||||||||||||| || || |||||||| || ||||| ||||| || |||||||| |||||
31 6423 AcaTAcTTTCCTACACCTAGcGGCTCcATGGTTACtTCaGATGCaCAAATtTTTAATAAACCATATTG
          || | |||  | || || ||||| || ||||| || ||  | ||  | ||||||||||||||||
18 6480 gtgTAtTcTCCctCtCCaAGtGGCTCtATtGTTACcTCtGActCcCAgtTgTTTAATAAACCATATTG
con     attTatttcc-aCaCCtAGtGGcTCtaTgGTtaC-TCtGA-gC-CAatTaTTtAATAAaCCaTATTG
        AT-LCR2B                    JJ39-GTTACNTCTGANGCNCAATTATTTAATAAACCATATTG
        TAA-LCR2B'
        AA-LCR3B
        TTA-LCR3B'
        GT-LCR4B
        CAC-LCR4B'
    O27-GCTTTTTTTCCCACTCCTAGTGGATCAATGGTTACTTCCGAATCTCAGTTATTTAATAAGCCATATTG-O27

6 6712 GCTaCAAAAaGCcCAGGGACATAACAATGGTATTTGtTGGGtAAtCAacTGTTTGTTACTGTGGTAG
          ||| ||||| || |||||||||||||||||||||||| ||||| || ||  ||||||||||||||||||
11 6697 GCTtCAAAAgGCtCAGGGACATAACAATGGTATTTGcTGGGaAAcCActTGTTTGTTACTGTGGTAG
          ||| |||   || || || ||||| ||||||||||| ||||| || ||  | |||||||||||||||
33 6529 GCTACAACGtGCACAaGGtCATAATAATGGTATTTGTTGGGcAAtCAggTATTTGTTACTGTGGTAG
          | ||||||| ||||| || || |||||||| |||||||||| || ||  |||||||||||| || |
16 6572 GtTACAACGaGCACAGGGcCACAATAATGGcATTTGTTGGGtAAcCAacTATTTGTTACTGTtGTtG
          | | ||||| || ||||| ||||||||||| |||||||||| || ||  |||||||||||| || |
31 6491 GaTgCAACGtGCtCAGGGaCACAATAATGGTATTTGTTGGGcAATCAgTTATTTGTTACTGTGGTAG
          | | ||   || ||||| || || |||||| |||| |||   ||||| ||||||||||||||||||
18 6548 GtTaCAtaagGCaCAGGGtCAtAAcAATGGTgTTTGcTGGcatAATCAaTTATTTGTTACTGTGGTAG

con     GcTaCAa---GCaCAgGGaCAtAA-AATGGtaTTTGtTGGggtAAtCAatTaTTTGTTACTGTgGTaG
        GCTACAANNNGCACA-JJ39 J41-AATGGTATTTGTTGGGGTAATCAATTATTTGTTACTGTGGTAG
              C6-GCMCAGGGWCATAAYAATGG-C6                          C1-CTGTGGTAG
                                                                  C7-CTGTTGTTG
                                                                  C8-CTGTGGTAG
                                                                 C10-CAGTTGTAG
                                                                 C11-CTGTGGTTG
                                                                 C12-CTGTTGTGG
                                                                 C13-CTGTTGTAG
                                                                 C14-CTGTGGTAG
                                                                 C15-CTGTAGTGG
    O27-GCTACAACGTGCACAAGGTCATAATAATGGTATTTGTTGGGGCAATCAGGTATTTGTTACTGTGGTAG-O27

6 6780 ATACCACACGCAGTACcAAcATGACAtTATG      TGCATCcGTaaCTAcATCTtCcACATACACc
       |||||||||||||||| || |||||| ||||      |||||| ||  ||| |||| | ||||||||
11 6765 ATACCACACGCAGTACaAATATGACAcTATG      TGCATCtGTgtCTAaATCTgCTACATACACt
       ||||||| |||||||| ||||||||  ||||        ||   ||  |||       |||||| |
33 6597 ATACCACtCGCAGTACtAATATGACtTTATG      caCAcaAGTaACTAgtGAcAgTACATATAAA
       |||| || |||||||| |||||| | |||||        |   |   |||   || | ||||||||||
16 6640 ATACtACACGCAGTACaAATATGTCaTTATGTGCT    GCcATAtCtACTtcaGAaACTACATATAAA
       |||| ||||| ||||| ||||||||  | ||||||    || || |  | |     || ||||||| ||||
31 6559 ATACCACACGtAGTACCAATATGTCtgTtTGTGCT    GCAATtgCaAacagtGATACTACATtTAAA
       ||||||| | ||||||||| |  |  | ||||||     ||    |         |     || | |
18 6616 ATACCACtCccAGTACCAATtTaaCaaTaTGTGCTtctaCAcagtCtcctgtaccTgggcaATaTgAt
con     ATACcACaCgcAGTACcAAtaTgaCatTaTGtgct--tgCa---g-aacta-ag-tactacATataaa
        ATACC-JJ41                        C16-CATCCGTAACTACATCTTCCA-C16
        ATACCACACGCAGTAC-C1                 C17-TCTGTGTCTAAATCTGCTACA-C17
        ATACTACACGCAGTAC-C7               C20-CACACAAGTAACTAGTGACAG-C20
        ATACCACTCGCAGTAC-C8                 C23-CAGTCTCCTGTACCTGGG-C23
        ATACTACTCGCAGCAC-C10                 C31-TTGCAAACAGTGATACTACATT-C31
        ATACTACCCGTAGTAC-C11
        ATACTACCAGAAGCAC-C12
        ATACTACTAGAAGCAC-C13
        ATACCACACGTAGTAC-C14
        ACACTACCCGCAGTAC-C15
    O27-ATACCACTCGCAGTACTAATATGACTTTATG      CACACAAGTAACTAGTGACAGTACATATAAA-O27

6 6842 AATTCtGATTATAAaGAgTACATGCGtCATGTGGAaGAGTaTGATTTACAaTTTATTTTTCAATTaTG
       ||||| |||||||| || |||||||| |||||||| |||| ||||||||| |||||||||||||| ||
11 6827 AATTCAGATTATAAgGAATACATGCGcCATGTGGAgGAGTtTGATTTACAGTTTATTTTTCAATTgTG
       |||  | ||| ||| ||||| ||  | ||||| ||||| | |||| |||||||| |||||||| | ||
33 6659 AATgaAAATTTTAAaGAATAtATAaGACATGTtGAaGAATATGATcTACAGTTTgTTTTTCAACTaTG
       |||   || ||||| || ||  || ||||||  || ||||||||| |||||||| |||||||||| ||
16 6705 AATAcTAAcTTTAAgGAGTAccTAcGACATGGgGAGGAATATGATTTACAGTTTATTTTTCAACTgTG
       | || ||| ||||| |||||  || ||||||| ||||||| ||||||||| ||||| |||||  | ||
31 6624 AgTAgTAAtTTTAAaGAGTATtTAAGACATGGTGAGGAATtTGATTTACAaTTTATaTTTCAGTTaTG
         ||  || |||||  |||||   ||||||| |||||||| |||||| || ||||| |||||||| ||
18 6684 gcTAccAAaTTTAAgcAGTATagcAGACATGtTGAGGAATaTGATTTgCAgTTTATtTTTCAGTTgTG

con     aaTactaAtTtTAA-gAgTA-ata-GaCATGt-GAgGAaTaTGATtTaCAgTTTaTtTTTCAatT-TG
    O27-AATGAAAATTTTAAAGAATATATAAGACATGTTGAAGAATATGATCTACAGTTTGTTTTTCAACTATG-O27

6 6910 TAGCATTACATTgTCTGCtGAAGTaATGGCCTATATtCACACAATGAATCCcTCTGTTTTGGAaGACT
       |||||||||||| ||||| ||||| ||||||||||| |||||||||||||| ||||||||||| ||||
11 6895 TAGCATTACATTATCTGCAGAAGTcATGGCCTATATaCACACAATGAATCCtTCTGTTTTGGAgGACT
        |   |||| ||| |||||||||| ||| | ||||| ||  | ||||||||   | |||| || || |
33 6727 CAAAgTTACCTTAACTGCAGAAGTTATGACATATATtCATgCTATGAATCCagaTATTTTaGAaGAtT
       |||| | |||||||||||||| ||||||||||| || ||| |||||||| |   |||||| || || |
16 6773 CAAAATAACCTTAACTGCAGACGTTATGACATAcATaCATtCTATGAATtCcaCTATTTTGGAgGAcT
       ||||||||| ||| |||||||| | |||||||| || ||   |||||||||  |||||||||| || |
31 6692 CAAAATAACaTTAtCTGCAGACaTaATGACATATATTCAcAGTATGAATcCtgCTATTTTGGAaGATT
        |  || || ||| |||||||  | ||| | |||||||| |||||||||     |||||| || ||||
18 6752 tActATtACtTTAaCTGCAGAtgTtATGtCcTATATTCAtAGTATGAATagcagTATTTTaGAgGATT

con     -AaaaTtACaTTa-CTGCaGAagTtATGaC-TAtATtCA-actATGAATccc-cTaTTTTgGA-GA-T
    O27-CAAAGTTACCTTAACTGCAG-O27

6 6978 GGAACTTTGGgTTATCGCCTCCcCCAAATGGTACAtTaGAaGATACcTATAGgTATGTgCAGTCACAG
||||||||| ||||||||||| ||||||||||| | || ||||| ||||| ||||| |||||||||
11 6963 GGAACTTTGGTTTATCGCCTCCaCCAAATGGTACAcTgGAGGATACtTATAGaTATGTaCAGTCACAG
|| | ||||||||| | ||||| ||| || || | ||||||| ||||| | ||| || |||
33 6795 GGcAaTTTGGTTTAaCACCTCCtCCAtcTGcTAgttTAcAGGATACcTATAGGTTTGTtaccTCtCAG
|| | |||||| || |||||| ||| | | || | ||||| ||||||||||| | |||
16 6841 GGAATTTTGGTcTAcaACCTCCcCCAggAGGcACacTAGAaGATACtTATAGGTTTGT   aaCcCAG
|||||||||| | | || || ||| | | || ||||| ||||||||||| | |||
31 6760 GGAATTTTGGatTgaCCaCaCCtCCctCAGGTtCTTTGGAGGATACcTATAGGTTTGTcaccTCaCAG
|||| ||||| | || | || || | | ||||| |||||| ||| | ||||| ||
18 6820 GGAAcTTTGGtgTtcCCcCcCCcCCaaCtacTagTTTGGtGGATACaTATcGtTTTGTacaaTCtgtt
con GGaAcTTTGGttTa-c-cCtCCcCCaactggtac-tT-gagGATACcTATaGgTtTGTaca-tCacag
C21-TTGT   AACCCAG
C34-TTTGT   AACCCAG
C35-GTTTGT   AACCCAG

6 7046 GCCATTACCTGTCAaAAgCCCACtCCTGAAAAgGAAAAgCcaGA    TCCCTATAAGaAccTtAGTT
|||||||||||||| || ||||| |||||||| ||||| | ||    |||||||||| | | ||||
11 7031 GCCATTACCTGTCAgAAACCCACACCTGAAAAaGAAAAaCAgGA    TCCCTATAAGgAtaTgAGTT
|| ||||| ||||| ||| | |||| ||| ||||| | ||    |||| | | |
33 6863 GCtATTACgTGTCAAAAAaCagtACCTCCAAAgGAAAAgGAAGA    CCCCTTAggtAAATAtACaT
|| ||| | ||||||||| ||||||| || |||||    ||||||| ||||| || |
16 6906 GCaATTgCtTGTCAAAAAcaTaCACCTCCAgcaCCtAAaGAAGATgatCCCCTTA AAAAATAcACtT
|| ||| | ||||||||| | | || | | || || ||||||    || ||| || | || ~|
31 6828 GCcATTACaTGTCAAAAAacTGCcCCcCaAaagCCcAAgGAAGAT   CCaTTTA AAgAtTAtgtaT
|| ||||| |||||||| ||| | | || | |||    || | | | | | |
18 6888 GCtATTACcTGTCAAAAggaTGCtgCaCcggctgaaAAtaAgGAT   CCcTaT gAtaAgTtaaagT

con GCcATTaCcTGTCAaAAacct-cacCtc-aaaggaaAAggaaGAt---cCccttaaa-aA-t-ta-tT
GCAATTGCT-C21 C18-CATACACCTCCAGCACCTAA-C18    JJ46-T
C19-GGATGCTGCACCGGCTGA-C19
C22-AAAAACAGTACCTCCAAAGGA-C22
C27-TTTTTGTCATGGAGGTTTCCT-C27
C24-CACACCTGAAAAAGAAAAACAG-C24
C28-GTGTGGACTTTTTCTTTTTGTC-C28
C25-CTCCTGAAAAGGAAAAGCCA-C25
C26-GAGGACTTTTCCTTTTCGGT-C26
C29-CCAAAAGCCCAAGGAAGAT   C-C29
C30-CAAAAGCCCAAGGAAGAT   C-C30
C32-CAGAAACCCACACCTGAAAAAGA-C32
C33-AGAAACCCACACCTGAAAAAGAA-C33
GCAATTGCT-C34    O23-GGA   TCCCTATAAGGATATGAGTT
GCAATTGCT-C35    O15-GGAT   CCCTAT GATAAGTTAAAGT

6 7110 TTTGGGAGGTTAAtTTAAAAGAAAAGTTTTCtAGTGAATTgGATCAGTaTCCttTgGGACGcAAGTTT
          ||||||||||||| |||||||||||||||||| |||||||| ||||||| |||  | ||||| ||||||
11 7095 TTTGGGAGGTTAAcTTAAAAGAAAAGTTTTCAAGTGAATTAGATCAGTTTCCccTtGGACGtAAGTTT
          ||||||| ||  | ||||| ||||| ||||||    || ||||||||||||||  | ||||| ||||||
33 6927 TTTGGGAAGTggATTTAAAGGAAAAaTTTTCAGCAGAtTTAGATCAGTTTCCTTTgGGACGCAAGTTT
          ||||||||||  ||||||||||||| ||||| |||||  |||||||||||||||| |||||||| |||
16 6973 TTTGGGAAGTaAATTTAAAGGAAAAGTTTTCTGCAGAccTAGATCAGTTTCCTTTaGGACGCAAATTT
          ||||||| || |||||||| ||||||||||||||||    ||||||||||||| . | || |||||||||
31 6892 TTTGGGAgGTtAATTTAAAaGAAAAGTTTTCTGCAGAtTTAGATCAGTTTCCaCTgGGtCGCAAATTT
          ||||| | ||  ||||||| |||||||||||  ||| |||||||| | ||| || || || ||||||
18 6952 TTTGGaAtGTggATTTAAAgGAAAAGTTTTCTttAGAcTTAGATCAaTaTCCcCTtGGaCGtAAATTT

con      TTTGGgAgGTtaAtTTAAA-GAAAAgTTTTCtgcaGA-tTaGATCAgTtTCCt-TgGGaCGcAA-TTT
         TTTGGGAGGTTAATTTAAANGAAAAGTTTTCTGCAGANTTAGATCA-JJ46
                                          C2-GATCAGTTTCCYYTKGGACG-C2
                                          C3-GATCAGTWTCCYYTKGGACG-C3
                                          C7-CTAGTCAWAGGRRAMCCTGC-C7
     O15-TTTGGAATGTGGATTTAAAGGAAAAGTTTTCTTTAGACTTAGATCAATATCCCCTTGGACGTAAATTT-O15
     O23-TTTGGGAGGTTAACTTAAAAGAAAAGTTTTCAAGTGAATTAGATCAGTTTCCCCTTGGACGTAAGTTT-O23


 6 7178 TT    gTT                                      aCAAAGTGGATATaGgGGACGGtCcT
          ||     ||                                       ||||||||||| | |||||| | |
11 7163 TTA    TT                                      gCAAAGTGGATATcGAGGACGGaCgT
          |||    ||                                        |    |      |
33 6995 TTA    TTAcagGcAggtcttaaagcAaaAcctaaacttaaACGtgcAGcccccaCAtCcaCCcgCA
          |||      |  | |        | |         | |  |        |  |  || ||
16 7041 TTA cTACAAGCAGGATtgAaGGCcaaACCAAAATTTAcAttAGGaAAACGaaaagCTaCACCCacCA
          |||  |||| ||||||| | |||    || ||||||| |  ||| |||||      | |||||
31 6960 TTA tTACAGGCAGGATatAGGGCacgtCCtAAATTTAaAgCAGGtAAACG   TagTgCACCC   t
          ||   | ||||| ||||   |       || |   |     |  | |||||    |
18 7020 TT ggTtCAGGCtGGATtgcGtcgcaagCCcAccaTaggccCtcGcAAACG   T         tctg

con      TTa--ta--agcaggattgagggcaaaaccaaaaataa-a-cacgaaaa-gatatag-gcaccc-cct
     O15-TT GGTTCAGGCTGGATTGCGTCGCAAGCCCACCATAGGCCCTCGCAAACG   T         TCTG-O15
     O23-TTA    TT                                      GCAAAGTGGATATCGAGGACGGACGT-O23


 6 7209 CTatTCGTACAGGTgTtAAGCGCCCtGCTGTtTCcAAagCCTCTgCtGCCCCtAAACGtAAgCGcgCC
          ||  |||||||||| | |||||||| ||||| || ||  ||||| | ||||| ||||| || ||  ||
11 7194 CTgCTCGTACAGGTaTaAAGCGCCCaGCTGTgTCtAAgcCCTCTaCAGCCCCcAAACGaAAaCGTaCC
          |  |   | ||     ||                |   |        |       |       |
33 7059 CaTCgTCTgCAaaacgcAAaaaggttaaaaAATAAcAcTttGtgtaAttgtgtTAtgtTGTtgtTttg
          | || ||| |      |                || ||   |   |       |   |||
16 7108 CcTCATCTACCtCTACAACtGCtAAACG cAAAAAACgTAAGctGTAA  GtaTTGTATGTA
          |  |||||||| ||||| | || |||||  ||||||| |||    ||||   |   ||||||||
31 7021 CagCATCTACCACTACAcCaGCaAAACGtaAAAAAAC TAAAaaGTAAtgGatgTGTATGTAAtaCaT
          |  ||||| |||||||                    | ||||                 ||  |  | |
18 7075 CtcCATCTgCCACTAC                gtcttC TAAA                ccTGccAagCgT

con      Ct-catcTaC-actacaaa---c--a--t-aat-aa-gtaa-ctg-a-cc-ct-a-c-tgta--tcc-
     O15-CTCCATCTGCCACTAC                GTCTTC TAAA                CCTGCCAAGCGT-O15
     O23-CTGCTCGTACAGGTATAAAGCGCCCAGCTGTGTCTAAGCCCTCTACAGCCCCCAAACGAAAACGTACC-O23

6 7277 AAAACtAAAAgGTAATATATGTGT aTaTGTacTGTT
11 7262 AAAACcAAAAaGTAATATATGTGTgtcagTGTGTTGTGTT
33 7127 TtcTGtcTAtGTactTtgtgTTGT TGTGTTGTGTTgtTGT
16 7167 TgtTGaaTtaGTGT TGtTTGT TGTGT ATATGT
31 7088 GTGTctgTatGTGTAtGTGCTTGTgctgtatTGT ATATGTGTGTTtgtgtgtTATATA tg
18 7113 GTG cGTGTAcGTGC caGgaagtaATATGTGTGTGT gtatataTATATAcat

con -t-tctataagtgtat-tgtttgtg----tgtGtagtgt-tatgtgtgtgt--------tatata---
O15-GTG CGTGTACGTGC CAGGAAGTAATATGTGTGTGT GTATATATATATACAT-O15
O23-AAAACCAAAAAGTAATATATGTGTGTCAGTGTGTTGTGTT-(O23)

6 7313 AT
11 7302 ATtTaTATG T TGTTGTA gTGTGT
33 7167 TTGT TtTtTgTGTATG TGttacaaTgtATgTTATGTTGTATGTtacTGTGTTTG
16 7199 TTGTATGTgctTGTATG TG CTTGTAaATATTAaGTTGTATGT GTGTTTG
31 7150 gTATaTGTATGTTTaTGTATG CgTGTGT aCTTGTATATAT GtaTaGTATGT TATGTgTG
18 7164 cTATtgtTgTGTTT GTATGtCcTGTGTttgtgtTTGT TgTAT G aTtgcattgTATG G

con -tatttgtatgtttttgtatg-c-tgtgt-tgt-cttgtatatattatgttgtatgtt-gtgtgtttg
O15-CTATTGTTGTGTTT GTATGTCCTGTGTTTGTGTTTGT TGTAT G ATTGCATTGTATG G-O15
O23- ATTTATATG T TGTTGTA GTGTGT(-O23)

6 7315 aTATATGT
11 7325 aTATGT TtCTTGT atTGTG TATATGT
33 7221 T ttTATGTgTaCTTGTttGTGTGcATGTTcTATGTacttgt
16 7248 TATGTATG gtaTAATAAA cacGTGTGTATGT
31 7209 TATGTATGctatgtaTGTTAATAAAtatgtgtatacctgtgtgtgtTGTGTATGTTGTcctTataTAc
18 7221 TATGTATG gtTGTT gtTGTATGTTGTatgTtacTAt

con tatgtatg-------tgttaataaa----ttatgt-ttcttgtt-gtgtgtatgtt-tatgta--tat
O15-TATGTATG GTTGTT GTTGTATGTTGTATGTTACTAT-O15
O23- ATATGT TTCTTGT ATTGTG TATATGT(-O23)

6 7323    GT                                                    gTATGTAcTGT
11 7351   GTataTGTTTGTGTATATGT                    GTAT            gtTATGTA TGT
33 7262  cagtTtccTGTTTGTGTATATGTtaataaaacattgTgTGTATtTgtTaaActATttgTATGTA TGT
16 7279              GTtTtTaAaTgcTTgtgtAACTATTGT gTcATgcAacATaAaTAaacttatT
31 7277  AccctaTtagtaacatacTaTtAcTAtTTataAACTATTGTtccTActTgtTcctAcTtgttCCTgc
18 7257  AtttgtTggtatgtggcaTtaaAtaAaaTatgttttgtggtTctgTgtgTtaTgtggtTgcgcCCTag

con       a---tat-tgtttgtgtatat-ataatataagaaactatgtttttatgtaatatt--Tatgtactgt
  O15-ATTTGTTGGTATGTGGCATTAAATAAAATATGTTTTGTGGTTCTGTGTGTTATGTGGTTGCGCCCTAG-O15
  O23-   GTATATGTTTGTGTATATGT                    GTAT            GTTATGTA TGT-O23

6 7336   TATGT   aTATGT  GTgTGTGTGTTctGTGTGTaatgtaAgtTATTTGTGtAATGTGTATGTgTGTT
11 7386  TATGTtgtTATGTatGTtTGTGTGTTtaGTGTGT     gtATaTATTTGTGGAATGTGTATGTATGTT
33 7329  TATGT          AtatgggtgtaccTataTGaGTAagGagTTgTATTgcTtGccctacCcTGCATTgc
16 7331  gtTTCaacAcctACtaaTtgTgtTgTggtTaTtcAtTGTATaTaAactaTatTtGctACATcCtgTtt
31 7345  TccTCccaAtagtCATgTacTTaTtTctgccTatAaTTTAggTgTcacgccaTaGTaAaAgTtgtaca
18 7325  TgagtaacAactgtATtTgtgTtTgTggtatgggtgTTgcttgtTgggctatataTtgtccTgtattt

con       tatgtaa-aa-gt-atttgt-tttT-tgtgtgtaatgtattttatttgt-taa-ttgtatgt-tttt
  O15-TGAGTAACAACTGTATTTGTGTTTGTGGTATGGGTGTTGCTTGTTGGGCTATATATTGTCCTGTATTT-O15
  O23-TATGTTGTTATGTATGTTTGTGTGTTTAGTGTGT     GTATATATTTGTGGAATGTGTATGTATGTT-O23

6 7400   TaTGTGCAATAAACAATTAcctcTtgtTacacCCTGT  gACtCAGTGgctgttgcacgcGTTtTGgT
11 7450  TtTGTGCAATAAACAATTA    TTatgTgtgtCCTGTTACACcCAGTG          actaaGTTgTGtT
33 7390  aaTGTaCcTAccTttATTtcccTaTAtTtgtAGtaCCTACATGTttaGTattgCtttacCtTTTGaca
16 7399  ttgtTTtaTATaTactaTAtTtTgTAgcgcCAGgcCCatTTTGTaGCtTCaAcCgaAttCggTTGcat
31 7413  CccGgTccgtTtTTtgcaACTaaAgctacTCCATTTTgaTTTtatGCagCCAtTTTAaaTcccTAACC
18 7393  CaaGtTataaaacTgcacACcttAcagcaTCCATTTTatccTacaatcctCcaTTTtgcTgtgcAACC

con       tatgttcaa-aatt-attaccttata-t-tcc-tt-t-acat-cagtg-c-atttta--cgttt-act
  O15-CAAGTTATAAAACTGCACACCTTACAGCATCCATTTTATCCTACAATCCTCCATTTTGCTGTGCAACC-O15
  O23-TTTGTGCAATAAACAATTA    TTATGTGTGTCCTGTTACACCCAGTG          ACTAAGTTGTGTT-O23
                                                          O24-GAATTCGGTTGCAT

6 7466 TTGCACGCGCCtTacacacataagTaATATacaTgcAcaATATATATaTtTttgtTtaaaATACTAT
11 7508 TTGCACGCGCCgTtTgtgttgccTTCATAT   TatAtTATATATATtTgTaataTacCTATACTATg
33 7458 TacTAgTGtCCaTATtgtacaaTTTCcTccattTTgTATGcCTAaccgTtTtcggTtACTTgGCAtac
16 7467 GcTTTtTGgCaCAAaatTgTgttTTTtaAaTAgTTCTATGtCagcaacTaTGgTtTaAacTTGTAcGT
31 7481 GtTTTCGGTTGCAttgTtTaaacaTgctAgTAcaaCTATGctgatgcagtaGTTcTGcggTTtTTgGT
18 7461 GaTTTCGGTTGC   ctttggcTTaTGtctgTggTttT

con -ttt-cgg-ccctat-t-ta-a-ttc-tataa-t-ctatgt-tatat-ttt-tt-T-actttgct-tt
O15-GATTTCGGTTGC   CTTTGGCTTATGTCTGTGGTTTT-O15
O23-TTGCACGCGCCGTTTGTGTTGCCTTCATAT   TATATTATATATATTTGTAATATACCTATACTATG-O23
O24-GCTTTTTGGCACAAAATGTGTTTTTTAAATAGTTCTATGTCAGCAACTATGGTTTAAACTTGTACGT-O24

6 7533 aCttttatAtTTGCAACCGTTTTCGGTTGCCCTTAgCATACACTTtCCaCcAATTTGTTAcAAC
11 7573 tTACCCccccccCAcTTGCAACCGTTTTCGGTTGCCCTTA CATACACTTaCCTCaAATTTGTTAtAAC
33 7526 aTACCCtaTgaCAtTGGCAGaacAgTTaaTccTTTtCTttCCTGCACTGtgtTtgTcTgTACTtgctg
16 7535 TTCCTG cTtgCcaTGcgtGccaAaTcccTgtTTTcCTgaCCTGCACTG   cTTgccaACcaTtcc
31 7549 TTCCTG   aaTAcTagTTTttGCcaacaTTCTggcTtgTagt
18 7496 cTgCacaatacagtacgctggcactattgcaaactttTAaTctTTTggGCactgcTcCTacaTatTttg

con tt-c-ct-tt-catt-gcagcc--tttcg-tt-ctcttatc-T-cact--c-tcttct-tattata-c
O15-CTGCACAATACAGTACGCTGGCACTATTGCAAACTTTAATCTTTTGGGCACTGCTCCTACATATTTTG-O15
O23-TTACCCCCCCCCACTTGCAACCGTTTTCGGTTGCCCTTA CATACACTTACCTCAAATTTGTTATAAC-O23
O24-TTCCTG CTTGCCATGCGTGCCAAATCCCTGTTTTCCTGACCTGCACTG   CTTGCCAACCATTCC-O24

6 7597 GTGTTTccTctTAATCCtATATattTTGTG   CcAGGTACAcATTGCCCTGCCAAGTtgCTTGCCAA
11 7640 GTGTTTtgTACTAATCCcATAT  gTTGTGtgcCAAGGTACAtATTGCCCTGCCAAGTatCTTGCCAA
33 7594 caTTggcaTACatAcCCtATgacatTgGCagaaCAgTtAATcctTTtCTTTcCTgcacTgtgTTtgtc
16 7598 aTTgTtttTtACACtgCacTatgtgcaACtActgAaTCAcTaTgTaCATTgtgTCataTAAaaTaaaT
31 7589 tTCcTgccTaACACacCTTgccaaCATATAAtccAgTCCaacTtTGCAATTAtaCtATgAAtCatgtT
18 7564 aaCaattggcgCgCctCTTtggcgCATATAA   ggCgcaccTGgtATTA gtcATtttcCtgtcc

con -t-ttt--ta-ca-tcCtatat---tt-taa-ccaa-g-aca--Ttgc-tt-c--aatt--ttt--a-
O15-AACAATTGGCGCGCCTCTTTGGCGCATATAA   GGCGCACCTGGTATTA GTCATTTTCCTGTCC-O15
O23-GTGTTTTGTACTAATCCCATAT  G-O23
O24-ATTGTTTTTTACACTGCACTATGTGCAACTACTGAATCACTATGTACATTGTGTCATATAAAATAAAT-O24

6 7662 gtgcatcatatcctgccaaCcACACACCTGGCgcCAGGGtGCGGTATTGC cTtactcATAA
11 7706 CAACACACCTGGC CAGGGcGCGGTATTGCaTgAcTaaTgTAcaATAA
33 7662 tgtacTtgctgcAttgacTCAtatataCatGCAGtgcaATtgcaaAaTaCTTaATTgtacTAatAgtT
16 7666 cacTaTgcgcCAACgcctTacatACcgCtgtTAGgcacATatTtTTggcTTgTtTTAactAACcTAAT
31 7657 tGtTtaaaTACAACtgtagttcaACtATgtgTcatgcAcaTATATTatatTaTCCTAcAcAcCTTAAA
18 7626 aGgTgcgcTACAAC aATtgcTtgcatAacTATAT ccactcCCTA AgtaaTaAAA

con tg-tatg-tacaacgccatc-a-acaactgg-agca-aatt-tata-t-cttt-cta-a--actaaaa
BE31-XXAGGCACAXAXXXX-BE31 hpv16+18+33
O15-AGGTGCGCTACAAC AATTGCTTGCATAACTATAT CCACTCCCTA AGTAATAAAA-O15
O24-CACTATGCGCCAACGCCTTACATACCGCTGTTAGGCACATATTTTGGCTTGTTTAACTAACCTAAT-O24

6 7723 ACCTGTC TTTGTgttAtAcTtTTaTGcAcTGtAGCCAActcTTAAAAGCATTTTTGGCTTgTAGCa
11 7753 ACCTGTCGgTTTGT ACAaTgTTgTGgATTGCAGCCAAAggTTAAAAGCATTTTTGGCTTcTAGCt
33 7730 TaCAcATGcTTTtaggcACATAtTTTTactTTaCtttCAAAccTTAAgtGCAGTTTTGGCTT aCa
16 7734 TgCATATTtGGCAtAaggTTTAaacTTcTAaggCcAaCtAAatgTcAccctAGTTCaTaCaTgaActg
31 7725 CTGCTTTTAGGCACATATTTT GTagaTTATctaTAtCctTgATTGCAgtgcTGGCTTttgcacAtgt
18 7680 CTGCTTTTAGGCACATATTTTaGTttgTTtTtacTtaagcTaATTGCAtactTGGCTT

con --c-tttta---atataat-tagtttt-tattgct--caaa--tTaaa-gcattt-t-gcttgtagc-
BE31-XXAGGCACAXAXXXX-BE31 hpv16+18+33
BE31-XXAGGCACAXAXXXX-BE31 hpv16+18+33
O15-CTGCTTTTAGGCACATATTTTAGTTTGTTTTTACTTAAGCTAATTGCATACTTGGCTT-(O15)
O24-TGCATATTTGGCATAAGGTTTAAACTTCTAAGGCCAACTAAATGTCACCCTAGTTCATACATGAACTG-O24

6 7789 GcACATTTTTtTgCtCTTAcTgTtTggTatACAATAaCataAAAATGAGTAACCTAAGGTCACACACC
11 7818 GAACATTTTTGTACcCTTAgTaTaTtaTgcACAATAcCcacAAAATGAGTAACCTAAGGTCACACACC
33 7795 cAAttgcTTTGTAtgCcaAactaTgccTTGTAAAAgtgagtcActacctgttTaTtAccaGGTGTGga
16 7802 TgtAAagGTTAgtcaTacATtgTTCATTTGTAAAA cTgcAcatgGGTGTGtg
31 7792 TtaAActGccAaggTTgtgTcaTgCATTaTaAATAagttgTatgttactcaTATAATtaATtgCatAt
18 7738 gtacaactacTTtcaTgtccaAcatTctgTctacccTtaacatgaacTATAAT ATgaCtaAg

con -aa-attttt-tact-ttatt-tt-a-tttaaaaaac-gtaaa-tg--tat--taagga-g--ta--
O15- GTACAACTACTTTCATGTCCAACATTCTGTCTACCCTTAACATGAACTATAAT ATGACTAAG-O15
O24-TGTAAAGGTTAGTCATACATTGTTCATTTGTAAAA CTGCACATGGGTGTGTG-O24

6 7857 TGCgACCGGTTTCGGTTAtCCACACCCTACATATTTCCTTCTTATA
11 7886 TGCAACCGGTTTCGGTTAcCCACACCCTACATATTTCCTTCTTATA
33 7863 CtAACCG TTTTaGGTcAtaTTggtCATTTA tAaTCtTTTATATAATA
16 7854 CaAACCGATTTT gGGTTACACATTTACAAGCAAcTTATATAATAATACTaa
31 7860 agGTattAcaccgtTTTcGGTTACAGtTTTACAAGCAAtTGtTCTTtTTATACT
18 7800 ctGTgcatacatagTTTatGcaACcGaaaTAggttgggcaGcaCaTacTATACTtttc
con cg-aac---ttt-ggttatg--acccat-tA-a-ttc-tt-ttataataatact----
O15-CTGTGCATACATAGTTTATGCAACCGAAATAGGTTGGGCAGCACATACTATACTTTTC-(O15)
O24-CAAACCGATTTT GGGTTACACATTTACAAGCAACTTATATAATAATACTAA(-O24)

## Claims (Claims for the following Contracting State(s): AT, BE, CH, LI, DE, DK, FR, GB, GR, IT, NL, SE)

1. A composition useful in LCR for amplifying the DNA of human papilloma virus present in a test sample, said composition comprising a set of four oligonucleotide probes, said probe sets being selected from the group consisting of the following oligonucleotide sets:

LCR5: SEQ ID No.
81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA;

LCR6: SEQ ID No.
85 CTTCACTGCA AGACATAGAA ATAA,
86 TTATTTCTAT GTCTTGCAGT GAA,
87 CCTGTGTATA TTGCAAGACA GTAT,
88 TACTGTCTTG CAATATACAC AGG;

LCR7: SEQ ID No.
89 TATATTGCAA GACAGTATTG GAAC,
90 GTTCCAATAC TGTCTTGCAA TTTA,
91 TTACAGAGGT ATTTGAATTT GCATT,
92 AATGCAAATT CAAATACCTC TGTAA; and

LCR8: SEQ ID No.
93 GTATGGAACA ACATTAGAAC AGCA,
94 TGCTGTTCTA ATGTTGTTCC ATAC,
95 ATACAACAAA CCGTTGTGTG ATTT,
96 AAATCACACA ACGGTTTGTT GTAT.

2. A composition according to claim 1 for amplifying the DNA of human papilloma virus type 1 6 present in a test sample, said composition comprising a set of four oligonucleotide probes, said probe sets being selected from the group consisting of the following oligonucleotide sets:
LCR5 (SEQ ID Nos. 81,82,83 and 84) and LCR8 (SEQ ID Nos. 93, 94, 95 and 96).

3. A composition according to claim 1 for amplifying the DNA of human papilloma virus type 18 present in a test sample, said composition comprising a set of four oligonucleotide probes, said probe sets being selected from the group consisting of the following oligonucleotide sets:
LCR 6(SEQ ID Nos. 85,86,87 and 88) and LCR 7 (SEQ ID Nos. 89,90,91 and 92).

4. A kit for detecting the presence of human papilloma virus DNA in a test sample, comprising:
a composition according to any of claims 1 to 3; and
further comprising a ligase.

5. A kit according to claim 4, wherein said ligase is thermostable.

6. A composition useful in PCR for amplifying the DNA of human papilloma virus present in a test sample, said composition comprising:
a first nucleic acid primer of sense direction, capable of hybridizing to the antisense strand of HPV DNA , said primer having from 10 to about 30 nucleotides in length and having a sequence selected from the group consisting of the following sequences: 
SEQ ID No.
1 CAGATGTCTC TGTGGCGGCC TAGTG,

6 GAATTAGTTA GACCATTTAA AAG,
7 GGGGAAACAC CAGAATGGAT A,

81 GCTGCAAACA ACTATACATG ATATAA,
85 CTTCACTGCA AGACATAGAA ATAA,
89 TATATTGCAA GACAGTATTG GAAC and
93 GTATGGAACA ACATTAGAAC AGCA; and
a second nucleic acid primer of antisense direction, capable of hybridizing to the sense strand of HPV DNA, said primer having from 10 to about 30 nucleotides in length and-having a sequence selected from the group consisting of the following sequences: 
SEQ ID No.

5 AGGTGTCAGG AAAACCAAAT TTATT,

84 TGCTTGCAGT ACACACATTC TAATA,
88 TACTGTCTTG CAATATACAC AGG,
92 AATGCAAATT CAAATACCTC TGTAA and
96 AAATCACACA ACGGTTTGTT GTAT;
provided said first and second primers hybridize to their respective antisense and sense strands at locations such that their 3' ends do not overlap and, in the direction of extension, the 5' ends of said primers are spaced further apart than the 3' ends of said primers.

7. A composition according to claim 6 wherein said first and second primers are selected from the following pairs of oligonucleotide sequences ( identified by Sequence ID No.):
1 and 5, 6 and 5, 7 and 5, 81 and 84,
85 and 88, 89 and 92, and 93 and 96.

8. A kit for detecting the presence of human papilloma virus DNA in a test sample, comprising:
a composition according to claim 6 or 7 ; and
further comprising a polymerase.

9. A kit according to claim 8 wherein said polymerase is thermostable.

10. A consensus oligonucleotide for hybridizing human papilloma virus types 6, 11, 16, 18, 31, 33 and 61, which oligonucleotide comprises from about 10 to about 60 nucleotides in length and is selected from the group of sequences consisting of: 
SEQ ID No.
1 CAGATGTCTC TGTGGCGGCC TAGTG,
5 AGGTGTCAGG AAAACCAAAT TTATT,
6 GAATTAGTTA GACCATTTAA AAG and
7 GGGGAAACAC CAGAATGGAT A;
 and their complements.

11. A type-specific oligonucleotide for determining the presence of human papilloma virus type 16, having a sequence selected from the group consisting of: 
SEQ ID No.

81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA,
93 GTATGGAACA ACATTAGAAC AGCA,
94 TGCTGTTCTA ATGTTGTTCC ATAC,
95 ATACAACAAA CCGTTGTGTG ATTT and
96 AAATCACACA ACGGTTTGTT GTAT;
 and their complements.

12. A type-specific oligonucleotide for determining the presence of human papilloma virus type 18, having a sequence selected from the group consisting of: SEQ ID No. 
SEQ ID No.

85 CTTCACTGCA AGACATAGAA ATAA,
86 TTATTTCTAT GTCTTGCAGT GAA,
87 CCTGTGTATA TTGCAAGACA GTAT,
88 TACTGTCTTG CAATATACAC AGG,
89 TATATTGCAA GACAGTATTG GAAC,
90 GTTCCAATAC TGTCTTGCAA TTTA,
91 TTACAGAGGT ATTTGAATTT GCATT and
92 AATGCAAATT CAAATACCTC TGTAA;
 and their complements.

13. A method for determining the presence of any human papilloma virus in a test sample, comprising:
a. hybridizing DNA in the test sample with at least one consensus oligonucleotide selected from the group of claim 10, said oligonucleotide being conjugated to a signal generating compound capable of producing a detectable signal; and
b. determining the presence of human papilloma virus by detecting the signal generated.

14. A method for determining the presence of human papilloma virus type 16 in a test sample, comprising:
a. hybridizing DNA in the test sample with at least one oligonucleotide selected from the group of claim 11, said oligonucleotide being conjugated to a signal generating compound capable of producing a detectable signal; and
b. determining the presence of human papilloma virus by detecting the signal generated.

15. A method for determining the presence of human papilloma virus type 18 in a test sample, comprising:
a. hybridizing DNA in the test sample with at least one oligonucleotide selected from the group of claim 12, said oligonucleotide being conjugated to a signal generating compound capable of producing a detectable signal; and
b. determining the presence of human papilloma virus by detecting the signal generated.

16. A method according to any of claims 13-15, further comprising a step of amplification prior to or concurrent with said hybridizing step.

17. A method according to claim 16, wherein said amplification step comprises PCR or LCR.

## Claims (Claims for the following Contracting State(s): ES)

1. A composition useful in LCR for amplifying the DNA of human papilloma virus present in a test sample, said composition comprising a set of four oligonucleotide probes, said probe sets being selected from the group consisting of the following oligonucleotide sets:

LCR5: SEQ ID No.
81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA;

LCR6: SEQ ID No.
85 CTTCACTGCA AGACATAGAA ATAA,
86 TTATTTCTAT GTCTTGCAGT GAA,
87 CCTGTGTATA TTGCAAGACA GTAT,
88 TACTGTCTTG CAATATACAC AGG;

LCR7: SEQ ID No.
89 TATATTGCAA GACAGTATTG GAAC,
90 GTTCCAATAC TGTCTTGCAA TTTA,
91 TTACAGAGGT ATTTGAATTT GCATT,
92 AATGCAAATT CAAATACCTC TGTAA; and

LCR8: SEQ ID No.
93 GTATGGAACA ACATTAGAAC AGCA,
94 TGCTGTTCTA ATGTTGTTCC ATAC,
95 ATACAACAAA CCGTTGTGTG ATTT,
96 AAATCACACA ACGGTTTGTT GTAT.

2. A composition according to claim 1 for amplifying the DNA of human papilloma virus type 16 present in a test sample, said composition comprising a set of four oligonucleotide probes, said probe sets being selected from the group consisting of the fol lowing oligonucleotide sets :
LCR5 (SEQ ID Nos. 81,82,83 and 84) and LCR8 (SEQ ID Nos. 93, 94, 95 and 96).

3. A composition according to claim 1 for amplifying the DNA of human papilloma virus type 18 present in a test sample, said composition comprising a set of four oligonucleotide probes, said probe sets being selected from the group consisting of the following oligonucleotide sets:
LCR6(SEQ ID Nos. 85,86,87 and 88) and LCR 7(SEQ ID Nos. 89,90,91 and 92).

4. A kit for detecting the presence of human papilloma virus DNA in a test sample, comprising:
a composition according to any of claims 1 to 3; and further comprising a ligase.

5. A kit according to claim 4, wherein said ligase is thermostable.

6. A composition useful in PCR for amplifying the DNA of human papilloma virus present in a test sample, said composition comprising:
a first nucleic acid primer of sense direction, capable of hybridizing to the antisense strand of HPV DNA, said primer having from 10 to about 30 nucleotides in length and having a sequence selected from the group consisting of the following sequences: 
SEQ ID No.
1 CAGATGTCTC TGTGGCGGCC TAGTG,
6 GAATTAGTTA GACCATTTAA AAG,
7 GGGGAAACAC CAGAATGGAT A,
81 GCTGCAAACA ACTATACATG ATATAA,
85 CTTCACTGCA AGACATAGAA ATAA,
89 TATATTGCAA GACAGTATTG GAAC and
93 GTATGGAACA ACATTAGAAC AGCA; and
a second nucleic acid primer of antisense direction, capable of hybridizing to the sense strand of HPV DNA, said primer having from 10 to about 30 nucleotides in length and having a sequence selected from the group consisting of the following sequences: 
SEQ ID No.

5 AGGTGTCAGG AAAACCAAAT TTATT,

84 TGCTTGCAGT ACACACATTC TAATA,
88 TACTGTCTTG CAATATACAC AGG,
92 AATGCAAATT CAAATACCTC TGTAA and
96 AAATCACACA ACGGTTTGTT GTAT;
provided said first and second primers hybridize to their respective antisense and sense strands at locations such that their 3' ends do not overlap and, in the direction of extension, the 5' ends of said primers are spaced further apart than the 3' ends of said primers.

7. A composition according to claim 6 wherein said first and second primers are selected from the following pairs of oligonucleotide sequences (identified by Sequence ID No.):
1 and 5, 6 and 5, 7 and 5, 81 and 84,
85 and 88, 89 and 92, and 93 and 96.

8. A kit for detecting the presence of human papilloma virus DNA in a test sample, comprising:
a composition according to claim 6 or 7; and
further comprising a polymerase.

9. A kit according to claim 8 wherein said polymerase is thermostable.

10. A method for determining the presence of any human papilloma virus in a test sample, comprising:
a. hybridizing DNA in the test sample with at least one consensus oligonucleotide selected from the group of sequences consisting of: 
SEQ ID No.
1 CAGATGTCTC TGTGGCGGCC TAGTG,
5 AGGTGTCAGG AAAACCAAAT TTATT,
6 GAATTAGTTA GACCATTTAA AAG and
7 GGGGAAACAC CAGAATGGAT A;
 and their complements,
said oligonucleotide being conjugated to a signal generating compound capable of producing a detectable signal; and
b. determining the presence of human papilloma virus by detecting the signal generated.

11. . A method for determining the presence of human papilloma virus type 16 in a test sample, comprising:
a. hybridizing DNA in the test sample with at least one oligonucleotide selected from the group of sequences consisting of: 
SEQ ID No.

81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA,
93 GTATGGAACA ACATTAGAAC AGCA,
94 TGCTGTTCTA ATGTTGTTCC ATAC,
95 ATACAACAAA CCGTTGTGTG ATTT and
96 AAATCACACA ACGGTTTGTT GTAT;
 and their complements, said oligonucleotide being conjugated to a signal generating compound capable of producing a detectable signal; and
b. determining the presence of human papilloma virus by detecting the signal generated.

12. . A method for determining the presence of human papilloma virus type 18 in a test sample, comprising:
a. hybridizing DNA in the test sample with at least one oligonucleotide selected from the group of sequences consisting of: 
SEQ ID No.

85 CTTCACTGCA AGACATAGAA ATAA,
86 TTATTTCTAT GTCTTGCAGT GAA,
87 CCTGTGTATA TTGCAAGACA GTAT,
88 TACTGTCTTG CAATATACAC AGG,
89 TATATTGCAA GACAGTATTG GAAC,
90 GTTCCAATAC TGTCTTGCAA TTTA,
91 TTACAGAGGT ATTTGAATTT GCATT and
92 AATGCAAATT CAAATACCTC TGTAA;
 and their complements,
said oligonucleotide being conjugated to a signal generating compound capable of producing a detectable signal; and
b. determining the presence of human papilloma virus by detecting the signal generated.

13. A method according to any of claims 10-12 , further comprising a step of amplification prior to or concurrent with said hybridizing step.

14. A method according to claim 13, wherein said amplification step comprises PCR or LCR.

## Patentansprüche (Patentansprüche für folgende(n) Vertragsstaat(en): AT, BE, CH, LI, DE, DK, FR, GB, GR, IT, NL, SE)

1. Zusammensetzung, die für die LCR ("ligase chain reaction", Ligasekettenreaktion) zur Vervielfachung der DNA des humanen Papillomavirus nützlich ist, der in einer Testprobe vorhanden ist, wobei die Zusammensetzung einen Satz von vier Oligonukleotidsonden umfaßt, wobei die Sondensätze aus der Gruppe gewählt sind, die aus den folgenden Oligonukleotidsätzen besteht:

LCRS: SEQ ID N[?]
81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA;

LCR6: SEQ ID N r
85 CTTCACTGCA AGACATAGAA ATAA,
86 TTATTTCTAT GTCTTGCAGT GAA,
87 CCTGTGTATA TTGCAAGACA GTAT,
88 TACTGTCTTG CAATATACAC AGG;

LCR7: SEQ ID Nr
89 TATATTGCAA GACAGTATTG GAAC,
90 GTTCCAATAC TGTCTTGCAA TTTA,
91 TTACAGAGGT ATTTGAATTT GCATT,
92 AATGCAAATT CAAATACCTC TGTAA; und

LCR8: SEQ ID Nr
93 GTATGGAACA ACATTAGAAC AGCA,
94 TGCTGTTCTA ATGTTGTTCC ATAC,
95 ATACAACAAA CCGTTGTGTG ATTT,
96 AAATCACACA ACGGTTTGTT GTAT.

2. Zusammensetzung nach Anspruch 1 zur Vervielfachung der DNA des humanen Papillomavirus Typ 16, der in einer Testprobe vorhanden ist, wobei die Zusammensetzung einen Satz von vier Oligonukleotidsonden umfaßt, wobei die Sondensätze aus der Gruppe gewählt sind, die aus den folgenden Oligonukleotidsätzen besteht:
LCR5 (SEQ ID Nrn 81, 82, 83 und 84) und LCR8 (SEQ ID Nrn 93, 94, 95 und 96)

3. Zusammensetzung nach Anspruch 1 zur Vervielfachung der DNA des humanen Papillomavirus Typ 18, der in einer Testprobe vorhanden ist, wobei die Zusammensetzung einen Satz von vier Oligonukleotidsonden umfaßt, wobei die Sondensätze aus der Gruppe gewählt sind, die aus den folgenden Oligonukleotidsätzen besteht:
LCR6 (SEQ ID Nrn 85, 86, 87 und 88) und LCR7 (SEQ ID Nrn 89, 90, 91 und 92).

4. Kit zum Nachweis der Anwesenheit der DNA des humanen Papillomavirus in einer Testprobe, das folgendes umfaßt:
eine Zusammensetzung nach einem der Ansprüche 1 bis 3, und des weiteren eine Ligase.

5. Kit nach Anspruch 4, worin die Ligase thermostabil ist.

6. Zusammensetzung, die bei der PCR ("polymerase chain reaction" Polymerasekettenreaktion) zur Vervielfachung der DNA des humanen Papillomavirus nützlich ist, der in einer Testprobe vorhanden ist, wobei die Zusammensetzung folgendes umfaßt:
einen ersten Nukleinsäureprimer, der zur Richtung gleichläufig ist, welcher zur Hybridisierung an den gegenläufigen Strang der HPV-DNA befähigt ist, wobei der Primer 10 bis ungefähr 30 Nukleotide lang ist und eine Sequenz aufweist, die aus der Gruppe gewählt ist, die aus den folgenden Sequenzen besteht: 
1 CAGATGTCTC TGTGGCGGCC TAGTG,

6 GAATTAGTTA GACCATTTAA AAG,
7 GGGGAAACAC CAGAATGGAT A,

81 GCTGCAAACA ACTATACATG ATATAA,
85 CTTCACTGCA AGACATAGAA ATAA,
89 TATATTGCAA GACAGTATTG GAAC und
93 GTATGGAACA ACATTAGAAC AGCA; und
einen zweiten Nukleinsäureprimer, der zur Richtung gegenläufig ist, welcher zur Hybridisierung an den gleichläufigen Strang der HPV-DNA befähigt ist, wobei der Primer 10 bis ungefähr 30 Nukleotide lang ist und eine Sequenz aufweist, die aus der Gruppe gewählt ist, die aus den folgenden Sequenzen besteht: 
SEQ ID Nr

5 AGGTGTCAGG AAAACCAAAT TTATT,

84 TGCTTGCAGT ACACACATTC TAATA,
88 TACTGTCTTG CAATATACAC AGG,
92 AATGCAAATT CAAATACCTC TGTAA und
96 AAATCACACA ACGGTTTGTT GTAT;
vorausgesetzt, daß der erste und der zweite Primer an ihre jeweiligen gleich- und gegenläufigen Stränge an solchen Stellen hybridisieren, daß ihre 3'-Enden nicht überlappen, und daß die 5'-Enden der Primer in Verlängerungsrichtung weiter räumlich abgesetzt sind als die 3'-Enden der Primer.

7. Zusammensetzung nach Anspruch 6, worin der erste und zweite Primer aus den folgenden Paaren von Oligonukleotidsequenzen (die durch die Sequenz ID Nr bezeichnet sind) gewählt sind:
1 und 5, 6 und 5, 7 und 5, 81 und 84,
85 und 88, 89 und 92, und 93 und 96.

8. Kit zum Nachweis der Anwesenheit der DNA des humanen Papillomavirus in einer Testprobe, das folgendes umfaßt:
eine Zusammensetzung nach Anspruch 6 oder ,7 und des weiteren eine Polymerase.

9. Kit nach Anspruch 8, worin die Polymerase thermostabil ist.

10. Consensus-Oligonukleotid zur Hybridisierung der humanen papillomaviren Typ 6, 11, 16, 18, 31, 33 und 61, wobei das Oligonukleotid ungefähr 10 bis ungefähr 60 Oligonukleotide lang ist und aus der Gruppe von Sequenzen gewählt ist, die aus folgendem besteht: 
SEO ID Nr

1 CAGATGTCTC TGTGGCGGCC TAGTG,
5 AGGTGTCAGG AAAACCAAAT TTATT,
6 GAATTAGTTA GACCATTTAA AAG
7 GGGGAAACAC CAGAATGGAT A; und
 und aus deren Komplementen.

11. Typ-spezifisches Oligonukleotid zur Bestimmung der Anwesenheit des humanen Papillomavirus Typ 16, das eine Sequenz aufweist, die aus der Gruppe gewählt ist, die aus folgendem besteht: 
SEO ID Nr

81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA,
93 GTATGGAACA ACATTAGAAC AGCA,
94 TGCTGTTCTA ATGTTGTTCC ATAC,
95 ATACAACAAA CCGTTGTGTG ATTT und
96 AAATCACACA ACGGTTTGTT GTAT;
 und aus deren Komplementen.

12. Typ-spezifisches Oligonukleotid zur Bestimmung der Anwesenheit des humanen Papillomavirus Typ 18, das eine Sequenz aufweist, die aus der Gruppe gewählt ist, die aus folgendem besteht: 
SEQ ID Nr

85 CTTCACTGCA AGACATAGAA ATAA,
86 TTATTTCTAT GTCTTGCAGT GAA,
87 CCTGTGTATA TTGCAAGACA GTAT,
88 TACTGTCTTG CAATATACAC AGG,
89 TATATTGCAA GACAGTATTG GAAC,
90 GTTCCAATAC TGTCTTGCAA TTTA,
91 TTACAGAGGT ATTTGAATTT GCATT und
92 AATGCAAATT CAAATACCTC TGTAA;
 und aus deren Komplementen.

13. Verfahren zur Bestimmung der Anwesenheit irgendeines humanen Papillomavirus in einer Testprobe, das folgendes umfaßt:
a. Hybridisieren der DNA in der Testprobe mit wenigstens einem Consensus-Oligonukleotid, das aus der Gruppe nach Anspruch 10 gewählt ist, wobei das Oligonukleotid an eine signalerzeugende Verbindung konjugiert ist, die zur Erzeugung eines nachweisbaren Signals befähigt ist, und
b. Bestimmen der Anwesenheit des humanen Papillomavirus, indem das erzeugte Signal nachgewiesen wird.

14. Verfahren zur Bestimmung der Anwesenheit des humanen Papillomavirus Typ 16 in einer Probe, das folgendes umfaßt:
a. Hybridisieren der DNA in der Testprobe mit wenigstens einem Oligonukleotid, das aus der Gruppe nach Anspruch 11 gewählt ist, wobei das Oligonukleotid an eine signalerzeugende Verbindung konjugiert ist, die zur Erzeugung eines nachweisbaren Signals befähigt ist, und
b. Bestimmen der Anwesenheit des humanen Papillomavirus, indem das erzeugte Signal nachgewiesen wird.

15. Verfahren zur Bestimmung der Anwesenheit des humanen Papillomavirus Typ 18 in einer Testprobe, das folgendes umfaßt:
a. Hybridisieren der DNA in der Testprobe mit wenigstens einem Oligonukleotid, das aus der Gruppe nach Anspruch 12 gewählt ist, wobei das Oligonukleotid an eine signalerzeugende Verbindung konjugiert ist, die zur Erzeugung eines nachweisbaren Signals befähigt ist, und
b. Bestimmen der Anwesenheit des humanen Papillomavirus, indem das erzeugte Signal nachgewiesen wird.

16. Verfahren nach einem der Ansprüche 13-15, das des weiteren einen Vervielfachungsschritt umfaßt, der vor oder in Konkurrenz mit dem Hybridisierungsschritt stattfindet.

17. Verfahren nach Anspruch 16, worin der Vervielfachungsschritt PCR oder LCR umfaßt.

## Patentansprüche (Patentansprüche für folgende(n) Vertragsstaat(en): ES)

1. Zusammensetzung, die für die LCR ("ligase chain reaction", Ligasekettenreaktion) zur Vervielfachung der DNA des humanen Papillomavirus nützlich ist, der in einer Testprobe vorhanden ist, wobei die Zusammensetzung einen Satz von vier Oligonukleotidsonden umfaßt, wobei die Sondensätze aus der Gruppe gewählt sind, die aus den folgenden Oligonukleotidsätzen besteht:

LCR5: SEQ ID Nr
81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA;

LCR6: SEQ ID Nr
85 CTTCACTGCA AGACATAGAA ATAA,
86 TTATTTCTAT GTCTTGCAGT GAA,
87 CCTGTGTATA TTGCAAGACA GTAT,
88 TACTGTCTTG CAATATACAC AGG;

LCR7: SEQ ID N r
89 TATATTGCAA GACAGTATTG GAAC,
90 GTTCCAATAC TGTCTTGCAA TTTA,
91 TTACAGAGGT ATTTGAATTT GCATT,
92 AATGCAAATT CAAATACCTC TGTAA;

LCR8: SEQ ID Nr
93 GTATGGAACA ACATTAGAAC AGCA,
94 TGCTGTTCTA ATGTTGTTCC ATAC,
95 ATACAACAAA CCGTTGTGTG ATTT,
96 AAATCACACA ACGGTTTGTT GTAT.
und

2. Zusammensetzung nach Anspruch 1 zur Vervielfachung der DNA des humanen Papillomavirus Typ 16, der in einer Testprobe vorhanden ist, wobei die Zusammensetzung einen Satz von vier Oligonukleotidsonden umfaßt, wobei die Sondensätze aus der Gruppe gewählt sind, die aus den folgenden Oligonukleotidsätzen besteht:
LCR5 (SEQ ID Nrn 81, 82, 83 und 84) und LCR8 (SEQ ID Nrn 93, 94, 95 und 96).

3. Zusammensetzung nach Anspruch 1 zur Vervielfachung der DNA des humanen Papillomavirus TYP 18, der in einer Testprobe vorhanden ist, wobei die Zusammensetzung einen Satz von vier Oligonukleotidsonden umfaßt, wobei die Sondensätze aus der Gruppe gewählt sind, die aus den folgenden Oligonukleotidsätzen besteht:
LCR6 (SEQ ID Nrn 85, 86, 87 und 88) und LCR7 (SEQ ID Nrn 89, 90, 91 und 92).

4. Kit zum Nachweis der Anwesenheit der DNA des humanen Papillomavirus in einer Testprobe, das folgendes umfaßt:
eine Zusammensetzung nach einem der Ansprüche 1 bis 3, und des weiteren eine Ligase.

5. Kit nach Anspruch 4, worin die Ligase thermostabil ist.

6. Zusammensetzung, die bei der PCR ("polymerase chain reaction" polymerasekettenreaktion) zur Vervielfachung der DNA des humanen Papillomavirus nützlich ist, der in einer Testprobe vorhanden ist, wobei die Zusammensetzung folgendes umfaßt:
einen ersten Nukleinsäureprimer, der zur Richtung gleichläufig ist, welcher zur Hybridisierung an den gegenläufigen Strang der HPV-DNA befähigt ist, wobei der Primer 10 bis ungefähr 30 Nukleotide lang ist und eine Sequenz aufweist, die aus der Gruppe gewählt ist, die aus den folgenden Sequenzen besteht: 
SEQ ID Nr

1 CAGATGTCTC TGTGGCGGCC TAGTG,

6 GAATTAGTTA GACCATTTAA AAG,
7 GGGGAAACAC CAGAATGGAT A,

81 GCTGCAAACA ACTATACATG ATATAA,
85 CTTCACTGCA AGACATAGAA ATAA,
89 TATATTGCAA GACAGTATTG GAAC und
93 GTATGGAACA ACATTAGAAC AGCA; und
einen zweiten Nukleinsäureprimer, der zur Richtung gegenläufig ist, welcher zur Hybridisierung an den gleichläufigen Strang der HPV-DNA befähigt ist, wobei der Primer 10 bis ungefähr 30 Nukleotide lang ist und eine Sequenz aufweist, die aus der Gruppe gewählt ist, die aus den folgenden Sequenzen besteht: 
SEQ ID Nr

5 AGGTGTCAGG AAAACCAAAT TTATT,

84 TGCTTGCAGT ACACACATTC TAATA,
88 TACTGTCTTG CAATATACAC AGG,
92 AATGCAAATT CAAATACCTC TGTAA und
96 AAATCACACA ACGGTTTGTT GTAT;
vorausgesetzt, daß der erste und der zweite Primer an ihre jeweiligen gleich- und gegenläufigen Stränge an solchen Stellen hybridisieren, daß ihre 3'-Enden nicht überlappen, und daß die 5'-Enden der Primer in Verlängerungsrichtung weiter räumlich abgesetzt sind als die 3'-Enden der Primer.

7. Zusammensetzung nach Anspruch 6, worin der erste und zweite Primer aus den folgenden Paaren von Oligonukleotidsequenzen (die durch die Sequenz ID Nr bezeichnet sind) gewählt sind:
1 und 5, 6 und 5, 7 und 5, 81 und 84,
85 und 88, 89 und 92, und 93 und 96.

8. Kit zum Nachweis der Anwesenheit der DNA des humanen Papillomavirus in einer Testprobe, das folgendes umfaßt:
eine Zusammensetzung nach Anspruch 6 oder 7, und des weiteren eine Polymerase.

9. Kit nach Anspruch 8, worin die Polymerase thermostabil ist.

10. Verfahren zur Bestimmung der Anwesenheit irgendeines humanen papillomavirus in einer Testprobe, das folgendes umfaßt:
a. Hybridisieren der DNA in der Testprobe mit wenigstens einem Consensus-Oligonukleotid, das aus der Gruppe von Sequenzen gewählt ist, die aus folgendem besteht: 
SEQ ID Nr

1 CAGATGTCTC TGTGGCGGCC TAGTG,
5 AGGTGTCAGG AAAACCAAAT TTATT
6 GAATTAGTTA GACCATTTAA AAG und
7 GGGGAAACAC CAGAATGGAT A;
 und aus deren Komplementen,
wobei das Oligonukleotid an eine signalerzeugende Verbindung konjugiert ist, die zur Erzeugung eines nachweisbaren Signals befähigt ist, und
b. Bestimmen der Anwesenheit des humanen Papillomavirus, indem das erzeugte Signal nachgewiesen wird.

11. Verfahren zur Bestimmung der Anwesenheit des humanen Papillomavirus Typ 16 in einer Probe, das folgendes umfaßt:
a. Hybridisieren der DNA in der Testprobe mit wenigstens einem Oligonukleotid, das aus der Gruppe von Sequenzen gewählt ist, die aus folgendem besteht: 
SEQ ID Nr

81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA,
93 GTATGGAACA ACATTAGAAC AGCA,
94 TGCTGTTCTA ATGTTGTTCC ATAC,
95 ATACAACAAA CCGTTGTGTG ATTT und
96 AAATCACACA ACGGTTTGTT GTAT;
 und aus deren Komplementen,
wobei das Oligonukleotid an eine signalerzeugende Verbindung konjugiert ist, die zur Erzeugung eines nachweisbaren Signals befähigt ist, und
b. Bestimmen der Anwesenheit des humanen Papillomavirus, indem das erzeugte Signal nachgewiesen wird.

12. Verfahren zur Bestimmung der Anwesenheit des humanen Papillomavirus Typ 18 in einer Testprobe, das folgendes umfaßt:
a. Hybridisieren der DNA in der Testprobe mit wenigstens einem Oligonukleotid, das aus der Gruppe von Sequenzen gewählt ist, die aus folgendem besteht: 
SEQ ID Nr

85 CTTCACTGCA AGACATAGAA ATAA,
86 TTATTTCTAT GTCTTGCAGT GAA,
87 CCTGTGTATA TTGCAAGACA GTAT,
88 TACTGTCTTG CAATATACAC AGG,
89 TATATTGCAA GACAGTATTG GAAC,
90 GTTCCAATAC TGTCTTGCAA TTTA,
91 TTACAGAGGT ATTTGAATTT GCATT und
92 AATGCAAATT CAAATACCTC TGTAA;
 und aus deren Komplementen,
wobei das Oligonukleotid an eine signalerzeugende Verbindung konjugiert ist, die zur Erzeugung eines nachweisbaren Signals befähigt ist, und
b. Bestimmen der Anwesenheit des humanen Papillomavirus, indem das erzeugte Signal nachgewiesen wird.

13. Verfahren nach einem der Ansprüche 10-12, das des weiteren einen Vervielfachungsschritt umfaßt, der vor oder in Konkurrenz mit dem Hybridisierungsschritt stattfindet.

14. Verfahren nach Anspruch 13, worin der vervielfachungsschritt PCR oder LCR umfaßt.

## Revendications (Revendications pour l'(les) Etat(s) contractant(s) suivant(s): AT, BE, CH, LI, DE, DK, FR, GB, GR, IT, NL, SE)

1. Composition utile dans la LCR pour amplifier l'ADN de virus du papillome humain présent dans échantillon à doser, ladite composition comprenant un ensemble de quatre sondes oligonucléotidiques, lesdits ensembles de sondes étant sélectionnés dans le groupe constitué par les ensembles d'oligonucléotides suivants :

LCR5 :     n° d'identification
81     GCTGCAAACA ACTATACATG ATATAA,
82     TTATATCATG TATAGTTGTT TGCAGC,
83     TATTAGAATG TGTGTACTGC AAGCA,
84     TGCTTGCAGT ACACACATTC TAATA ;

LCR6 : n° d'identification
85 CTTCACTGCA AGACATAGAA ATAA,
86 TTATTTCTAT GTCTTGCAGT GAA,
87 CCTGTGTATA TTGCAAGACA GTAT,
88 TACTGTCTTG CAATATACAC AGG ;

LCR7 :    n° d'identification
89    TATATTGCAA  GACAGTATTG  GAAC
90    GTTCCAATAC  TGTCTTGCAA  TTTA,
91    TTACAGAGGT  ATTTGAATTT  GCATT,
92    AATGCAAATT  CAAATACCTC  TGTAA ; et

LCR8 : n° d'identification
93 GTATGGAACA ACATTAGAAC AGCA,
94 TGCTGTTCTA ATGTTGTTCC ATAC,
95 ATACAACAAA CCGTTGTGTG ATTT,
96 AAATCACACA ACGGTTTGTT GTAT.

2. Composition selon la revendication 1, destinée à amplifier l'ADN de virus du papillome humain de type 16 présent dans un échantillon à doser, ladite composition comprenant un ensemble de quatre sondes oligonucléotidiques, lesdits ensembles de sondes étant sélectionnés dans le groupe constitué par les ensembles d'oligonucléotides suivants :
LCR5 (n° d'identification 81, 82, 83 et 84) et LCR8 (n° d'identification 93, 94, 95 et 96).

3. Composition selon la revendication 1, destinée à amplifier l'ADN de virus du papillome humain de type 18 présent dans un échantillon à doser, ladite composition comprenant un ensemble de quatre sondes oligonucléotidiques, lesdits ensembles de sondes étant sélectionnés dans le groupe constitué par les ensembles d'oligonucléotides suivants :
LCR6 (n° d'identification 85, 86, 87 et 88) et LCR7 (n° d'identification 89, 90, 91 et 92).

4. Kit de détection de la présence d'ADN de virus du papillome humain dans un échantillon à doser, comprenant :
une composition selon l'une quelconque des revendications 1 à 3, et en outre une ligase.

5. Kit selon la revendication 4, dans lequel ladite ligase est thermostable.

6. Composition utile dans la PCR pour amplifier l'ADN de virus du papillome humain présent dans un échantillon à doser, ladite composition comprenant :
une première amorce d'acide nucléique de direction sens, capable de s'hybrider au brin antisens de l'ADN de HPV, ladite amorce ayant de 10 à environ 30 nucléotides de long et une séquence sélectionnée dans le groupe constitué par les séquences suivantes : 
N° d'identification
1 | CAGATGTCTC TGTGGCGGCC TAGTG,

6 | GAATTAGTTA GACCATTTAA AAG,
7 | GGGGAAACAC CAGAATGGAT A,

81 | GCTGCAAACA ACTATACATG ATATAA,
85 | CTTCACTGCA AGACATAGAA ATAA,
89 | TATATTGCAA GACAGTATTG GAAC et
93 | GTATGGAACA ACATTAGAAC AGCA ; et
une deuxième amorce d'acide nucléique de direction antisens, capable de s'hybrider au brin sens de l'ADN de HPV, ladite amorce ayant de 10 à environ 30 nucléotides de long et une séquence sélectionnée dans le groupe constitué par les séquences suivantes : 
N° d'identification
5 | AGGTGTCAGG AAAACCAAAT TTATT,

84 | TGCTTGCAGT ACACACATTC TAATA,
88 | TACTGTCTTG CAATATACAC AGG,
92 | AATGCAAATT CAAATACCTC TGTAA et
96 | AAATCACACA ACGGTTTGTT GTAT ;
pour autant que lesdites première et deuxième amorces s'hybrident à leurs brins respectifs antisens et sens à des emplacements tels que leurs extrémités 3' ne se chevauchent pas et que, dans la direction d'extension, les extrémités 5' desdites amorces soient plus espacées que les extrémités 3' desdites amorces.

7. Composition selon la revendication 6, dans laquelle lesdites première et deuxième amorces sont sélectionnées parmi les paires suivantes de séquences oligonucléotidiques (identifiées par leur numéro d'identification) :
1 et 5, 6 et 5, 7 et 5, 81 et 84,
85 et 88, 89 et 92, et 93 et 96.

8. Kit de détection de la présence d'ADN de virus du papillome humain dans un échantillon à doser, comprenant :
une composition selon la revendication 6 ou 7 et en outre une polymérase.

9. Kit selon la revendication 8, dans lequel ladite polymérase est thermostable.

10. Oligonucléotide consensus pour hybridation du virus du papillome humain des types 6, 11, 16, 18, 31, 33 et 61, lequel oligonucléotide a d'environ 10 à environ 60 nucléotides de long et est sélectionné dans le groupe de séquences constitué par : 
N° d'identification
1 CAGATGTCTC TGTGGCGGCC TAGTG,
5 AGGTGTCAGG AAAACCAAAT TTATT,
6 GAATTAGTTA GACCATTTAA AAG et
7 GGGGAAACAC CAGAATGGAT A ;
 et leurs compléments.

11. Oligonucléotide spécifique d'un type, destiné à déterminer la présence du virus du papillome humain de type 16, ayant une séquence sélectionnée dans le groupe constitué par : 
N° d'identification
81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA,
93 GTATGGAACA ACATTAGAAC AGCA,
94 TGCTGTTCTA ATGTTGTTCC ATAC,
95 ATACAACAAA CCGTTGTGTG ATTT et
96 AAATCACACA ACGGTTTGTT GTAT ;
 et leurs compléments.

12. Oligonucléotide spécifique d'un type, destiné à déterminer la présence du virus du papillome humain de type 18, ayant une séquence sélectionnée dans le groupe constitué par : 
N° d'identification
85 | CTTCACTGCA AGACATAGAA ATAA,
86 | TTATTTCTAT GTCTTGCAGT GAA,
87 | CCTGTGTATA TTGCAAGACA GTAT,
88 | TACTGTCTTG CAATATACAC AGG,
89 | TATATTGCAA GACAGTATTG GAAC,
90 | GTTCCAATAC TGTCTTGCAA TTTA,
91 | TTACAGAGGT ATTTGAATTT GCATT et
92 | AATGCAAATT CAAATACCTC TGTAA ;
 et leurs compléments.

13. Procédé de détermination de la présence d'un virus quelconque du papillome humain dans un échantillon à doser, comprenant :
a. l'hybridation de l'ADN dans l'échantillon à doser avec au moins un oligonucléotide consensus sélectionné dans le groupe selon la revendication 10, ledit oligonucléotide étant conjugué à un composé émetteur d'un signal, capable de produire un signal détectable, et
b. la détermination de la présence du virus du papillome humain par détection du signal émis.

14. Procédé de détermination de la présence du virus du papillome humain de type 16 dans un échantillon à doser, comprenant :
a. l'hybridation de l'ADN dans l'échantillon à doser avec au moins un oligonucléotide sélectionné dans le groupe selon la revendication 11, ledit oligonucléotide étant conjugué à un composé émetteur d'un signal, capable de produire un signal détectable, et
b. la détermination de la présence du virus du papillome humain par détection du signal émis.

15. Procédé de détermination de la présence du virus du papillome humain de type 18 dans un échantillon à doser, comprenant :
a. l'hybridation de l'ADN dans l'échantillon à doser avec au moins un oligonucléotide sélectionné dans le groupe selon la revendication 12, ledit oligonucléotide étant conjugué à un composé émetteur d'un signal, capable de produire un signal détectable, et
b. la détermination de la présence du virus du papillome humain par détection du signal émis.

16. Procédé selon une quelconque des revendications 13 à 15, comprenant en outre une étape d'amplification avant ou pendant ladite étape d'hybridation.

17. Procédé selon la revendication 16, dans lequel ladite étape d'amplification comprend la PCR ou la LCR.

## Revendications (Revendications pour l'(les) Etat(s) contractant(s) suivant(s): ES)

1. Composition utile dans la LCR pour amplifier l'ADN de virus du papillome humain présent dans échantillon à doser, ladite composition comprenant un ensemble de quatre sondes oligonucléotidiques, lesdits ensembles de sondes étant sélectionnés dans le groupe constitué par les ensembles d'oligonucléotides suivants :

LCR5 : n° d'identification
81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA ;

LCR6 :     n° d'identification
85     CTTCACTGCA   AGACATAGAA  ATAA,
86     TTATTTCTAT   GTCTTGCAGT   GAA,
87     CCTGTGTATA   TTGCAAGACA   GTAT,
88     TACTGTCTTG   CAATATACAC   AGG ;

LCR7 : n° d'identification
89 TATATTGCAA GACAGTATTG GAAC
90 GTTCCAATAC TGTCTTGCAA TTTA,
91 TTACAGAGGT ATTTGAATTT GCATT,
92 AATGCAAATT CAAATACCTC TGTAA ; et

LCR8 : n° d'identification
93 GTATGGAACA ACATTAGAAC AGCA,
94 TGCTGTTCTA ATGTTGTTCC ATAC,
95 ATACAACAAA CCGTTGTGTG ATTT,
96 AAATCACACA ACGGTTTGTT GTAT.

2. Composition selon la revendication 1, destinée à amplifier l'ADN de virus du papillome humain de type 16 présent dans un échantillon à doser, ladite composition comprenant un ensemble de quatre sondes oligonucléotidiques, lesdits ensembles de sondes étant sélectionnés dans le groupe constitué par les ensembles d'oligonucléotides suivants :
LCR5 (n° d'identification 81, 82, 83 et 84) et LCR8 (n° d'identification 93, 94, 95 et 96).

3. Composition selon la revendication 1, destinée à amplifier l'ADN de virus du papillome humain de type 18 présent dans un échantillon à doser, ladite composition comprenant un ensemble de quatre sondes oligonucléotidiques, lesdits ensembles de sondes étant sélectionnés dans le groupe constitué par les ensembles d'oligonucléotides suivants :
LCR6 (n° d'identification 85, 86, 87 et 88) et LCR7 (n° d'identification 89, 90, 91 et 92).

4. Kit de détection de la présence d'ADN de virus du papillome humain dans un échantillon à doser, comprenant :
une composition selon l'une quelconque des revendications 1 à 3, et en outre une ligase.

5. Kit selon la revendication 4, dans lequel ladite ligase est thermostable.

6. Composition utile dans la PCR pour amplifier l'ADN de virus du papillome humain présent dans un échantillon à doser, ladite composition comprenant :
une première amorce d'acide nucléique de direction sens, capable de s'hybrider au brin antisens de l'ADN de HPV, ladite amorce ayant de 10 à environ 30 nucléotides de long et une séquence sélectionnée dans le groupe constitué par les séquences suivantes : 
N° d'identification
1 CAGATGTCTC TGTGGCGGCC TAGTG,

6 GAATTAGTTA GACCATTTAA AAG,
7 GGGGAAACAC CAGAATGGAT A,

81 GCTGCAAACA ACTATACATG ATATAA,
85 CTTCACTGCA AGACATAGAA ATAA,
89 TATATTGCAA GACAGTATTG GAAC et
93 GTATGGAACA ACATTAGAAC AGCA ; et
une deuxième amorce d'acide nucléique de direction antisens, capable de s'hybrider au brin sens de l'ADN de HPV, ladite amorce ayant de 10 à environ 30 nucléotides de long et une séquence sélectionnée dans le groupe constitué par les séquences suivantes : 
N° d'identification
5 AGGTGTCAGG AAAACCAAAT TTATT,

84 TGCTTGCAGT ACACACATTC TAATA,
88 TACTGTCTTG CAATATACAC AGG,
92 AATGCAAATT CAAATACCTC TGTAA et
96 AAATCACACA ACGGTTTGTT GTAT ;
pour autant que lesdites première et deuxième amorces s'hybrident à leurs brins respectifs antisens et sens à des emplacements tels que leurs extrémités 3' ne se chevauchent pas et que, dans la direction d'extension, les extrémités 5' desdites amorces soient plus espacées que les extrémités 3' desdites amorces.

7. Composition selon la revendication 6, dans laquelle lesdites première et deuxième amorces sont sélectionnées parmi les paires suivantes de séquences oligonucléotidiques (identifiées par leur numéro d'identification) :
1 et 5, 6 et 5, 7 et 5, 81 et 84,
85 et 88, 89 et 92, et 93 et 96.

8. Kit de détection de la présence d'ADN de virus du papillome humain dans un échantillon à doser, comprenant :
une composition selon la revendication 6 ou 7 et
en outre une polymérase.

9. Kit selon la revendication 8, dans lequel ladite polymérase est thermostable.

10. Procédé de détermination de la présence d'un virus quelconque du papillome humain dans un échantillon à doser, comprenant :
a. l'hybridation de l'ADN dans l'échantillon à doser avec au moins un oligonucléotide consensus sélectionné dans le groupe de séquences constitué par : 
N° d'identification
1 CAGATGTCTC TGTGGCGGCC TAGTG,
5 AGGTGTCAGG AAAACCAAAT TTATT,
6 GAATTAGTTA GACCATTTAA AAG et
7 GGGGAAACAC CAGAATGGAT A ;
 et leurs compléments, ledit oligonucléotide étant conjugué à un composé émetteur d'un signal, capable de produire un signal détectable, et
b. la détermination de la présence du virus du papillome humain par détection du signal émis.

11. Procédé de détermination de la présence du virus du papillome humain de type 16 dans un échantillon à doser, comprenant :
a. l'hybridation de l'ADN dans l'échantillon à doser avec au moins un oligonucléotide sélectionné dans le groupe de séquences constitué par : 
N° d'identification
81 GCTGCAAACA ACTATACATG ATATAA,
82 TTATATCATG TATAGTTGTT TGCAGC,
83 TATTAGAATG TGTGTACTGC AAGCA,
84 TGCTTGCAGT ACACACATTC TAATA,
93 GTATGGAACA ACATTAGAAC AGCA,
94 TGCTGTTCTA ATGTTGTTCC ATAC,
95 ATACAACAAA CCGTTGTGTG ATTT et
96 AAATCACACA ACGGTTTGTT GTAT ;
 et leurs compléments,
ledit oligonucléotide étant conjugué à un composé émetteur d'un signal, capable de produire un signal détectable, et
b. la détermination de la présence du virus du papillome humain par détection du signal émis.

12. Procédé de détermination de la présence du virus du papillome humain de type 18 dans un échantillon à doser, comprenant :
a. l'hybridation de l'ADN dans l'échantillon à doser avec au moins un oligonucléotide sélectionné dans le groupe de séquences constitué par : 
N° d'identification
85 | CTTCACTGCA AGACATAGAA ATAA,
86 | TTATTTCTAT GTCTTGCAGT GAA,
87 | CCTGTGTATA TTGCAAGACA GTAT,
88 | TACTGTCTTG CAATATACAC AGG,
89 | TATATTGCAA GACAGTATTG GAAC,
90 | GTTCCAATAC TGTCTTGCAA TTTA,
91 | TTACAGAGGT ATTTGAATTT GCATT et
92 | AATGCAAATT CAAATACCTC TGTAA ;
 et leurs compléments,
ledit oligonucléotide étant conjugué à un composé émetteur d'un signal, capable de produire un signal détectable, et
b. la détermination de la présence du virus du papillome humain par détection du signal émis.

13. Procédé selon une quelconque des revendications 10 à 12, comprenant en outre une étape d'amplification avant ou pendant ladite étape d'hybridation.

14. Procédé selon la revendication 13, dans lequel ladite étape d'amplification comprend la PCR ou la LCR.
